# EUROPEAN PATENT SPECIFICATION

(11) **EP 1 818 393 B1**
(45) Date of publication and mention of the grant of the patent: **14.12.2011**
(21) Application number: 06101431.2
(22) Date of filing: 08.02.2006
(51) Int. Cl.: C12N 9/16, A61K 45/00

(54) **Neutral sphingomyelinase-3 and its use**
Neutrale Sphingomyelinase-3 und deren Verwendung
Sphingomyelinase-3 neutre et son utilisation

(43) Date of publication of application: 15.08.2007
(73) Proprietor: Krut, Oleg, 50739 Köln (DE); Krönke, Martin, 50968 Köln (DE)
(72) Inventor: Krönke, Martin, 50968 Köln (DE); Hoppe, Uta, 50924 Köln (DE); Krönke-Wiegmann, Katja, 50968 Köln (DE); Krut, Oleg, 50739 Köln (DE)
(74) Representative: von Kreisler Selting Werner

(56) References cited:
- DATABASE EMBL [Online] Human nSMase3 5 July 2004 (2004-07-05), STRAUSBER ET AL.: XP002393852 retrieved from EBI accession no. Q6P1P7 Database accession no. Q6P1P7
- DATABASE EMBL [Online] KIAA1418 protein 1 October 2000 (2000-10-01), NAGASE ET AL.: XP002393853 retrieved from EBI accession no. Q9P2C9 Database accession no. Q9P2C9
- BERNARDO KATUSSEVANI ET AL: "Purification and characterization of a magnesium-dependent neutral sphingomyelinase from bovine brain" JOURNAL OF BIOLOGICAL CHEMISTRY, AMERICAN SOCIETY OF BIOLOCHEMICAL BIOLOGISTS, BIRMINGHAM,, US, vol. 275, no. 11, 17 March 2000 (2000-03-17), pages 7641-7647, XP002177719 ISSN: 0021-9258
- KRUT OLEG ET AL: "Novel tumor necrosis factor-responsive mammalian neutral sphingomyelinase-3 is a C-tail-anchored protein." THE JOURNAL OF BIOLOGICAL CHEMISTRY. 12 MAY 2006, vol. 281, no. 19, 12 May 2006 (2006-05-12), pages 13784-13793, XP002393849 ISSN: 0021-9258

### SEQUENCE LISTING

<110> Universität zu Köln
<120> Neutral Sphingomyelinase-3 and its Use
<130> 052389ep
<160> 33
<170> PatentIn version 3.3
<210> 1
   <211> 4896
   <212> DNA
   <213> Homo sapiens
<400> 1
   gggcagagac acggccgcag ggtcctgggg ctcgctcgct agcctctgcc cggcacacat 60
   ggacttgagc atctggaaga cggcgagggg ggccacgttc agcttcagca ggtccaccag 120
   gatcctggcg gggacagacg cggggccggt gagccctctc cgcccggcgc ggccacctcc 180
   ctgaagaccc gacaaggacg ggggcggggg ctcctcccga ccagcgggcc gggcgtaccc 240
   ggaaagcaga ggtcgggggg tgtctggcta gcatgcggcc cccacccgcc ccgctcactt 300
   gaacacgtcg gggtcgatag cgccgcccgc cgcctgcgcc agctcgtaca gctccatctc 360
   ctcggtgctc agcaccttct tccgccgcag cgccagcttc tgccgggccg cctccagccc 420
   cgggggcgcc gccgaccccg gcccaggccc tacgccctgc gccgccatcc ccgcggcccg 480
   cggaaaggtg cgctccgccc cgccgcgccc cctagcggct gcgcccacca ccctctccct 540
   gcagcgtcaa cgtctggcct cccgggctgc cctggcggga ggggcgggag cgcggggacg 600
   gggccgcctc cgcggcgtca ttgaagccca acagttattg ggcgctcacg gtgtgctgag 660
   cggcgctcta ggagccggga gtggcatggc ggaccgcagg ggtccgctgc ttggcgatct 720
   gggcctccct gatagactgc attggtcgtg ctcgcttagg tggcagcacc cagcccagtg 780
   ccaggcatac attgggcgct cggtaaatgc ctctttcaag aaagcgtgca attttctggt 840
   ccgcaccggt gcaccactag gggtcgcttt tcggggcggg cggggggaag gggggggcac 900
   taatcaacaa tactgcttac gcgcacgcgg attccttgct ggggagaaag taccttgggg 960
   cgccggaggc cgccacaacg caggcgcatt cagctaagga ccactccctc ccccgcactc 1020
   ctgcctcgcc atttctcttc cccgcccggc cggccttcgc tttgcgcacg cgccttttga 1080
   ggtaacggcc caaagaggtg gaagcgcttt tcccgcccgg ccgcggggcg tggctctgcg 1140
   cgcagcttga tgacgacttt cggcgccgtg gcggaatggc ggcttccatc tctgaggcga 1200
   gcgacgctat ggatcccaca gtggtttgct aagaaggcca ttttcaactc tccactggag 1260
   gctgctatgg cgttccctca cctgcagcag cccagctttc tactggctag cctgaaagct 1320
   gactctataa ataagccctt tgcacagcag tgccaagact tggttaaagt cattgaggac 1380
   tttccagcaa aggagctgca caccatcttc ccatggctgg tagaaagcat ttttggcagc 1440
   ctagatggtg tcctcgttgg ctggaacctc cgctgcttac aggggcgcgt gaatcctgtg 1500
   gagtacagca tcgtgatgga atttctcgac cctggtggcc caatgatgaa gttggtttat 1560
   aagcttcaag ctgaagacta taagttcgac tttcctgtct cctacttgcc tggtcctgtg 1620
   aaggcgtcca tccaggagtg catcctccct gacagtcctc tgtaccacaa caaggtccag 1680
   ttcaccccta ctgggggcct tggtctgaac ttggccctga atccgttcga gtattacata 1740
   ttcttctttg ccttgagcct catcactcag aagccacttc ctgtgtccct ccacgtccgt 1800
   acttcagact gtgcctattt catcctggtg gacaggtacc tgtcatggtt cctgcccacc 1860
   gaaggcagtg tgcccccacc actctcctcc agcccagggg ggaccagccc ctcaccacct 1920
   cccaggacac cagccatacc ctttgcttcc tatggcctcc accacactag cctcctaaag 1980
   cgacacatct ctcatcagac gtctgtgaat gcagaccccg cctcccacga gatctggagg 2040
   tcagaaactc tgctccaggt ttttgttgaa atgtggcttc atcactattc cttggagatg 2100
   tatcaaaaaa tgcagtcccc tcatgccaag ctggaggttc tgcactaccg actcagtgtc 2160
   tccagcgccc tctacagccc cgcccaaccc agcctccagg ccctccacgc ctaccaagag 2220
   tcgttcacgc ctactgagga gcatgtgttg gtggtgcgcc tgctgctgaa gcacctgcac 2280
   gcctttgcca acagcctgaa gccagagcag gcctcaccct ccgcccactc ccacgccacc 2340
   agccccctgg aggagttcaa acgggctgct gtcccgaggt tcgtccagca gaaactctac 2400
   ctcttcttgc agcattgctt tggccactgg cccctggacg catcgttcag agctgtcctg 2460
   gagatgtggc tgagctacct gcagccgtgg cggtacgcgc ctgacaagca ggctccgggc 2520
   agcgactccc agccccggtg tgtgtcggag aaatgggcac cctttgtcca ggagaacctg 2580
   ctgatgtaca ccaagttgtt tgtgggcttt ctgaaccgcg cgctccgcac agacctggtc 2640
   agccccaagc acgcgctcat ggtgttccga gtggccaaag tctttgccca gcccaacctg 2700
   gctgagatga ttcagaaagg tgagcagcta ttcctggagc cagagctggt catcccccac 2760
   cgccagcacc gactcttcac ggcccccaca ttcactggga gcttcctgtc accctggcca 2820
   ccagcggtca ctgatgcctc cttcaaggtg aagagccacg tctacagcct ggagggccag 2880
   gactgcaagt acaccccgat gtttgggccc gaggcccgca ccctggtcct gcgcctcgct 2940
   cagctcatca cacaggccaa acacacagcc aagtccatct ccgaccagtg tgcggagagc 3000
   ccggctggcc actccttcct ctcatggctg ggctttagct ccatggacac caatggctcc 3060
   tacacagcca acgacctgga cgagatgggg caagacagtg tccggaagac agatgaatac 3120
   ctggagaagg ccctggagta cctgcgccag atattccggc tcagcgaagc gcagctcagg 3180
   cagttcacac tcgccttggg caccacccag gatgagaatg gaaaaaagca actccccgac 3240
   tgcatcgtgg gtgaggacgg actcatcctt acgcccctgg ggcggtacca gatcatcaat 3300
   gggctgcgaa ggtttgaaat tgagtaccag ggggacccgg agctgcagcc catccggagc 3360
   tatgagatcg ccagcttggt ccgcacactc tttaggctgt cgtctgccat caaccacaga 3420
   tttgcaggac agatggcggc tctgtgttcc cgggatgact tcctcggcag cttctgtcgc 3480
   taccacctca cagaacctgg gctggccagc aggcacctgc tgagccctgt ggggcggagg 3540
   caggtggccg gccacacccg cggccccagg ctcagcctgc gcttcctggg cagttaccgg 3600
   acgctggtct cgctgctgct ggccttcttc gtggcctctc tgttctgcgt cgggcccctc 3660
   ccatgcacgc tgctgctcac cctgggctat gtcctctacg cctctgccat gacactgctg 3720
   accgagcggg ggaagctgca ccagccctga aggtgtcagc tgccttcaga gcaggctgga   3780
   gggatttgcc acacagcccc acccttgggc tgagaggacc tgggaagccc ctccaggagg   3840
   gaacacggtc atcctcgggc ttctggagcg gggttcctgc agccgcagag gcatctggag 3900
   gaaacgcaac caagaaagga aggcaggtgg gccccagcaa aggagtagct gccagggctc 3960
   aacagctacg ctctgtgaca gcgcagagct cagcggcggc ctttccctcc ctccgccaag 4020
   gactcacggc caagccagct ctcggggcct tttttccact gcccatttgg ctactctgct 4080
   gcaccaagct tgggagccag cctgccaaca gccacctggg cctggcctcc ccactggctg 4140
   gccttgaggt tggcagagtg ggttgtggcg cttcctctct ctgtgtggga ccaggacagt 4200
   ggcttaagtc tccactccag gaaagaatca aagtttctag agttgtgaga aaaccagaga 4260
   gtggctgtcc tgattcttca ctgtgagggg cgttcttcat gttctcccag ctgttccaag 4320
   actgggccgt agaattccat gtttcaggag cctaagaccc tcccagagcc caggggcttc 4380
   accgcagacc ccaagccatt gagcacatca cccaaagcag tggccaacat cgcggacccc 4440
   tgtgccttgt cacagatggg tgctggtcct caggcgttgg ggacactgct gggtcgatgg 4500
   ggtcggattc tgccagtttc tgctctgcag ccaaagatgg tcagaagcat tgtcacttca 4560
   gtaacatcaa gtgctcaaag acatggcaac cgttcagtgg tacttaagta ttcaaaatat 4620
   acaactacag attctctgac agaaaccagc acggggtctt caccttcatt caccccacag 4680
   gcgacatgcg agggagaaca gcatctcagt ggtgatttcc aaaccaagcc tttgttttcg 4740
   gtgtggggtt ttgggggttt gctttaatgt ttttgaaatt gtaaatgttg ggctttgtat 4800
   tttgatgtaa actgagcata atggcatttt agggcctgtg accaaaaatg aagcttgtaa 4860
   cgaccatgga tctgaataaa catgtccttg cttctg 4896
<210> 2
   <211> 2598
   <212> DNA
   <213> Homo sapiens
<400> 2
   atgacgactt tcggcgccgt ggcggaatgg cggcttccat ctctgaggcg agcgacgcta 60
   tggatcccac agtggtttgc taagaaggcc attttcaact ctccactgga ggctgctatg 120
   gcgttccctc acctgcagca gcccagcttt ctactggcta gcctgaaagc tgactctata   180
   aataagccct ttgcacagca gtgccaagac ttggttaaag tcattgagga ctttccagca   240
   aaggagctgc acaccatctt cccatggctg gtagaaagca tttttggcag cctagatggt   300
   gtcctcgttg gctggaacct ccgctgctta caggggcgcg tgaatcctgt ggagtacagc   360
   atcgtgatgg aatttctcga ccctggtggc ccaatgatga agttggttta taagcttcaa   420
   gctgaagact ataagttcga ctttcctgtc tcctacttgc ctggtcctgt gaaggcgtcc   480
   atccaggagt gcatcctccc tgacagtcct ctgtaccaca acaaggtcca gttcacccct   540
   actgggggcc ttggtctgaa cttggccctg aatccgttcg agtattacat attcttcttt   600
   gccttgagcc tcatcactca gaagccactt cctgtgtccc tccacgtccg tacttcagac   660
   tgtgcctatt tcatcctggt ggacaggtac ctgtcatggt tcctgcccac cgaaggcagt   720
   gtgcccccac cactctcctc cagcccaggg gggaccagcc cctcaccacc tcccaggaca   780
   ccagccatac cctttgcttc ctatggcctc caccacacta gcctcctaaa gcgacacatc   840
   tctcatcaga cgtctgtgaa tgcagacccc gcctcccacg agatctggag gtcagaaact   900
   ctgctccagg tttttgttga aatgtggctt catcactatt ccttggagat gtatcaaaaa   960
   atgcagtccc ctcatgccaa gctggaggtt ctgcactacc gactcagtgt ctccagcgcc  1020
   ctctacagcc ccgcccaacc cagcctccag gccctccacg cctaccaaga gtcgttcacg  1080
   cctactgagg agcatgtgtt ggtggtgcgc ctgctgctga agcacctgca cgcctttgcc  1140
   aacagcctga agccagagca ggcctcaccc tccgcccact cccacgccac cagccccctg  1200
   gaggagttca aacgggctgc tgtcccgagg ttcgtccagc agaaactcta cctcttcttg  1260
   cagcattgct ttggccactg gcccctggac gcatcgttca gagctgtcct ggagatgtgg  1320
   ctgagctacc tgcagccgtg gcggtacgcg cctgacaagc aggctccggg cagcgactcc  1380
   cagccccggt gtgtgtcgga gaaatgggca ccctttgtcc aggagaacct gctgatgtac  1440
   accaagttgt ttgtgggctt tctgaaccgc gcgctccgca cagacctggt cagccccaag  1500
   cacgcgctca tggtgttccg agtggccaaa gtctttgccc agcccaacct ggctgagatg  1560
   attcagaaag gtgagcagct attcctggag ccagagctgg tcatccccca ccgccagcac  1620
   cgactcttca cggcccccac attcactggg agcttcctgt caccctggcc accagcggtc  1680
   actgatgcct ccttcaaggt gaagagccac gtctacagcc tggagggcca ggactgcaag  1740
   tacaccccga tgtttgggcc cgaggcccgc accctggtcc tgcgcctcgc tcagctcatc  1800
   acacaggcca aacacacagc caagtccatc tccgaccagt gtgcggagag cccggctggc  1860
   cactccttcc tctcatggct gggctttagc tccatggaca ccaatggctc ctacacagcc  1920
   aacgacctgg acgagatggg gcaagacagt gtccggaaga cagatgaata cctggagaag  1980
   gccctggagt acctgcgcca gatattccgg ctcagcgaag cgcagctcag gcagttcaca  2040
   ctcgccttgg gcaccaccca ggatgagaat ggaaaaaagc aactccccga ctgcatcgtg   2100
   ggtgaggacg gactcatcct tacgcccctg gggcggtacc agatcatcaa tgggctgcga   2160
   aggtttgaaa ttgagtacca gggggacccg gagctgcagc ccatccggag ctatgagatc   2220
   gccagcttgg tccgcacact ctttaggctg tcgtctgcca tcaaccacag atttgcagga   2280
   cagatggcgg ctctgtgttc ccgggatgac ttcctcggca gcttctgtcg ctaccacctc   2340
   acagaacctg ggctggccag caggcacctg ctgagccctg tggggcggag gcaggtggcc   2400
   ggccacaccc gcggccccag gctcagcctg cgcttcctgg gcagttaccg gacgctggtc   2460
   tcgctgctgc tggccttctt cgtggcctct ctgttctgcg tcgggcccct cccatgcacg   2520
   ctgctgctca ccctgggcta tgtcctctac gcctctgcca tgacactgct gaccgagcgg   2580
   gggaagctgc accagccc                                                 2598
<210> 3
   <211> 866
   <212> PRT
   <213> Homo sapiens
<400> 3
   Met Thr Thr Phe Gly Ala Val Ala Glu Trp Arg Leu Pro Ser Leu Arg
   1               5                   10                  15
   Arg Ala Thr Leu Trp Ile Pro Gln Trp Phe Ala Lys Lys Ala Ile Phe
               20                  25                  30
   Asn Ser Pro Leu Glu Ala Ala Met Ala Phe Pro His Leu Gln Gln Pro
           35                  40                  45
   Ser Phe Leu Leu Ala Ser Leu Lys Ala Asp Ser Ile Asn Lys Pro Phe
       50                  55                  60
   Ala Gln Gln Cys Gln Asp Leu Val Lys Val Ile Glu Asp Phe Pro Ala
   65                  70                  75                  80
   Lys Glu Leu His Thr Ile Phe Pro Trp Leu Val Glu Ser Ile Phe Gly
                   85                  90                  95
   Ser Leu Asp Gly Val Leu Val Gly Trp Asn Leu Arg Cys Leu Gln Gly
               100                 105                 110
   Arg Val Asn Pro Val Glu Tyr Ser Ile Val Met Glu Phe Leu Asp Pro
           115                 120                 125
   Gly Gly Pro Met Met Lys Leu Val Tyr Lys Leu Gln Ala Glu Asp Tyr
       130                 135                 140
   Lys Phe Asp Phe Pro Val Ser Tyr Leu Pro Gly Pro Val Lys Ala Ser
   145 150 155 160
   Ile Gln Glu Cys Ile Leu Pro Asp Ser Pro Leu Tyr His Asn Lys Val
   165 170 175
   Gln Phe Thr Pro Thr Gly Gly Leu Gly Leu Asn Leu Ala Leu Asn Pro
   180 185 190
   Phe Glu Tyr Tyr Ile Phe Phe Phe Ala Leu Ser Leu Ile Thr Gln Lys
   195 200 205
   Pro Leu Pro Val Ser Leu His Val Arg Thr Ser Asp Cys Ala Tyr Phe
   210 215 220
   Ile Leu Val Asp Arg Tyr Leu Ser Trp Phe Leu Pro Thr Glu Gly Ser
   225 230 235 240
   Val Pro Pro Pro Leu Ser Ser Ser Pro Gly Gly Thr Ser Pro Ser Pro
   245 250 255
   Pro Pro Arg Thr Pro Ala Ile Pro Phe Ala Ser Tyr Gly Leu His His
   260 265 270
   Thr Ser Leu Leu Lys Arg His Ile Ser His Gln Thr Ser Val Asn Ala
   275 280 285
   Asp Pro Ala Ser His Glu Ile Trp Arg Ser Glu Thr Leu Leu Gln Val
   290 295 300
   Phe Val Glu Met Trp Leu His His Tyr Ser Leu Glu Met Tyr Gln Lys
   305 310 315 320
   Met Gln Ser Pro His Ala Lys Leu Glu Val Leu His Tyr Arg Leu Ser
   325 330 335
   Val Ser Ser Ala Leu Tyr Ser Pro Ala Gln Pro Ser Leu Gln Ala Leu
   340 345 350
   His Ala Tyr Gln Glu Ser Phe Thr Pro Thr Glu Glu His Val Leu Val
   355 360 365
   Val Arg Leu Leu Leu Lys His Leu His Ala Phe Ala Asn Ser Leu Lys
   370 375 380
   Pro Glu Gln Ala Ser Pro Ser Ala His Ser His Ala Thr Ser Pro Leu
   385 390 395 400
   Glu Glu Phe Lys Arg Ala Ala Val Pro Arg Phe Val Gln Gln Lys Leu
                   405                 410                 415
   Tyr Leu Phe Leu Gln His Cys Phe Gly His Trp Pro Leu Asp Ala Ser
               420                 425                 430
   Phe Arg Ala Val Leu Glu Met Trp Leu Ser Tyr Leu Gln Pro Trp Arg
           435                 440                 445
   Tyr Ala Pro Asp Lys Gln Ala Pro Gly Ser Asp Ser Gln Pro Arg Cys
       450                 455                 460
   Val Ser Glu Lys Trp Ala Pro Phe Val Gln Glu Asn Leu Leu Met Tyr
   465                 470                 475                 480
   Thr Lys Leu Phe Val Gly Phe Leu Asn Arg Ala Leu Arg Thr Asp Leu
                   485                 490                 495
   Val Ser Pro Lys His Ala Leu Met Val Phe Arg Val Ala Lys Val Phe
               500                 505                 510
   Ala Gln Pro Asn Leu Ala Glu Met Ile Gln Lys Gly Glu Gln Leu Phe
           515                 520                 525
   Leu Glu Pro Glu Leu Val Ile Pro His Arg Gln His Arg Leu Phe Thr
       530                 535                 540
   Ala Pro Thr Phe Thr Gly Ser Phe Leu Ser Pro Trp Pro Pro Ala Val
   545                 550                 555                 560
   Thr Asp Ala Ser Phe Lys Val Lys Ser His Val Tyr Ser Leu Glu Gly
                   565                 570                 575
   Gln Asp Cys Lys Tyr Thr Pro Met Phe Gly Pro Glu Ala Arg Thr Leu
               580                 585                 590
   Val Leu Arg Leu Ala Gln Leu Ile Thr Gln Ala Lys His Thr Ala Lys
           595                 600                 605
   Ser Ile Ser Asp Gln Cys Ala Glu Ser Pro Ala Gly His Ser Phe Leu
       610                 615                 620
   Ser Trp Leu Gly Phe Ser Ser Met Asp Thr Asn Gly Ser Tyr Thr Ala
   625                 630                 635                 640
   Asn Asp Leu Asp Glu Met Gly Gln Asp Ser Val Arg Lys Thr Asp Glu
                   645                 650                 655
   Tyr Leu Glu Lys Ala Leu Glu Tyr Leu Arg Gln Ile Phe Arg Leu Ser
               660                 665                 670
   Glu Ala Gln Leu Arg Gln Phe Thr Leu Ala Leu Gly Thr Thr Gln Asp
           675                 680                 685
   Glu Asn Gly Lys Lys Gln Leu Pro Asp Cys Ile Val Gly Glu Asp Gly
       690                 695                 700
   Leu Ile Leu Thr Pro Leu Gly Arg Tyr Gln Ile Ile Asn Gly Leu Arg
   705                 710                 715                 720
   Arg Phe Glu Ile Glu Tyr Gln Gly Asp Pro Glu Leu Gln Pro Ile Arg
                   725                 730                 735
   Ser Tyr Glu Ile Ala Ser Leu Val Arg Thr Leu Phe Arg Leu Ser Ser
               740                 745                 750
   Ala Ile Asn His Arg Phe Ala Gly Gln Met Ala Ala Leu Cys Ser Arg
           755                 760                 765
   Asp Asp Phe Leu Gly Ser Phe Cys Arg Tyr His Leu Thr Glu Pro Gly
       770                 775                 780
   Leu Ala Ser Arg His Leu Leu Ser Pro Val Gly Arg Arg Gln Val Ala
   785                 790                 795                 800
   Gly His Thr Arg Gly Pro Arg Leu Ser Leu Arg Phe Leu Gly Ser Tyr
                   805                 810                 815
   Arg Thr Leu Val Ser Leu Leu Leu Ala Phe Phe Val Ala Ser Leu Phe
               820                 825                 830
   Cys Val Gly Pro Leu Pro Cys Thr Leu Leu Leu Thr Leu Gly Tyr Val
           835                 840                 845
   Leu Tyr Ala Ser Ala Met Thr Leu Leu Thr Glu Arg Gly Lys Leu His
       850                 855                 860
   Gln Pro
   865
<210> 4
   <211> 827
   <212> PRT
   <213> Canis familiaris
<400> 4
   Met Ala Phe Pro His Leu Gln Gln Pro Ser Phe Leu Leu Ala Ser Leu
   1               5                   10                  15
   Lys Ala Asp Ser Ile Asn Lys Pro Phe Ala Gln Arg Cys Gln Asp Leu
               20                  25                  30
   Val Lys Val Ile Glu Asp Phe Pro Ala Lys Glu Leu His Thr Ile Phe
           35                  40                  45
   Pro Trp Leu Val Glu Ser Ile Phe Gly Ser Leu Asp Gly Val Leu Val
       50                  55                  60
   Gly Trp Asn Leu Arg Cys Leu Gln Gly Arg Val Asn Pro Met Glu Tyr
   65                  70                  75                  80
   Ser Ile Ala Met Glu Phe Leu Asp Pro Gly Gly Pro Met Met Lys Leu
                   85                  90                  95
   Val Tyr Lys Leu Gln Ala Glu Asp Tyr Lys Phe Asp Phe Pro Val Ser
               100                 105                 110
   Tyr Leu Pro Gly Pro Val Lys Ala Ser Ile Gln Glu Arg Val Leu Pro
           115                 120                 125
   Asp Ser Pro Leu Tyr His Asn Lys Val Gln Phe Pro Ala Thr Gly Gly
       130                 135                 140
   Leu Gly Leu Asn Leu Ala Leu Asn Pro Phe Glu Tyr Tyr Met Phe Phe
   145                 150                 155                 160
   Phe Ala Leu Ser Leu Ile Thr Gln Lys Pro Leu Pro Gly Ala Leu His
                   165                 170                 175
   Ile Arg Thr Ser Asp Cys Ala Tyr Phe Ile Leu Val Asp Arg Tyr Leu
               180                 185                 190
   Ser Trp Phe Leu Pro Thr Glu Gly Ser Val Leu Pro Pro Leu Ser Ser
           195                 200                 205
   Ser Pro Gly Gly Pro Ser Pro Ser Pro Ala Pro Arg Thr Pro Ala Ile
       210                 215                 220
   Pro Phe Ala Ser Tyr Gly Leu His His Thr Ser Leu Leu Lys Arg His
   225                 230                 235                 240
   Val Ser His Gln Thr Ser Val Asn Ala Asp Pro Ala Ser His Glu Ile
                   245                 250                 255
   Trp Arg Ser Glu Thr Leu Leu Gln Val Phe Val Glu Met Trp Leu His
               260                 265                 270
   His Tyr Ser Leu Glu Met Tyr Gln Lys Met Gln Ser Pro His Ala Lys
           275                 280                 285
   Leu Glu Val Leu His Tyr Arg Leu Ser Val Ser Ser Ala Leu His Ser
       290                 295                 300
   Pro Ala Gln Pro Ser Leu Gln Ala Leu His Ala Tyr Gln Glu Ser Phe
   305                 310                 315                 320
   Thr Pro Thr Glu Glu His Val Leu Val Val Arg Leu Leu Leu Lys His
                   325                 330                 335
   Leu His Ala Phe Ser Asn Ser Leu Lys Pro Glu Gln Val Ser Pro Ser
               340                 345                 350
   Ala His Ser His Ala Thr Ser Pro Leu Glu Glu Phe Lys Arg Ala Ala
           355                 360                 365
   Val Pro Arg Phe Val Gln Gln Lys Leu Tyr Leu Phe Leu Gln His Cys
       370                 375                 380
   Phe Gly His Trp Pro Leu Asp Ala Ser Phe Arg Ala Val Leu Glu Met
   385                 390                 395                 400
   Trp Leu Ser Tyr Leu Gln Pro Trp Arg Tyr Ala Pro Glu Lys Pro Ala
                   405                 410                 415
   Pro Ser Ser Asp Cys Gln Ala Arg Cys Val Ser Glu Arg Trp Ala Pro
               420                 425                 430
   Phe Val Gln Glu Asn Leu Leu Met Tyr Thr Lys Leu Phe Val Gly Phe
           435                 440                 445
   Leu Ser Arg Ala Leu Arg Thr Asp Leu Val Ser Pro Lys Asn Ala Leu
       450                 455                 460
   Met Val Phe Arg Val Ala Lys Val Phe Ala Gln Pro Asn Leu Ala Glu
   465                 470                 475                 480
   Met Ile Gln Lys Gly Glu Gln Leu Phe Leu Glu Pro Glu Leu Val Ile
                   485                 490                 495
   Pro His Arg Gln His Arg Leu Phe Thr Ala Pro Thr Phe Thr Gly Ser
               500                 505                 510
   Phe Leu Ser Ser Trp Pro Pro Ala Val Thr Asp Thr Ser Phe Arg Val
   515 520 525
   Lys Ser His Val Tyr Ser Leu Glu Gly Gln Asp Cys Lys Tyr Thr Pro
   530 535 540
   Met Phe Gly Pro Glu Val Arg Thr Leu Val Leu Arg Leu Ala Gln Leu
   545 550 555 560
   Ile Thr Gln Ala Lys Gln Thr Ala Lys Ser Ile Ser Asp Gln Cys Gly
   565 570 575
   Glu Ser Thr Pro Gly His Pro Phe Leu Ser Trp Leu Gly Phe Cys Ser
   580 585 590
   Thr Asp Thr Asn Gly Ser Tyr Pro Ala Asn Asp Leu Asp Glu Met Gly
   595 600 605
   Gln Asp Ser Val Arg Lys Thr Asp Glu Tyr Leu Glu Lys Ala Leu Glu
   610 615 620
   Tyr Leu Cys Gln Met Phe Arg Leu Ser Glu Ala Gln Leu Ala Gln Leu
   625 630 635 640
   Thr Leu Ala Leu Gly Thr Thr Gln Asp Glu Asn Gly Lys Lys Gln Leu
   645 650 655
   Pro Asp Cys Ile Val Gly Glu Asp Gly Leu Ile Leu Thr Pro Leu Gly
   660 665 670
   Arg Tyr Gln Ile Ile Asn Gly Leu Arg Lys Phe Asp Ile Glu Tyr Gln
   675 680 685
   Gly Asp Leu Glu Leu Gln Pro Ile Arg Ser Tyr Glu Ile Ala Ser Leu
   690 695 700
   Val Arg Met Leu Phe Arg Leu Ser Ser Ala Ile Asn Cys Arg Phe Ala
   705 710 715 720
   Gly Gln Met Ala Ala Leu Cys Ser Arg Ala Asp Phe Leu Gly Ser Phe
   725 730 735
   Cys Arg Tyr His Leu Thr Glu Pro Gly Val Thr Gly Arg His Leu Leu
   740 745 750
   Ser Pro Val Ala Arg Glu Ser Ala Ala Ser Arg Ala Arg Gly Pro Arg
   755 760 765
   Leu Ser Leu Arg Phe Leu Gly Ser Tyr Arg Thr Leu Leu Ser Leu Leu
       770                 775                 780
   Leu Ala Phe Phe Val Ala Ser Leu Phe Cys Ile Gly Pro Leu Pro Cys
   785                 790                 795                 800
   Ala Leu Leu Leu Met Leu Gly Tyr Leu Leu Tyr Ala Val Ala Met Thr
                   805                 810                 815
   Leu Leu Thr Glu Arg Ser Lys Leu His Gln Leu
               820                 825
<210> 5
   <211> 823
   <212> PRT
   <213> Mus musculus
<400> 5
   Met Ala Phe Pro His Leu Gln Gln Pro Ser Phe Leu Leu Ala Ser Leu
   1               5                   10                  15
   Lys Ala Asp Ser Ile Asn Lys Pro Phe Ala Gln Arg Cys Gln Asp Leu
               20                  25                  30
   Val Lys Val Ile Glu Asp Phe Pro Ala Lys Glu Leu His Ala Val Phe
           35                  40                  45
   Pro Trp Leu Val Glu Ser Ile Phe Gly Ser Leu Asp Gly Val Leu Val
       50                  55                  60
   Gly Trp Asn Leu Arg Cys Leu Gln Gly Arg Val Asn Pro Val Glu Tyr
   65                  70                  75                  80
   Ser Thr Ala Met Glu Phe Leu Asp Pro Ser Gly Pro Met Met Lys Leu
                   85                  90                  95
   Val Tyr Lys Leu Gln Ala Glu Asp Tyr Asn Phe Asp Phe Pro Val Ser
               100                 105                 110
   Cys Leu Pro Gly Pro Val Lys Ala Ser Ile Gln Glu Asn Val Leu Pro
           115                 120                 125
   Asp Ser Pro Leu Tyr His Asn Lys Val Gln Phe Pro Pro Thr Gly Gly
       130                 135                 140
   Leu Gly Leu Asn Leu Ala Leu Asn Pro Phe Glu Tyr Tyr Met Phe Tyr
   145                 150                 155                 160
   Phe Ala Leu Ser Leu Ile Ser Gln Lys Pro Met Ser Met Thr Leu His
                   165                 170                 175
   Val Arg Thr Ser Asp Cys Ala Tyr Phe Thr Leu Val Asp Arg Tyr Leu
               180                 185                 190
   Ser Trp Phe Leu Pro Thr Glu Gly Ser Val Pro Pro Pro Leu Cys Ser
           195                 200                 205
   Ser Pro Gly Gly Ser Ser Pro Ser Pro Ala Pro Arg Thr Pro Ala Met
       210                 215                 220
   Pro Phe Ala Ser Tyr Gly Leu His Thr Ser Leu Leu Lys Arg His Ile
   225                 230                 235                 240
   Ser His Gln Thr Ser Val Asn Ala Asp Pro Ala Ser His Glu Ile Trp
                   245                 250                 255
   Arg Ser Glu Thr Leu Leu Gln Val Phe Val Glu Met Trp Leu His His
               260                 265                 270
   Tyr Ser Leu Glu Met Tyr Gln Lys Met Gln Ser Pro His Ala Lys Leu
           275                 280                 285
   Glu Val Leu His Tyr Arg Leu Thr Val Ser Ser Ala Leu His Ser Pro
       290                 295                 300
   Ala Gln Pro Ser Leu Gln Ala Leu His Ala Tyr Gln Glu Ser Phe Thr
   305                 310                 315                 320
   Pro Thr Glu Glu His Val Leu Val Val Arg Leu Leu Leu Lys His Leu
                   325                 330                 335
   His Ala Phe Ala Asn Ser Leu Lys Pro Asp Gln Ala Ser Pro Ser Ala
               340                 345                 350
   His Ser His Ala Thr Ser Pro Leu Glu Glu Phe Lys Arg Ala Ala Glu
           355                 360                 365
   Pro Arg Phe Val Gln Gln Lys Leu Tyr Val Phe Leu Gln His Cys Phe
       370                 375                 380
   Gly His Trp Pro Leu Asp Ala Thr Phe Arg Ala Val Leu Glu Met Trp
   385                 390                 395                 400
   Leu Ser Tyr Leu Gln Pro Trp Arg Tyr Ala Pro Glu Lys Gln Ala Gln
                   405                 410                 415
   Gly Ser Asp Pro Gln Pro Arg Cys Val Ser Glu Lys Trp Ala Pro Phe
               420                 425                 430
   Ile Gln Glu Asn Leu Leu Met Tyr Thr Lys Leu Phe Val Ser Phe Leu
           435                 440                 445
   Asn Arg Ala Leu Arg Thr Asp Leu Val Ser Pro Lys Asn Ala Leu Met
       450                 455                 460
   Val Phe Arg Val Ala Lys Val Phe Ala Gln Pro Asn Leu Ala Glu Met
   465                 470                 475                 480
   Ile Gln Lys Gly Glu Gln Leu Phe Leu Glu Pro Glu Leu Ile Ile Pro
                   485                 490                 495
   His Arg Gln His Arg Leu Phe Thr Val Thr Thr Ser Phe Leu Ser Pro
               500                 505                 510
   Trp Pro Pro Val Val Thr Asp Ala Ser Phe Lys Val Lys Ser His Val
           515                 520                 525
   Tyr Ser Leu Glu Gly Gln Asp Cys Lys Tyr Thr Pro Met Phe Gly Pro
       530                 535                 540
   Glu Ile Arg Thr Leu Val Leu Arg Leu Ala Gln Leu Ile Thr Gln Ala
   545                 550                 555                 560
   Lys Gln Thr Ala Lys Ser Ile Ser Asp Gln Tyr Val Glu Ser Pro Thr
                   565                 570                 575
   Gly Arg Ser Phe Leu Ser Trp Leu Thr Phe Gly Leu Thr Asp Thr Asn
               580                 585                 590
   Ser Cys Tyr Pro Ala Asn Asp Leu Asp Glu Ile Gly Gln Asp Ser Ile
           595                 600                 605
   Arg Lys Thr Asp Glu Tyr Leu Glu Lys Ala Leu Glu Tyr Leu Arg Gln
       610                 615                 620
   Ile Phe Arg Leu Ser Glu Ala Gln Leu Ala Gln Leu Thr Leu Ala Leu
   625                 630                 635                 640
   Gly Ser Ala Arg Asp Glu Asn Gly Lys Gln Gln Leu Pro Asp Cys Ile
                   645                 650                 655
   Val Gly Glu Glu Gly Leu Ile Leu Thr Pro Leu Gly Arg Tyr Gln Ile
               660                 665                 670
   Ile Asn Gly Leu Arg Arg Phe Glu Ile Glu Tyr Gln Gly Asp Leu Glu
   675 680 685
   Leu Gln Pro Ile Arg Ser Tyr Glu Ile Thr Ser Leu Val Arg Ala Leu
   690 695 700
   Phe Arg Leu Ser Ser Ala Ile Asn Arg Arg Phe Ala Gly Gln Met Ala
   705 710 715 720
   Ala Leu Cys Ser Arg Asn Asp Phe Leu Gly Ser Phe Cys Arg Tyr His
   725 730 735
   Leu Thr Glu Pro Ala Leu Ser Asn Arg His Leu Leu Ser Pro Val Gly
   740 745 750
   Arg Arg Gln Val Thr Asn Pro Ala Arg Gly Pro Arg Leu Ser Leu Arg
   755 760 765
   Phe Leu Gly Ser Tyr Arg Thr Leu Leu Leu Leu Leu Met Ala Phe Phe
   770 775 780
   Val Ala Ser Leu Phe Cys Ile Gly Pro Leu Ser Cys Ser Leu Leu Leu
   785 790 795 800
   Val Leu Gly Tyr Val Leu Tyr Ala Ile Ala Met Thr Leu Leu Thr Glu
   805 810 815
   Arg Gly Lys Leu His Gln Leu
   820
<210> 6
   <211> 851
   <212> PRT
   <213> Gallus gallus
<400> 6
   Met Asn Asp Asp Ala Asn Glu Ser Ala Thr Ala Glu Ala Arg Arg Glu
   1               5                   10                  15
   Ala Arg Leu Gly Asp Met Ala Gly Pro Tyr Leu Gln Gln Pro Ser Phe
               20                  25                  30
   Leu Leu Ala Thr Leu Lys Ala Asp Cys Val Asn Lys Pro Phe Ala Gln
           35                  40                  45
   Arg Cys His Asp Leu Glu Thr Val Ile Glu Glu Phe Pro Ala Lys Glu
       50                  55                  60
   Leu His Gly Ile Phe Pro Trp Leu Val Glu Ser Ile Phe Gly Ser Leu
   65                  70                  75                  80
   Asp Gly Thr Ile Val Gly Trp Asn Leu Arg Cys Leu Gln Gly Arg Thr
                   85                  90                  95
   Asn Pro Thr Glu Tyr Ser Val Ala Leu Asp Phe Leu Asp Pro Ser Gly
               100                 105                 110
   Pro Met Met Lys Leu Val Tyr Lys Leu Gln Ala Glu Glu Tyr Arg Tyr
           115                 120                 125
   Asp Phe Pro Val Ser Tyr Leu Pro Gly Pro Val Lys Ala Ser Ile Gln
       130                 135                 140
   Glu Gln Val Leu Pro Glu Cys Ser Leu Tyr His Asn Lys Val Gln Phe
   145                 150                 155                 160
   Pro Ser Ser Gly Gly Leu Gly Leu Asn Leu Ala Leu Asn Pro Phe Glu
                   165                 170                 175
   Tyr Tyr Met Phe Tyr Phe Ala Ile Ser Leu Ile Thr Gln Lys Asn Ser
               180                 185                 190
   Pro Ile Thr His His Ile Ser Pro Ser Asn Ser Ala Tyr Phe Ile Leu
           195                 200                 205
   Val Asp Thr Tyr Leu Lys Cys Phe Leu Pro Thr Glu Gly Ser Val Leu
       210                 215                 220
   Pro Pro Pro Ser Ser Asn Pro Gly Gly Ala Ile Pro Ser Pro Thr Pro
   225                 230                 235                 240
   Arg Ser Pro Ala Val Pro Phe Thr Ser Tyr Gly Met His His Thr Ser
                   245                 250                 255
   Leu Leu Lys Arg His Ile Ala His Gln Pro Ser Val Asn Ala Asp Pro
               260                 265                 270
   Ala Ser Gln Glu Ile Trp Arg Ser Glu Thr Leu Leu Gln Ile Phe Val
           275                 280                 285
   Glu Met Trp Leu His His Tyr Ser Leu Glu Met Tyr Gln Lys Met Gln
       290                 295                 300
   Ser Pro His Ile Lys Leu Glu Val Leu His Tyr Arg Leu Ser Ile Ser
   305                 310                 315                 320
   Ser Lys His His Gly Ser Pro Ala Gln Ser Ser Tyr Gln Ala Leu His
                   325                 330                 335
   Ala Tyr Gln Glu Ser Phe Lys Pro Thr Glu Glu His Val Leu Val Val
   340 345 350
   Arg Leu Leu Val Lys His Leu His Ala Phe Ser Asn Ser Leu Lys Pro
   355 360 365
   Glu Pro Leu Ser Pro Ser Ala His Ser His Thr Ala Ser Pro Leu Glu
   370 375 380
   Glu Phe Lys Arg Val Val Ile Pro Arg Phe Val Gln Gln Lys Leu Tyr
   385 390 395 400
   Ile Phe Leu Gln His Cys Phe Gly His Trp Pro Leu Asp Ala Ser Phe
   405 410 415
   Arg Ala Val Leu Glu Met Trp Leu Ser Tyr Leu Gln Pro Trp Arg Tyr
   420 425 430
   Ala Pro Glu Lys Pro Pro Gln Thr Ala Glu Leu Leu Pro Arg Ser Val
   435 440 445
   Ser Glu Lys Trp Ala Ala Phe Ile Gln Glu Asn Leu Leu Met Tyr Thr
   450 455 460
   Lys Leu Phe Val Gly Phe Leu Asn Arg Thr Leu Arg Thr Asp Leu Val
   465 470 475 480
   Ser Pro Lys Asn Ala Leu Met Val Phe Arg Val Ala Lys Val Phe Ala
   485 490 495
   Gln Pro Asn Leu Ala Glu Met Ile Leu Lys Gly Glu Gln Leu Phe Leu
   500 505 510
   Glu Pro Glu Leu Val Ile Pro His Arg Gln His Arg Leu Phe Met Thr
   515 520 525
   Pro Thr Leu Gly Gly Ser Phe Leu Ser Ser Trp Pro Pro Ala Ile Thr
   530 535 540
   Asp Ala Ser Phe Lys Val Lys Ser His Val Tyr Ser Leu Glu Gly Gln
   545 550 555 560
   Asp Phe Gln Tyr Lys Gln Met Phe Gly Thr Glu Val Arg Asn Leu Val
   565 570 575
   Leu Lys Leu Ala Gln Leu Ile Cys Gln Ala Gln Gln Thr Ala Lys Ser
   580 585 590
   Ile Ser Asp His Ser Ala Glu Thr Thr Ala Ser His Ser Phe Phe Ser
           595                 600                 605
   Trp Phe Lys Phe Thr Pro Ser Glu Met Asn Gly Ser Tyr Thr Gly Asn
       610                 615                 620
   Asp Leu Asp Glu Ile Gly Gln Asp Ser Ile Lys Lys Thr Asp Glu Tyr
   625                 630                 635                 640
   Leu Glu Lys Ala Leu Glu Tyr Leu Cys Gln Ile Phe Lys Leu Asn Glu
                   645                 650                 655
   Ala Gln Leu Thr Gln Met Met Met Lys Cys Gly Thr Ala Gln Asp Glu
               660                 665                 670
   Asn Gly Lys Lys Gln Leu Pro Asp Cys Ile Glu Ser Glu Asn Gly Leu
           675                 680                 685
   Ile Leu Thr Pro Leu Gly Arg Tyr Gln Met Ile Asn Gly Leu Arg Arg
       690                 695                 700
   Phe Asp Val Gln Tyr Gln Gly Asp Thr Glu Leu Gln Pro Ile Arg Ser
   705                 710                 715                 720
   Tyr Glu Asn Ala Thr Leu Val Arg Leu Leu Tyr Arg Leu Ser Ser Ala
                   725                 730                 735
   Leu Asn Glu Arg Leu Ala Asp Gln Met Asp Val Leu Cys Ser Arg Glu
               740                 745                 750
   Asp Phe Val Gly Arg Phe Cys Arg Tyr His Leu Thr Asn Pro His Leu
           755                 760                 765
   Val His Lys Ile Arg Tyr Ser Pro Val Leu Lys Asp Arg Thr Ser Gln
       770                 775                 780
   Asn Trp Gly Pro Arg Ile Ser Leu Arg Phe Leu Ala Ser Tyr Arg Thr
   785                 790                 795                 800
   Leu Ile Ser Leu Leu Met Leu Tyr Phe Ile Ala Ser Trp Phe His Ile
                   805                 810                 815
   Gly Pro Arg Leu His Ser Phe Gln Lys Leu Ser Leu Phe Met Pro Asn
               820                 825                 830
   His Phe Ser Ala Asn Gln Lys Pro Val Pro Leu Ile Phe Asp Lys Val
           835                 840                 845
   Pro Lys His
   850
<210> 7
   <211> 791
   <212> PRT
   <213> Danio rerio
<400> 7
   Met Ala Ala Ser Ala Leu Gln Gln Pro Ser Tyr Leu Leu Ala Asn Leu
   1               5                   10                  15
   Lys Ala Asp Trp Thr Asn Lys Pro Leu His Gln Arg Cys His Glu Leu
               20                  25                  30
   Cys Lys Ile Ile Asp Asp Tyr Pro Ala Lys Glu Leu His Ala Ile Phe
           35                  40                  45
   Pro Trp Leu Val Glu Cys Val Phe Gly Ser Leu Asp Gly Ile Leu Thr
       50                  55                  60
   Gly Trp Asn Leu Arg Phe Leu Gln Ala Arg Ser Ala Glu Tyr Ser Ile
   65                  70                  75                  80
   Ala Met Glu Phe Leu Asp Pro Ser Gly Pro Met Met Lys Leu Val Tyr
                   85                  90                  95
   Lys Leu Gln Ala Glu Glu Tyr Lys Tyr Glu Phe Pro Ile Ser Tyr Leu
               100                 105                 110
   Pro Gly Pro Ile Lys Ser Ser Ile His Ala Gly Val Leu Pro Asp Cys
           115                 120                 125
   Pro Leu Phe His Asn Lys Ile Gln Phe Pro Met Ser Gly Leu Leu Phe
       130                 135                 140
   Leu Asn Pro Phe Glu Tyr Tyr Met Phe Asn Phe Ala Ser Ser Leu Ile
   145                 150                 155                 160
   Ala Pro Lys Asn Tyr Pro Gln Gly Gln His Gly Ser Ser Ser Asp Ser
                   165                 170                 175
   Ala Tyr Phe Val Leu Val Asp Thr Tyr Leu Lys Tyr Phe Leu Pro Thr
               180                 185                 190
   Glu Gly Asn Val Pro Pro Ser Pro Phe Ser Asp Thr Arg Gly Thr Val
           195                 200                 205
   Ala Ser Pro Ala Pro Arg Ser Thr Asn Val Pro Tyr Val Gly Tyr Gly
   210 215 220
   Gly His Ser Thr Ser Leu Leu Lys Arg His Ile Thr His Gln Ser Ser
   225 230 235 240
   Val Asn Ala Asp Pro Ala Ala Gln Glu Ile Trp Arg Ser Glu Thr Leu
   245 250 255
   Leu Gln Val Phe Val Glu Met Trp Leu His His Tyr Ser Leu Glu Met
   260 265 270
   Tyr Gln Lys Leu Gln Ser Pro Gln Val Lys Glu Pro Phe Met Pro Ser
   275 280 285
   Glu Glu His Val Leu Val Val Arg Leu Leu Val Lys His Leu His Thr
   290 295 300
   Phe Ser Ser Ser Leu Lys Pro Glu Ser Ile Ser Ser Ser Pro Ser Ala
   305 310 315 320
   His Ser His Ser Ser Pro Leu Glu Glu Leu Lys Arg Val Val Val Gln
   325 330 335
   Arg Phe Val Gln Gln Lys Leu Tyr Val Phe Leu Gln His Cys Phe Gly
   340 345 350
   His Trp Pro Leu Asp Ala Ser Phe Arg Ala Val Leu Glu Thr Trp Leu
   355 360 365
   Ser Tyr Ile Gln Pro Trp Arg Tyr Thr Gly Asp Lys Asn Asn Thr Gln
   370 375 380
   Thr Asp Gly Pro Asn Arg Thr Val Pro Asp Lys Trp Ala Ser Phe Val
   385 390 395 400
   Gln Glu Asn Leu Leu Leu Tyr Thr Lys Leu Phe Gln Gly Phe Leu Asn
   405 410 415
   Arg Ala Met Arg Thr Asp Leu Val Asn Ala Lys Asn Ala Leu Met Val
   420 425 430
   Phe Arg Val Ala Lys Val Phe Ala Gln Pro Ser Leu Ser Glu Met Ile
   435 440 445
   Gln Lys Gly Glu Gln Leu Phe Leu Glu Pro Glu His Ala Ile Leu Gln
   450 455 460
   Arg His Asn Arg Val Phe Leu Thr Pro Ser His Gly Gly Ser Phe Leu
   465                 470                 475                 480
   Ser Ala Arg Gln Pro Met Gly Thr Asp Asn Val Phe Lys Val Lys Ser
                   485                 490                 495
   His Val Tyr Ser Leu Glu Gly Gln Asp Cys Gln Tyr Asn Leu Met Phe
               500                 505                 510
   Gly Pro Asp Gln Arg Lys Asn Val Leu Lys Leu Ile Gln Ile Ile Ala
           515                 520                 525
   Gln Ala Arg Gln Thr Ala Lys Arg Ile Ser Asp His Ser Thr Glu Met
       530                 535                 540
   Ala Ala Asn Asn Ser Phe Leu Ser Trp Phe Gly Val Gly Ser Pro Asp
   545                 550                 555                 560
   His Asn Ser Thr Phe Thr Gly Gly Glu Met Asp Glu Met Gly Gly Glu
                   565                 570                 575
   Gly Val Lys Lys Thr His Glu Phe Leu Asp Lys Ala Leu Asp Tyr Leu
               580                 585                 590
   Cys Gln Ile Phe Arg Leu Asn Ala Gly Gln Leu Ser Gln Leu Ile Ser
           595                 600                 605
   Asn Val Ala Ser Val Asp Asn Asn Gly Ala Ser Lys Gln Leu Pro Asp
       610                 615                 620
   Cys Ile Pro Ser Glu Asn Gly Leu Val Leu Thr Asp Leu Gly Arg Leu
   625                 630                 635                 640
   Gln Ile Ile Asn Gly Leu Arg Arg Phe Glu Ile Glu Tyr Gln Gly Asp
                   645                 650                 655
   Pro Glu Leu Gln Pro Ile Arg Ser Tyr Glu Asn Ala Phe Leu Val Arg
               660                 665                 670
   Leu Leu Phe Gln Ile Ser Ser Phe Ile Asn Glu Arg Leu Gly Glu His
           675                 680                 685
   Met Glu Val Leu Cys Ser Arg Gln Asp Phe Leu Gly Ser Val Gly Arg
       690                 695                 700
   His Tyr Leu Ser Ser Ser Ser Ala Val Val Glu Gln Arg Arg Lys Ser
   705                 710                 715                 720
   Pro Val Thr Arg Gln Met Arg Asp Arg Pro Gln Arg Ala Arg Leu Ser
                   725                     730                 735
   Leu Arg Ala Leu Ala Ser Tyr Arg Thr Leu Leu Thr Leu Leu Leu Leu
               740                 745                 750
   Tyr Met Leu Phe Ala Leu Leu Ser Phe Gly Leu Phe Ser Ser Thr Gly
           755                 760                 765
   Leu Ile Leu Ile Ile Ser Phe Leu Tyr Glu Leu Leu Ser Asn Phe Phe
       770                 775                 780
   His Glu Lys Leu Lys Thr His
   785                 790
<210> 8
   <211> 767
   <212> PRT
   <213> Drosophila melanogaster
<400> 8
   Met Ser Gln Thr Met Pro Pro Glu Asn Leu Ser Ser Arg Leu Leu Thr
   1               5                   10                  15
   Val Leu Ser Leu Pro Leu Phe Glu Arg Val His Glu Leu Ser Ile Leu
               20                  25                  30
   Phe Asp Arg Cys Ser Leu Arg Gln Val Gln Glu Ile Phe Pro His Val
           35                  40                  45
   Val His Ser Ile Phe Gly Ile Asp Gly Asn Pro Leu Gly Trp Gly Leu
       50                  55                  60
   Arg Thr Thr Thr Leu Glu Asn Asn Pro Val Gln Phe Gln Thr Leu His
   65                  70                  75                  80
   Gln Phe Phe Gly Val Cys Gly Pro Leu Met His Val Cys His Arg Leu
                   85                  90                  95
   Leu Val Asp Gln Tyr Lys Phe Glu Leu Asp Ile Asn Leu Leu Pro Ala
               100                 105                 110
   Lys Phe Val Ser Leu Leu Gln Asn Gly Gln Asn Pro Ser Phe Tyr Ala
           115                 120                 125
   Glu Leu Ile Asn Val Glu Thr Met Met His Gln Val Ser Thr Leu Ser
       130                 135                 140
   Leu Asn Ala Phe Asp Phe Tyr Val Ile His Phe Val Leu Tyr Ala Leu
   145                 150                 155                 160
   Gln Pro Leu His Ser Ile Asn Pro Ile Ala Met Gln Ile His Asn Val
                   165                 170                 175
   Arg Ser Lys Thr Val Tyr Leu Lys Leu Val Ala Glu Tyr Leu Asn Asn
               180                 185                 190
   Phe Leu Pro Leu Leu Pro Asp Ala Arg Ile Glu Pro Val His Phe Ser
           195                 200                 205
   Ser Gly Val Lys Ala Pro Gln Pro Leu Pro Ala Gln Ala Leu Gln Pro
       210                 215                 220
   Gln Arg Gln Pro Arg Tyr Ile Lys Ile Pro Ser Ser Tyr Arg Asn Gly
   225                 230                 235                 240
   Gly Asn Val Ser Gly Gly Gly Gly Ile Pro Ser Ala Ser Val Gly Val
                   245                 250                 255
   Gly Asn Ile Ser Pro Gln Ser Asn Ser Pro Ser Ala Ser Ser Asn Arg
               260                 265                 270
   Ala Tyr Ala Trp Arg Ser Glu Ser Val Leu His Phe Phe Val Asp Ile
           275                 280                 285
   Trp Leu Arg Tyr Asp Ile Glu Ser Glu His His Leu Pro Ser Ser Asp
       290                 295                 300
   Phe Val Arg Gly Val Arg Thr Leu Val Lys Gln Val His Phe Phe Ala
   305                 310                 315                 320
   Asn Gly Ala Gln His Asp His Ser Ser Leu Cys Ala Leu Arg Lys Val
                   325                 330                 335
   Ser Leu Ser Met Val Lys Ala Arg Ile Tyr Ala Phe Ile Val Gly Leu
               340                 345                 350
   Ile Asp Arg Trp Pro Leu Asp Ser Ser Leu Met Val Val Leu Glu Leu
           355                 360                 365
   Trp Leu Ser Tyr Ile Gln Pro Trp Arg Tyr Thr Ile Ala Ser Leu Asn
       370                 375                 380
   Asn Lys Thr Pro Ser Leu Ser Tyr Arg Pro Pro Ile Ser Ser Cys Phe
   385                 390                 395                 400
   Asp Gly Phe Ile Ile Asp Asn Leu Ile Met Tyr Thr His Ile Phe Met
   405 410 415
   Gln Leu Leu Pro His Phe Gly Arg Leu Asp Tyr Thr Val Tyr Arg Asn
   420 425 430
   Ala Phe Met Leu Tyr Arg Leu Ala Thr Ile Phe Ser Gln His Asp Leu
   435 440 445
   Val Asp Lys Leu Gln Arg Phe Glu Ala Ile His Ala Gly Asn Ser Phe
   450 455 460
   Gly Phe Asp Ser Pro Gln Arg Gln Val Asn Ile Met Asn Lys Ser Val
   465 470 475 480
   Ser Pro Gly Thr Pro Trp Asn Gln Ala Val Asn Ile Ser Ser Ala Lys
   485 490 495
   Leu Phe Ser Tyr Thr Met Gln Thr Gln Met Glu Thr Phe Leu Leu Leu
   500 505 510
   Ile Ser Met Ala Arg Asn Ser Val Leu Arg Asp Ile Ala Lys Leu Arg
   515 520 525
   Asn Glu Ile Ala Glu Arg Gln Arg Ser Glu Gly Phe Leu Lys Asn Phe
   530 535 540
   Tyr Lys Lys Ile Phe Gly Glu Cys Thr Gln Glu Glu Val Thr Leu Arg
   545 550 555 560
   Glu Phe Ser Arg Ile Pro Glu Val Leu Arg Gln Cys Ile Asp Ala Phe
   565 570 575
   Cys Arg Thr Phe Asn Val Asp Gln Ala Asn Leu Ser Met His Glu Thr
   580 585 590
   Leu Pro Glu Glu Pro Ser Val Pro Ser Ala Pro Ala Val Gln Asn Phe
   595 600 605
   Ser Phe Phe Asp Ala Ser Asp Thr Leu Asp Thr Ser Lys Leu Asn Pro
   610 615 620
   His Gln Met Ser Leu Asn Ala Ser Ser Leu His Ala Ala Val Asp Pro
   625 630 635 640
   Ala Thr Leu Pro Ile Gln Thr Asn Glu Val Arg Ala Leu Val Arg Met
   645 650 655
   Leu His Phe Val Ser Glu Lys Ile Asn Asn Lys Tyr Gly Ser Gln Leu
               660                 665                 670
   Gln Gly Phe Tyr Val Arg Asp Asp Tyr Phe Gly Lys Val Ala Arg Gln
           675                 680                 685
   Leu Leu Tyr Ala Pro Met Thr Glu Gln Trp Phe Asp Lys Ser Ser Gly
       690                 695                 700
   His Val Glu Ile Cys Glu Asn Leu Val Pro Pro Arg Val Cys Leu Arg
   705                 710                 715                 720
   Pro Leu Gly Ser Ile Pro Ala Leu Thr Thr Ile Gly Cys Ser Met Ile
                   725                 730                 735
   Phe Gly Gln Leu Val Trp Gly Ala Pro Leu Leu Gly Ile Phe Ile Leu
               740                 745                 750
   Ala Thr Val Leu Leu Val Tyr Ile Met Leu Gln Ala Leu Phe Ser
           755                 760                 765
<210> 9
   <211> 640
   <212> PRT
   <213> Caenorhabditis elegans
<400> 9
   Met Phe Gln Gln Arg Gln Thr Pro Thr Glu Phe Ser Gln Met Asp Asp
   1               5                   10                  15
   Val Val Ser Ile Cys Arg Glu Ile Ser Ser Asn Ile Ile Ser Thr Asp
               20                  25                  30
   Lys Asn Val Pro Ile Ser Lys Met Ile Ile His Lys Ile Phe Lys Asn
           35                  40                  45
   Pro His Ile Asp Leu Cys Ser Ile Leu Pro Pro Leu Ile Gln Gln His
       50                  55                  60
   Tyr Pro Asp Ser Pro Tyr Cys His Ile Gln Ser Leu Ile Asn Ser Asn
   65                  70                  75                  80
   Ser Val Phe Phe Glu Thr Val Leu Glu Asp Asp Gln Leu Cys Thr Glu
                   85                  90                  95
   Leu Trp Asp Ser Ile Leu His Gln Cys Glu Val Ser Thr Ala Pro Thr
               100                 105                 110
   Glu Pro Ala Lys Asp Ser Ile Leu Phe Ser Ala Val Ser Gln Tyr Val
           115                 120                 125
   Thr Arg Ile Leu Pro Cys Asp Glu Thr Ala Pro Ser Ser Met Ser Val
       130                 135                 140
   Pro Arg Ser Pro Lys Ile Thr His Gln Thr Ile Arg Thr Pro Leu Gln
   145                 150                 155                 160
   Leu Phe Arg Ala Asp Ser Pro Leu Val Asn Arg Val Ser Ile Glu Glu
                   165                 170                 175
   Arg Ser Gln Met Met Ser Val Ser Thr Val Ser Phe Gln Pro Arg Leu
               180                 185                 190
   Val Gln Asn Phe Cys Asn Phe Val Thr Lys Ser Cys Thr Thr Ala Gln
           195                 200                 205
   Phe Thr Asn Ala Asn Lys Leu Leu Leu Ile Arg Tyr Leu Leu Lys Gln
       210                 215                 220
   Phe His Val Phe Cys Gln Phe Ser Thr Gly Asp Pro Glu Val Asp Asn
   225                 230                 235                 240
   Trp Lys Leu Asp Leu Met Ala Ser Ala Pro Phe Arg Phe Ser Leu Tyr
                   245                 250                 255
   Asp Phe Phe Lys Ser Ser Val Thr Gln Val Pro Thr Thr Ile Val Phe
               260                 265                 270
   Arg Asp Ile Thr Met Cys Trp Leu Thr Tyr Cys Arg Pro Trp Arg Tyr
           275                 280                 285
   His Asn Met Ser Asn Pro Ser Asp Phe Ala Pro Ala Ala Lys Tyr Arg
       290                 295                 300
   Gln Phe Phe Glu Lys Asn Val Glu Phe Tyr Glu Val Ile Leu Gly Lys
   305                 310                 315                 320
   Ile Leu Lys Arg Phe Ser Ala Phe Glu Met Cys Glu Glu Leu Leu Gly
                   325                 330                 335
   Ser Leu Arg Ala Ile Ile Glu Phe Ala Trp Lys Glu Pro Gln Thr Leu
               340                 345                 350
   Leu Leu Arg His Ile Gln Leu Asp Ile Gln Pro His Ser Leu Glu Leu
           355                 360                 365
   Leu Lys Gln Met Gln Val Val Val Arg Met His Arg Ser Ala Ile Gln
   370 375 380
   Lys Glu Gln Asp Glu His Ser Gly Phe Trp Asn Ser Leu Phe Tyr Ser
   385 390 395 400
   Gly Asp Ser Ala Arg Thr Gln Ser Ser Arg Arg Leu Val Ser Glu Leu
   405 410 415
   Leu Thr Leu Met Lys Asp Ser Asp Ser Tyr Val Gly Thr His Phe Met
   420 425 430
   Pro Glu Met Glu Asp Ser Val Leu Ala Asn Arg Thr Gly Asn Glu Thr
   435 440 445
   Phe His Lys Glu Lys Leu Tyr Thr Ser Leu Leu Asn Glu Thr Pro Lys
   450 455 460
   Ser Gly Leu Gly Ile Pro Asp His Phe Val Asp Gly Pro Ser Asn His
   465 470 475 480
   Met Leu Leu Thr Pro Ile Gly Gln Arg Gln Val Leu Asn Arg Glu Lys
   485 490 495
   Arg Phe Asp Tyr Ser Asn Cys Ile Lys Asp Asp Pro Lys Ala Pro Thr
   500 505 510
   Arg Ser Tyr Glu Leu Gly Ile Ala Val Lys Leu Thr Thr Ser Leu Ala
   515 520 525
   Gln Lys Leu Asn Glu Leu Ser Phe Val Gln Lys Ile Gly Asp His Tyr
   530 535 540
   Ser Gln Gln Ser Val Ile Gly Ala Ile Ala Arg Lys Val Met Tyr Pro
   545 550 555 560
   Pro Val Pro Asn Leu Asp Pro Asn Ala Thr Val Pro Ala Tyr Ser Ala
   565 570 575
   Arg Ile Ile Arg Ser Pro Pro Leu Leu Arg Leu Arg Tyr Phe Ala Ser
   580 585 590
   Tyr Gln Ser Ile Phe Phe Met Ile Trp Leu Leu Phe Ala Leu Lys Tyr
   595 600 605
   Gly Leu Thr Phe Thr Ile Val Ser Leu Ile Leu Ile Ala Leu Ser Val
   610 615 620
   Thr Val Leu Leu Phe Ile Glu Leu Asp Ser Gly Ser Ser Ser Ser Tyr
   625 630 635 640
<210> 10
   <211> 7303
   <212> DNA
   <213> Artificial
<220>
   <223> pRK-nSMase3
<400> 10
   tatgccaagt acgcccccta ttgacgtcaa tgacggtaaa tggcccgcct ggcattatgc      60
   ccagtacatg accttatggg actttcctac ttggcagtac atctacgtat tagtcatcgc     120
   tattaccatg gtgatgcggt tttggcagta catcaatggg cgtggatagc ggtttgactc     180
   acggggattt ccaagtctcc accccattga cgtcaatggg agtttgtttt ggcaccaaaa     240
   tcaacgggac tttccaaaat gtcgtaacaa ctccgcccca ttgacgcaaa tgggcggtag     300
   gcgtgtacgg tgggaggtct atataagcag agctcgttta gtgaaccgtc agatcgcctg     360
   gagacgccat ccacgctgtt ttgacctcca tagaagacac cgggaccgat ccagcctccg     420
   cggccgggaa cggtgcattg gaacgcggat tccccgtgcc aagagtgacg taagtaccgc     480
   ctatagagtc tataggccca cccccttggc ttcgttagaa cgcggctaca attaatacat     540
   aaccttatgt atcatacaca tacgatttag gtgacactat agaataacat ccactttgcc     600
   tttctctcca caggtgtcca ctcccaggtc caactgcacc tcggttctat cgattgaatt     660
   ctgatgacga ctttcggcgc cgtggcggaa tggcggcttc catctctgag gcgagcgacg     720
   ctatggatcc cacagtggtt tgctaagaag gccattttca actctccact ggaggctgct     780
   atggcgttcc ctcacctgca gcagcccagc tttctactgg ctagcctgaa agctgactct     840
   ataaataagc cctttgcaca gcagtgccaa gacttggtta aagtcattga ggactttcca     900
   gcaaaggagc tgcacaccat cttcccatgg ctggtagaaa gcatttttgg cagcctagat     960
   ggtgtcctcg ttggctggaa cctccgctgc ttacaggggc gcgtgaatcc tgtggagtac    1020
   agcatcgtga tggaatttct cgaccctggt ggcccaatga tgaagttggt ttataagctt    1080
   caagctgaag actataagtt cgactttcct gtctcctact tgcctggtcc tgtgaaggcg    1140
   tccatccagg agtgcatcct ccctgacagt cctctgtacc acaacaaggt ccagttcacc    1200
   cctactgggg gccttggtct gaacttggcc ctgaatccgt tcgagtatta catattcttc    1260
   tttgccttga gcctcatcac tcagaagcca cttcctgtgt ccctccacgt ccgtacttca    1320
   gactgtgcct atttcatcct ggtggacagg tacctgtcat ggttcctgcc caccgaaggc    1380
   agtgtgcccc caccactctc ctccagccca ggggggacca gcccctcacc acctcccagg     1440
   acaccagcca taccctttgc ttcctatggc ctccaccaca ctagcctcct aaagcgacac    1500
   atctctcatc agacgtctgt gaatgcagac cccgcctccc acgagatctg gaggtcagaa    1560
   actctgctcc aggtttttgt tgaaatgtgg cttcatcact attccttgga gatgtatcaa 1620
   aaaatgcagt cccctcatgc caagctggag gttctgcact accgactcag tgtctccagc 1680
   gccctctaca gccccgccca acccagcctc caggccctcc acgcctacca agagtcgttc 1740
   acgcctactg aggagcatgt gttggtggtg cgcctgctgc tgaagcacct gcacgccttt 1800
   gccaacagcc tgaagccaga gcaggcctca ccctccgccc actcccacgc caccagcccc 1860
   ctggaggagt tcaaacgggc tgctgtcccg aggttcgtcc agcagaaact ctacctcttc 1920
   ttgcagcatt gctttggcca ctggcccctg gacgcatcgt tcagagctgt cctggagatg 1980
   tggctgagct acctgcagcc gtggcggtac gcgcctgaca agcaggctcc gggcagcgac 2040
   tcccagcccc ggtgtgtgtc ggagaaatgg gcaccctttg tccaggagaa cctgctgatg 2100
   tacaccaagt tgtttgtggg ctttctgaac cgcgcgctcc gcacagacct ggtcagcccc 2160
   aagcacgcgc tcatggtgtt ccgagtggcc aaagtctttg cccagcccaa cctggctgag 2220
   atgattcaga aaggtgagca gctattcctg gagccagagc tggtcatccc ccaccgccag 2280
   caccgactct tcacggcccc cacattcact gggagcttcc tgtcaccctg gccaccagcg 2340
   gtcactgatg cctccttcaa ggtgaagagc cacgtctaca gcctggaggg ccaggactgc 2400
   aagtacaccc cgatgtttgg gcccgaggcc cgcaccctgg tcctgcgcct cgctcagctc 2460
   atcacacagg ccaaacacac agccaagtcc atctccgacc agtgtgcgga gagcccggct 2520
   ggccactcct tcctctcatg gctgggcttt agctccatgg acaccaatgg ctcctacaca 2580
   gccaacgacc tggacgagat ggggcaagac agtgtccgga agacagatga atacctggag 2640
   aaggccctgg agtacctgcg ccagatattc cggctcagcg aagcgcagct caggcagttc 2700
   acactcgcct tgggcaccac ccaggatgag aatggaaaaa agcaactccc cgactgcatc 2760
   gtgggtgagg acggactcat ccttacgccc ctggggcggt accagatcat caatgggctg 2820
   cgaaggtttg aaattgagta ccagggggac ccggagctgc agcccatccg gagctatgag 2880
   atcgccagct tggtccgcac actctttagg ctgtcgtctg ccatcaacca cagatttgca 2940
   ggacagatgg cggctctgtg ttcccgggat gacttcctcg gcagcttctg tcgctaccac 3000
   ctcacagaac ctgggctggc cagcaggcac ctgctgagcc ctgtggggcg gaggcaggtg 3060
   gccggccaca cccgcggccc caggctcagc ctgcgcttcc tgggcagtta ccggacgctg 3120
   gtctcgctgc tgctggcctt cttcgtggcc tctctgttct gcgtcgggcc cctcccatgc 3180
   acgctgctgc tcaccctggg ctatgtcctc tacgcctctg ccatgacact gctgaccgag 3240
   cgggggaagc tgcaccagcc ctgacgtcga cctgcagaag cttggccgcc atggcccaac 3300
   ttgtttattg cagcttataa tggttacaaa taaagcaata gcatcacaaa tttcacaaat 3360
   aaagcatttt tttcactgca ttctagttgt ggtttgtcca aactcatcaa tgtatcttat 3420
   catgtctgga tcgatcggga attaattcgg cgcagcacca tggcctgaaa taacctctga 3480
   aagaggaact tggttaggta ccttctgagg cggaaagaac cagctgtgga atgtgtgtca 3540
   gttagggtgt ggaaagtccc caggctcccc agcaggcaga agtatgcaaa gcatgcatct 3600
   caattagtca gcaaccaggt gtggaaagtc cccaggctcc ccagcaggca gaactatgca 3660
   aagcatgcat ctcaattagt cagcaaccat agtcccgccc ctaactccgc ccatcccgcc 3720
   cctaactccg cccagttccg cccattctcc gccccatggc tgactaattt tttttattta 3780
   tgcagaggcc gaggccgcct cggcctctga gctattccag aagtagtgag gaggcttttt 3840
   tggaggccta ggcttttgca aaaagctgtt aacagcttgg cactggccgt cgttttacaa 3900
   cgtcgtgact gggaaaaccc tggcgttacc caacttaatc gccttgcagc acatccccct 3960
   ttcgccagct ggcgtaatag cgaagaggcc cgcaccgatc gcccttccca acagttgcgt 4020
   agcctgaatg gcgaatggcg cctgatgcgg tattttctcc ttacgcatct gtgcggtatt 4080
   tcacaccgca tacgtcaaag caaccatagt acgcgccctg tagcggcgca ttaagcgcgg 4140
   cgggtgtggt ggttacgcgc agcgtgaccg ctacacttgc cagcgcccta gcgcccgctc 4200
   ctttcgcttt cttcccttcc tttctcgcca cgttcgccgg ctttccccgt caagctctaa 4260
   atcgggggct ccctttaggg ttccgattta gtgctttacg gcacctcgac cccaaaaaac 4320
   ttgatttggg tgatggttca cgtagtgggc catcgccctg atagacggtt tttcgccctt 4380
   tgacgttgga gtccacgttc tttaatagtg gactcttgtt ccaaactgga acaacactca 4440
   accctatctc gggctattct tttgatttat aagggatttt gccgatttcg gcctattggt 4500
   taaaaaatga gctgatttaa caaaaattta acgcgaattt taacaaaata ttaacgttta 4560
   caattttatg gtgcactctc agtacaatct gctctgatgc cgcatagtta agccaactcc 4620
   gctatcgcta cgtgactggg tcatggctgc gccccgacac ccgccaacac ccgctgacgc 4680
   gccctgccgg gcttgtctgc tcccggcatc cgcttacaga caagctgtga ccgtctccgg 4740
   gagctgcatg tgtcagaggt tttcaccgtc atcaccgaaa cgcgcgaggc agtattcttg 4800
   aagacgaaag ggcctcgtga tacgcctatt tttataggtt aatgtcatga taataatggt 4860
   ttcttagacg tcaggtggca cttttcgggg aaatgtgcgc ggaaccccta tttgtttatt 4920
   tttctaaata cattcaaata tgtatccgct catgagacaa taaccctgat aaatgcttca 4980
   ataatattga aaaaggaaga gtatgagtat tcaacatttc cgtgtcgccc ttattccctt 5040
   ttttgcggca ttttgccttc ctgtttttgc tcacccagaa acgctggtga aagtaaaaga 5100
   tgctgaagat cagttgggtg cacgagtggg ttacatcgaa ctggatctca acagcggtaa 5160
   gatccttgag agttttcgcc ccgaagaacg ttttccaatg atgagcactt ttaaagttct 5220
   gctatgtggc gcggtattat cccgtgatga cgccgggcaa gagcaactcg gtcgccgcat 5280
   acactattct cagaatgact tggttgagta ctcaccagtc acagaaaagc atcttacgga 5340
   tggcatgaca gtaagagaat tatgcagtgc tgccataacc atgagtgata acactgcggc 5400
   caacttactt ctgacaacga tcggaggacc gaaggagcta accgcttttt tgcacaacat   5460
   gggggatcat gtaactcgcc ttgatcgttg ggaaccggag ctgaatgaag ccataccaaa   5520
   cgacgagcgt gacaccacga tgccagcagc aatggcaaca acgttgcgca aactattaac   5580
   tggcgaacta cttactctag cttcccggca acaattaata gactggatgg aggcggataa   5640
   agttgcagga ccacttctgc gctcggccct tccggctggc tggtttattg ctgataaatc   5700
   tggagccggt gagcgtgggt ctcgcggtat cattgcagca ctggggccag atggtaagcc   5760
   ctcccgtatc gtagttatct acacgacggg gagtcaggca actatggatg aacgaaatag   5820
   acagatcgct gagataggtg cctcactgat taagcattgg taactgtcag accaagttta   5880
   ctcatatata ctttagattg atttaaaact tcatttttaa tttaaaagga tctaggtgaa   5940
   gatccttttt gataatctca tgaccaaaat cccttaacgt gagttttcgt tccactgagc   6000
   gtcagacccc gtagaaaaga tcaaaggatc ttcttgagat cctttttttc tgcgcgtaat   6060
   ctgctgcttg caaacaaaaa aaccaccgct accagcggtg gtttgtttgc cggatcaaga   6120
   gctaccaact ctttttccga aggtaactgg cttcagcaga gcgcagatac caaatactgt   6180
   ccttctagtg tagccgtagt taggccacca cttcaagaac tctgtagcac cgcctacata   6240
   cctcgctctg ctaatcctgt taccagtggc tgctgccagt ggcgataagt cgtgtcttac   6300
   cgggttggac tcaagacgat agttaccgga taacgcgcag cggtcgggct gaacgggggg   6360
   ttcgtgcaca cagcccagct tggagcgaac gacctacacc gaactgagat acctacagcc   6420
   tgagcattga gaaagcgcca cgcttcccga agggagaaag gcggacaggt atccggtaag   6480
   cggcagggtc ggaacaggag agcgcacgag ggagcttcca gggggaaacg cctggtatct   6540
   ttatagtcct gtcgggtttc gccacctctg acttgagcgt cgatttttgt gatgctcgtc   6600
   aggggggcgg agcctatgga aaaacgccag caacgcggcc tttttacggt tcctggcctt   6660
   ttgctggcct tttgctcaca tgttctttcc tgcgttatcc cctgattctg tggataaccg   6720
   tattaccgcc tttgagtgag ctgataccgc tcgccgcagc cgaacgaccg agcgcagcga   6780
   gtcagtgagc gaggaagcgg aagagcgccc aatacgcaaa ccgcctctcc ccgcgcgttg   6840
   gccgaatcat taatccagct ggcacgacag gtttcccgac tggaaagcgg gcagtgagcg   6900
   caacgcaatt aatgtgagtt acctcactca ttaggcaccc caggctttac actttatgct   6960
   tccggctcgt atgttgtgtg gaattgtgag cggataacaa tttcacacag gaaacagcta   7020
   tgaccatgat tacgaattaa ttcgagctcg cccgacattg attattgact agttattaat   7080
   agtaatcaat tacggggtca ttagttcata gcccatatat ggagttccgc gttacataac   7140
   ttacggtaaa tggcccgcct ggctgaccgc ccaacgaccc ccgcccattg acgtcaataa   7200
   tgacgtatgt tcccatagta acgccaatag ggactttcca ttgacgtcaa tgggtggagt   7260
   atttacggta aactgcccac ttggcagtac atcaagtgta tca                     7303
<210> 11
   <211> 6678
   <212> DNA
   <213> Artificial
<220>
   <223> pRK-nSMase2
<400> 11
   tatgccaagt acgcccccta ttgacgtcaa tgacggtaaa tggcccgcct ggcattatgc    60
   ccagtacatg accttatggg actttcctac ttggcagtac atctacgtat tagtcatcgc   120
   tattaccatg gtgatgcggt tttggcagta catcaatggg cgtggatagc ggtttgactc   180
   acggggattt ccaagtctcc accccattga cgtcaatggg agtttgtttt ggcaccaaaa   240
   tcaacgggac tttccaaaat gtcgtaacaa ctccgcccca ttgacgcaaa tgggcggtag   300
   gcgtgtacgg tgggaggtct atataagcag agctcgttta gtgaaccgtc agatcgcctg   360
   gagacgccat ccacgctgtt ttgacctcca tagaagacac cgggaccgat ccagcctccg   420
   cggccgggaa cggtgcattg gaacgcggat tccccgtgcc aagagtgacg taagtaccgc   480
   ctatagagtc tataggccca cccccttggc ttcgttagaa cgcggctaca attaatacat   540
   aaccttatgt atcatacaca tacgatttag gtgacactat agaataacat ccactttgcc   600
   tttctctcca caggtgtcca ctcccaggtc caactgcacc tcggttctat cgattgaatt   660
   ccccggggat ccatggtttt gtacacgacc ccctttccta acagctgtct gtccgccctg   720
   cactgtgtgt cctgggccct tatctttcca tgctactggc tggtggaccg gctcgctgcc   780
   tccttcatac ccaccaccta cgagaagcgc cagcgggcag acgacccgtg ctgcctgcag   840
   ctgctctgca ctgccctctt cacgcccatc tacctggccc tcctggtggc ctcgctgccc   900
   tttgcgtttc tcggctttct cttctggtcc ccactgcagt cggcccgccg gccctacatc   960
   tattcacggc tggaagacaa gggcctggcc ggtggggcag ccctgctcag tgaatggaag  1020
   ggcacggggc ctggcaaaag cttctgcttt gccactgcca acgtctgcct cctgcccgac 1080
   tcactcgcca gggtcaacaa cctttttaac acccaagcgc gggccaagga gatcgggcag 1140
   agaatccgca atggggccgc ccggccccag atcaaaattt acatcgactc ccccaccaat  1200
   acctccatca gcgccgctag cttcagcagc ctggtgtcac cacagggcgg cgatggggtg  1260
   gcccgggccg tccccgggag cattaagagg acagcctctg tggagtacaa gggtgacggt  1320
   gggcggcacc ccggtgacga ggctgccaac ggcccagcct ctggggaccc tgtcgacagc  1380
   agcagcccgg aggatgcctg catcgtgcgc atcggtggcg aggagggcgg ccggccacct  1440
   gaagctgacg accctgtgcc tgggggccag gccaggaacg gagctggcgg gggcccaagg  1500
   ggccagacgc ccaaccataa tcagcaggac ggggattcag ggagcctggg cagcccctcg  1560
   gcctcccggg agtccctggt gaaggggcga gctgggccag acaccagtgc cagcggggag  1620
   ccaggtgcca acagcaagct cctgtacaag gcctcggtgg tgaagaaggc ggctgcacgc 1680
   aggaggcggc accccgacga ggccttcgac catgaggtct ccgccttctt ccccgccaac 1740
   ctggacttcc tgtgcctgca ggaggtgttt gacaagcgag cagccaccaa attgaaagag 1800
   cagctgcacg gctacttcga gtacatcctg tacgacgtcg ggtctacggc ctgccagggc 1860
   tgctgcagct tcaagtgtct caacagcggc ctcctctttg ccagccgcta ccccatcatg 1920
   gacgtggcct atcactgtta ccccaacaag tgtaacgacg atgccctggc ctctaaggga 1980
   gctctgtttc tcaaggtgca ggtgggaagc acacctcagg accaaagaat cgtcgggtac 2040
   atcgcctgca cacacctgca tgccccacaa gaggacagcg ccatccggtg tgggcagctg 2100
   gacctgcttc aggactggct ggctgatttc cgaaaatcta cctcctcgtc cagcgcagcc 2160
   aaccccgagg agctggtggc atttgacgtc gtctgtggag atttcaactt tgataactgc 2220
   tcctctgacg acaagctgga gcagcaacac tccctgttca cccactacag ggacccctgc 2280
   cgcctggggc ctggtgagga gaagccgtgg gccatcggta ctctgctgga cacgaacggc 2340
   ctgtacgatg aggatgtgtg cacccccgac aacctgcaga aggtcctgga gagtgaggag 2400
   ggccgcaggg agtacctggc gtttcccacc agcaagagct cgggccagaa ggggcggaag 2460
   gagctgctga agggcaacgg ccggcgcatc gactacatgc tgcatgcaga ggaggggctg 2520
   tgcccagact ggaaggccga ggtggaagaa ttcagtttta tcacccagct gtccggcctg 2580
   acggaccacc tgccagtagc catgcgactg atggtgtctt cgggggagga ggaggcatag 2640
   ctcgacctgc agaagcttgg ccgccatggc ccaacttgtt tattgcagct tataatggtt 2700
   acaaataaag caatagcatc acaaatttca caaataaagc atttttttca ctgcattcta 2760
   gttgtggttt gtccaaactc atcaatgtat cttatcatgt ctggatcgat cgggaattaa 2820
   ttcggcgcag caccatggcc tgaaataacc tctgaaagag gaacttggtt aggtaccttc 2880
   tgaggcggaa agaaccagct gtggaatgtg tgtcagttag ggtgtggaaa gtccccaggc 2940
   tccccagcag gcagaagtat gcaaagcatg catctcaatt agtcagcaac caggtgtgga 3000
   aagtccccag gctccccagc aggcagaact atgcaaagca tgcatctcaa ttagtcagca 3060
   accatagtcc cgcccctaac tccgcccatc ccgcccctaa ctccgcccag ttccgcccat 3120
   tctccgcccc atggctgact aatttttttt atttatgcag aggccgaggc cgcctcggcc 3180
   tctgagctat tccagaagta gtgaggaggc ttttttggag gcctaggctt ttgcaaaaag 3240
   ctgttaacag cttggcactg gccgtcgttt tacaacgtcg tgactgggaa aaccctggcg 3300
   ttacccaact taatcgcctt gcagcacatc cccccttcgc cagctggcgt aatagcgaag 3360
   aggcccgcac cgatcgccct tcccaacagt tgcgtagcct gaatggcgaa tggcgcctga 3420
   tgcggtattt tctccttacg catctgtgcg gtatttcaca ccgcatacgt caaagcaacc 3480
   atagtacgcg ccctgtagcg gcgcattaag cgcggcgggt gtggtggtta cgcgcagcgt 3540
   gaccgctaca cttgccagcg ccctagcgcc cgctcctttc gctttcttcc cttcctttct 3600
   cgccacgttc gccggctttc cccgtcaagc tctaaatcgg gggctccctt tagggttccg 3660
   atttagtgct ttacggcacc tcgaccccaa aaaacttgat ttgggtgatg gttcacgtag 3720
   tgggccatcg ccctgataga cggtttttcg ccctttgacg ttggagtcca cgttctttaa 3780
   tagtggactc ttgttccaaa ctggaacaac actcaaccct atctcgggct attcttttga 3840
   tttataaggg attttgccga tttcggccta ttggttaaaa aatgagctga tttaacaaaa 3900
   atttaacgcg aattttaaca aaatattaac gtttacaatt ttatggtgca ctctcagtac   3960
   aatctgctct gatgccgcat agttaagcca actccgctat cgctacgtga ctgggtcatg   4020
   gctgcgcccc gacacccgcc aacacccgct gacgcgccct gccgggcttg tctgctcccg 4080
   gcatccgctt acagacaagc tgtgaccgtc tccgggagct gcatgtgtca gaggttttca 4140
   ccgtcatcac cgaaacgcgc gaggcagtat tcttgaagac gaaagggcct cgtgatacgc 4200
   ctatttttat aggttaatgt catgataata atggtttctt agacgtcagg tggcactttt 4260
   cggggaaatg tgcgcggaac ccctatttgt ttatttttct aaatacattc aaatatgtat 4320
   ccgctcatga gacaataacc ctgataaatg cttcaataat attgaaaaag gaagagtatg 4380
   agtattcaac atttccgtgt cgcccttatt cccttttttg cggcattttg ccttcctgtt 4440
   tttgctcacc cagaaacgct ggtgaaagta aaagatgctg aagatcagtt gggtgcacga 4500
   gtgggttaca tcgaactgga tctcaacagc ggtaagatcc ttgagagttt tcgccccgaa 4560
   gaacgttttc caatgatgag cacttttaaa gttctgctat gtggcgcggt attatcccgt 4620
   gatgacgccg ggcaagagca actcggtcgc cgcatacact attctcagaa tgacttggtt 4680
   gagtactcac cagtcacaga aaagcatctt acggatggca tgacagtaag agaattatgc 4740
   agtgctgcca taaccatgag tgataacact gcggccaact tacttctgac aacgatcgga 4800
   ggaccgaagg agctaaccgc ttttttgcac aacatggggg atcatgtaac tcgccttgat 4860
   cgttgggaac cggagctgaa tgaagccata ccaaacgacg agcgtgacac cacgatgcca 4920
   gcagcaatgg caacaacgtt gcgcaaacta ttaactggcg aactacttac tctagcttcc 4980
   cggcaacaat taatagactg gatggaggcg gataaagttg caggaccact tctgcgctcg 5040
   gcccttccgg ctggctggtt tattgctgat aaatctggag ccggtgagcg tgggtctcgc 5100
   ggtatcattg cagcactggg gccagatggt aagccctccc gtatcgtagt tatctacacg 5160
   acggggagtc aggcaactat ggatgaacga aatagacaga tcgctgagat aggtgcctca 5220
   ctgattaagc attggtaact gtcagaccaa gtttactcat atatacttta gattgattta 5280
   aaacttcatt tttaatttaa aaggatctag gtgaagatcc tttttgataa tctcatgacc 5340
   aaaatccctt aacgtgagtt ttcgttccac tgagcgtcag accccgtaga aaagatcaaa 5400
   ggatcttctt gagatccttt ttttctgcgc gtaatctgct gcttgcaaac aaaaaaacca 5460
   ccgctaccag cggtggtttg tttgccggat caagagctac caactctttt tccgaaggta 5520
   actggcttca gcagagcgca gataccaaat actgtccttc tagtgtagcc gtagttaggc 5580
   caccacttca agaactctgt agcaccgcct acatacctcg ctctgctaat cctgttacca 5640
   gtggctgctg ccagtggcga taagtcgtgt cttaccgggt tggactcaag acgatagtta 5700
   ccggataacg cgcagcggtc gggctgaacg gggggttcgt gcacacagcc cagcttggag 5760
   cgaacgacct acaccgaact gagataccta cagcctgagc attgagaaag cgccacgctt 5820
   cccgaaggga gaaaggcgga caggtatccg gtaagcggca gggtcggaac aggagagcgc 5880
   acgagggagc ttccaggggg aaacgcctgg tatctttata gtcctgtcgg gtttcgccac 5940
   ctctgacttg agcgtcgatt tttgtgatgc tcgtcagggg ggcggagcct atggaaaaac 6000
   gccagcaacg cggccttttt acggttcctg gccttttgct ggccttttgc tcacatgttc 6060
   tttcctgcgt tatcccctga ttctgtggat aaccgtatta ccgcctttga gtgagctgat 6120
   accgctcgcc gcagccgaac gaccgagcgc agcgagtcag tgagcgagga agcggaagag 6180
   cgcccaatac gcaaaccgcc tctccccgcg cgttggccga atcattaatc cagctggcac 6240
   gacaggtttc ccgactggaa agcgggcagt gagcgcaacg caattaatgt gagttacctc 6300
   actcattagg caccccaggc tttacacttt atgcttccgg ctcgtatgtt gtgtggaatt 6360
   gtgagcggat aacaatttca cacaggaaac agctatgacc atgattacga attaattcga 6420
   gctcgcccga cattgattat tgactagtta ttaatagtaa tcaattacgg ggtcattagt 6480
   tcatagccca tatatggagt tccgcgttac ataacttacg gtaaatggcc cgcctggctg 6540
   accgcccaac gacccccgcc cattgacgtc aataatgacg tatgttccca tagtaacgcc 6600
   aatagggact ttccattgac gtcaatgggt ggagtattta cggtaaactg cccacttggc 6660
   agtacatcaa gtgtatca 6678
<210> 12
   <211> 8433
   <212> DNA
   <213> Artificial
<220>
   <223> pYES-nSMase3
<400> 12
   acggattaga agccgccgag cgggtgacag ccctccgaag gaagactctc ctccgtgcgt      60
   cctcgtcttc accggtcgcg ttcctgaaac gcagatgtgc ctcgcgccgc actgctccga     120
   acaataaaga ttctacaata ctagctttta tggttatgaa gaggaaaaat tggcagtaac     180
   ctggccccac aaaccttcaa atgaacgaat caaattaaca accataggat gataatgcga     240
   ttagtttttt agccttattt ctggggtaat taatcagcga agcgatgatt tttgatctat     300
   taacagatat ataaatgcaa aaactgcata accactttaa ctaatacttt caacattttc     360
   ggtttgtatt acttcttatt caaatgtaat aaaagtatca acaaaaaatt gttaatatac 420
   ctctatactt taacgtcaag gagaaaaaac cccggatcgg actactagca gctgtaatac 480
   gactcactat agggaatatt aagcttggta ccgagctcgg atccactagt aacggccgcc 540
   agtgtgctgg aattctgatg acgactttcg gcgccgtggc ggaatggcgg cttccatctc 600
   tgaggcgagc gacgctatgg atcccacagt ggtttgctaa gaaggccatt ttcaactctc 660
   cactggaggc tgctatggcg ttccctcacc tgcagcagcc cagctttcta ctggctagcc 720
   tgaaagctga ctctataaat aagccctttg cacagcagtg ccaagacttg gttaaagtca 780
   ttgaggactt tccagcaaag gagctgcaca ccatcttccc atggctggta gaaagcattt 840
   ttggcagcct agatggtgtc ctcgttggct ggaacctccg ctgcttacag gggcgcgtga 900
   atcctgtgga gtacagcatc gtgatggaat ttctcgaccc tggtggccca atgatgaagt 960
   tggtttataa gcttcaagct gaagactata agttcgactt tcctgtctcc tacttgcctg 1020
   gtcctgtgaa ggcgtccatc caggagtgca tcctccctga cagtcctctg taccacaaca 1080
   aggtccagtt cacccctact gggggccttg gtctgaactt ggccctgaat ccgttcgagt 1140
   attacatatt cttctttgcc ttgagcctca tcactcagaa gccacttcct gtgtccctcc 1200
   acgtccgtac ttcagactgt gcctatttca tcctggtgga caggtacctg tcatggttcc 1260
   tgcccaccga aggcagtgtg cccccaccac tctcctccag cccagggggg accagcccct 1320
   caccacctcc caggacacca gccataccct ttgcttccta tggcctccac cacactagcc 1380
   tcctaaagcg acacatctct catcagacgt ctgtgaatgc agaccccgcc tcccacgaga 1440
   tctggaggtc agaaactctg ctccaggttt ttgttgaaat gtggcttcat cactattcct 1500
   tggagatgta tcaaaaaatg cagtcccctc atgccaagct ggaggttctg cactaccgac 1560
   tcagtgtctc cagcgccctc tacagccccg cccaacccag cctccaggcc ctccacgcct 1620
   accaagagtc gttcacgcct actgaggagc atgtgttggt ggtgcgcctg ctgctgaagc 1680
   acctgcacgc ctttgccaac agcctgaagc cagagcaggc ctcaccctcc gcccactccc 1740
   acgccaccag ccccctggag gagttcaaac gggctgctgt cccgaggttc gtccagcaga 1800
   aactctacct cttcttgcag cattgctttg gccactggcc cctggacgca tcgttcagag 1860
   ctgtcctgga gatgtggctg agctacctgc agccgtggcg gtacgcgcct gacaagcagg 1920
   ctccgggcag cgactcccag ccccggtgtg tgtcggagaa atgggcaccc tttgtccagg 1980
   agaacctgct gatgtacacc aagttgtttg tgggctttct gaaccgcgcg ctccgcacag 2040
   acctggtcag ccccaagcac gcgctcatgg tgttccgagt ggccaaagtc tttgcccagc 2100
   ccaacctggc tgagatgatt cagaaaggtg agcagctatt cctggagcca gagctggtca 2160
   tcccccaccg ccagcaccga ctcttcacgg cccccacatt cactgggagc ttcctgtcac 2220
   cctggccacc agcggtcact gatgcctcct tcaaggtgaa gagccacgtc tacagcctgg 2280
   agggccagga ctgcaagtac accccgatgt ttgggcccga ggcccgcacc ctggtcctgc 2340
   gcctcgctca gctcatcaca caggccaaac acacagccaa gtccatctcc gaccagtgtg 2400
   cggagagccc ggctggccac tccttcctct catggctggg ctttagctcc atggacacca 2460
   atggctccta cacagccaac gacctggacg agatggggca agacagtgtc cggaagacag 2520
   atgaatacct ggagaaggcc ctggagtacc tgcgccagat attccggctc agcgaagcgc 2580
   agctcaggca gttcacactc gccttgggca ccacccagga tgagaatgga aaaaagcaac 2640
   tccccgactg catcgtgggt gaggacggac tcatccttac gcccctgggg cggtaccaga 2700
   tcatcaatgg gctgcgaagg tttgaaattg agtaccaggg ggacccggag ctgcagccca 2760
   tccggagcta tgagatcgcc agcttggtcc gcacactctt taggctgtcg tctgccatca 2820
   accacagatt tgcaggacag atggcggctc tgtgttcccg ggatgacttc ctcggcagct 2880
   tctgtcgcta ccacctcaca gaacctgggc tggccagcag gcacctgctg agccctgtgg 2940
   ggcggaggca ggtggccggc cacacccgcg gccccaggct cagcctgcgc ttcctgggca 3000
   gttaccggac gctggtctcg ctgctgctgg ccttcttcgt ggcctctctg ttctgcgtcg 3060
   ggcccctccc atgcacgctg ctgctcaccc tgggctatgt cctctacgcc tctgccatga 3120
   cactgctgac cgagcggggg aagctgcacc agccctgacg tcgagcatgc atctagaggg 3180
   ccgcatcatg taattagtta tgtcacgctt acattcacgc cctcccccca catccgctct 3240
   aaccgaaaag gaaggagtta gacaacctga agtctaggtc cctatttatt tttttatagt 3300
   tatgttagta ttaagaacgt tatttatatt tcaaattttt cttttttttc tgtacagacg 3360
   cgtgtacgca tgtaacatta tactgaaaac cttgcttgag aaggttttgg gacgctcgaa 3420
   ggctttaatt tgcggccctg cattaatgaa tcggccaacg cgcggggaga ggcggtttgc 3480
   gtattgggcg ctcttccgct tcctcgctca ctgactcgct gcgctcggtc gttcggctgc 3540
   ggcgagcggt atcagctcac tcaaaggcgg taatacggtt atccacagaa tcaggggata 3600
   acgcaggaaa gaacatgtga gcaaaaggcc agcaaaaggc caggaaccgt aaaaaggccg 3660
   cgttgctggc gtttttccat aggctccgcc cccctgacga gcatcacaaa aatcgacgct 3720
   caagtcagag gtggcgaaac ccgacaggac tataaagata ccaggcgttt ccccctggaa 3780
   gctccctcgt gcgctctcct gttccgaccc tgccgcttac cggatacctg tccgcctttc 3840
   tcccttcggg aagcgtggcg ctttctcata gctcacgctg taggtatctc agttcggtgt 3900
   aggtcgttcg ctccaagctg ggctgtgtgc acgaaccccc cgttcagccc gaccgctgcg 3960
   ccttatccgg taactatcgt cttgagtcca acccggtaag acacgactta tcgccactgg 4020
   cagcagccac tggtaacagg attagcagag cgaggtatgt aggcggtgct acagagttct 4080
   tgaagtggtg gcctaactac ggctacacta gaaggacagt atttggtatc tgcgctctgc 4140
   tgaagccagt taccttcgga aaaagagttg gtagctcttg atccggcaaa caaaccaccg 4200
   ctggtagcgg tggttttttt gtttgcaagc agcagattac gcgcagaaaa aaaggatctc 4260
   aagaagatcc tttgatcttt tctacggggt ctgacgctca gtggaacgaa aactcacgtt 4320
   aagggatttt ggtcatgaga ttatcaaaaa ggatcttcac ctagatcctt ttaaattaaa 4380
   aatgaagttt taaatcaatc taaagtatat atgagtaaac ttggtctgac agttaccaat 4440
   gcttaatcag tgaggcacct atctcagcga tctgtctatt tcgttcatcc atagttgcct 4500
   gactccccgt cgtgtagata actacgatac gggagcgctt accatctggc cccagtgctg 4560
   caatgatacc gcgagaccca cgctcaccgg ctccagattt atcagcaata aaccagccag 4620
   ccggaagggc cgagcgcaga agtggtcctg caactttatc cgcctccatt cagtctatta 4680
   attgttgccg ggaagctaga gtaagtagtt cgccagttaa tagtttgcgc aacgttgttg 4740
   gcattgctac aggcatcgtg gtgtcactct cgtcgtttgg tatggcttca ttcagctccg 4800
   gttcccaacg atcaaggcga gttacatgat cccccatgtt gtgcaaaaaa gcggttagct 4860
   ccttcggtcc tccgatcgtt gtcagaagta agttggccgc agtgttatca ctcatggtta 4920
   tggcagcact gcataattct cttactgtca tgccatccgt aagatgcttt tctgtgactg 4980
   gtgagtactc aaccaagtca ttctgagaat agtgtatgcg gcgaccgagt tgctcttgcc 5040
   cggcgtcaat acgggataat agtgtatcac atagcagaac tttaaaagtg ctcatcattg 5100
   gaaaacgttc ttcggggcga aaactctcaa ggatcttacc gctgttgaga tccagttcga 5160
   tgtaacccac tcgtgcaccc aactgatctt cagcatcttt tactttcacc agcgtttctg 5220
   ggtgagcaaa aacaggaagg caaaatgccg caaaaaaggg aataagggcg acacggaaat 5280
   gttgaatact catactcttc ctttttcaat gggtaataac tgatataatt aaattgaagc 5340
   tctaatttgt gagtttagta tacatgcatt tacttataat acagtttttt agttttgctg 5400
   gccgcatctt ctcaaatatg cttcccagcc tgcttttctg taacgttcac cctctacctt 5460
   agcatccctt ccctttgcaa atagtcctct tccaacaata ataatgtcag atcctgtaga 5520
   gaccacatca tccacggttc tatactgttg acccaatgcg tctcccttgt catctaaacc 5580
   cacaccgggt gtcataatca accaatcgta accttcatct cttccaccca tgtctctttg 5640
   agcaataaag ccgataacaa aatctttgtc gctcttcgca atgtcaacag tacccttagt   5700
   atattctcca gtagataggg agcccttgca tgacaattct gctaacatca aaaggcctct 5760
   aggttccttt gttacttctt ctgccgcctg cttcaaaccg ctaacaatac ctgggcccac 5820
   cacaccgtgt gcattcgtaa tgtctgccca ttctgctatt ctgtatacac ccgcagagta 5880
   ctgcaatttg actgtattac caatgtcagc aaattttctg tcttcgaaga gtaaaaaatt 5940
   gtacttggcg gataatgcct ttagcggctt aactgtgccc tccatggaaa aatcagtcaa 6000
   gatatccaca tgtgttttta gtaaacaaat tttgggacct aatgcttcaa ctaactccag 6060
   taattccttg gtggtacgaa catccaatga agcacacaag tttgtttgct tttcgtgcat 6120
   gatattaaat agcttggcag caacaggact aggatgagta gcagcacgtt ccttatatgt 6180
   agctttcgac atgatttatc ttcgtttcct gcaggttttt gttctgtgca gttgggttaa 6240
   gaatactggg caatttcatg tttcttcaac actacatatg cgtatatata ccaatctaag 6300
   tctgtgctcc ttccttcgtt cttccttctg ttcggagatt accgaatcaa aaaaatttca 6360
   aagaaaccga aatcaaaaaa aagaataaaa aaaaaatgat gaattgaatt gaaaagctag 6420
   cttatcgatg ataagctgtc aaagatgaga attaattcca cggactatag actatactag 6480
   atactccgtc tactgtacga tacacttccg ctcaggtcct tgtcctttaa cgaggcctta 6540
   ccactctttt gttactctat tgatccagct cagcaaaggc agtgtgatct aagattctat 6600
   cttcgcgatg tagtaaaact agctagaccg agaaagagac tagaaatgca aaaggcactt 6660
   ctacaatggc tgccatcatt attatccgat gtgacgctgc agcttctcaa tgatattcga 6720
   atacgctttg aggagataca gcctaatatc cgacaaactg ttttacagat ttacgatcgt 6780
   acttgttacc catcattgaa ttttgaacat ccgaacctgg gagttttccc tgaaacagat 6840
   agtatatttg aacctgtata ataatatata gtctagcgct ttacggaaga caatgtatgt 6900
   atttcggttc ctggagaaac tattgcatct attgcatagg taatcttgca cgtcgcatcc 6960
   ccggttcatt ttctgcgttt ccatcttgca cttcaatagc atatctttgt taacgaagca 7020
   tctgtgcttc attttgtaga acaaaaatgc aacgcgagag cgctaatttt tcaaacaaag 7080
   aatctgagct gcatttttac agaacagaaa tgcaacgcga aagcgctatt ttaccaacga 7140
   agaatctgtg cttcattttt gtaaaacaaa aatgcaacgc gacgagagcg ctaatttttc 7200
   aaacaaagaa tctgagctgc atttttacag aacagaaatg caacgcgaga gcgctatttt 7260
   accaacaaag aatctatact tcttttttgt tctacaaaaa tgcatcccga gagcgctatt 7320
   tttctaacaa agcatcttag attacttttt ttctcctttg tgcgctctat aatgcagtct 7380
   cttgataact ttttgcactg taggtccgtt aaggttagaa gaaggctact ttggtgtcta 7440
   ttttctcttc cataaaaaaa gcctgactcc acttcccgcg tttactgatt actagcgaag 7500
   ctgcgggtgc attttttcaa gataaaggca tccccgatta tattctatac cgatgtggat 7560
   tgcgcatact ttgtgaacag aaagtgatag cgttgatgat tcttcattgg tcagaaaatt 7620
   atgaacggtt tcttctattt tgtctctata tactacgtat aggaaatgtt tacattttcg 7680
   tattgttttc gattcactct atgaatagtt cttactacaa tttttttgtc taaagagtaa 7740
   tactagagat aaacataaaa aatgtagagg tcgagtttag atgcaagttc aaggagcgaa 7800
   aggtggatgg gtaggttata tagggatata gcacagagat atatagcaaa gagatacttt 7860
   tgagcaatgt ttgtggaagc ggtattcgca atgggaagct ccaccccggt tgataatcag 7920
   aaaagcccca aaaacaggaa gattgtataa gcaaatattt aaattgtaaa cgttaatatt 7980
   ttgttaaaat tcgcgttaaa tttttgttaa atcagctcat tttttaacga atagcccgaa 8040
   atcggcaaaa tcccttataa atcaaaagaa tagaccgaga tagggttgag tgttgttcca 8100
   gtttccaaca agagtccact attaaagaac gtggactcca acgtcaaagg gcgaaaaagg 8160
   gtctatcagg gcgatggccc actacgtgaa ccatcaccct aatcaagttt tttggggtcg 8220
   aggtgccgta aagcagtaaa tcggaagggt aaacggatgc ccccatttag agcttgacgg 8280
   ggaaagccgg cgaacgtggc gagaaaggaa gggaagaaag cgaaaggagc gggggctagg 8340
   gcggtgggaa gtgtaggggt cacgctgggc gtaaccacca cacccgccgc gcttaatggg 8400
   gcgctacagg gcgcgtgggg atgatccact agt 8433
<210> 13
   <211> 7766
   <212> DNA
   <213> Artificial
<220>
   <223> pYES-nSMase2
<400> 13
   acggattaga agccgccgag cgggtgacag ccctccgaag gaagactctc ctccgtgcgt 60
   cctcgtcttc accggtcgcg ttcctgaaac gcagatgtgc ctcgcgccgc actgctccga 120
   acaataaaga ttctacaata ctagctttta tggttatgaa gaggaaaaat tggcagtaac 180
   ctggccccac aaaccttcaa atgaacgaat caaattaaca accataggat gataatgcga 240
   ttagtttttt agccttattt ctggggtaat taatcagcga agcgatgatt tttgatctat 300
   taacagatat ataaatgcaa aaactgcata accactttaa ctaatacttt caacattttc 360
   ggtttgtatt acttcttatt caaatgtaat aaaagtatca acaaaaaatt gttaatatac 420
   ctctatactt taacgtcaag gagaaaaaac cccggatcgg actactagca gctgtaatac 480
   gactcactat agggaatatt aagcttggta ccgagctcgg atccatggtt ttgtacacga 540
   ccccctttcc taacagctgt ctgtccgccc tgcactgtgt gtcctgggcc cttatctttc 600
   catgctactg gctggtggac cggctcgctg cctccttcat acccaccacc tacgagaagc 660
   gccagcgggc agacgacccg tgctgcctgc agctgctctg cactgccctc ttcacgccca 720
   tctacctggc cctcctggtg gcctcgctgc cctttgcgtt tctcggcttt ctcttctggt 780
   ccccactgca gtcggcccgc cggccctaca tctattcacg gctggaagac aagggcctgg 840
   ccggtggggc agccctgctc agtgaatgga agggcacggg gcctggcaaa agcttctgct 900
   ttgccactgc caacgtctgc ctcctgcccg actcactcgc cagggtcaac aaccttttta 960
   acacccaagc gcgggccaag gagatcgggc agagaatccg caatggggcc gcccggcccc 1020
   agatcaaaat ttacatcgac tcccccacca atacctccat cagcgccgct agcttcagca 1080
   gcctggtgtc accacagggc ggcgatgggg tggcccgggc cgtccccggg agcattaaga 1140
   ggacagcctc tgtggagtac aagggtgacg gtgggcggca ccccggtgac gaggctgcca 1200
   acggcccagc ctctggggac cctgtcgaca gcagcagccc ggaggatgcc tgcatcgtgc 1260
   gcatcggtgg cgaggagggc ggccggccac ctgaagctga cgaccctgtg cctgggggcc 1320
   aggccaggaa cggagctggc gggggcccaa ggggccagac gcccaaccat aatcagcagg 1380
   acggggattc agggagcctg ggcagcccct cggcctcccg ggagtccctg gtgaaggggc 1440
   gagctgggcc agacaccagt gccagcgggg agccaggtgc caacagcaag ctcctgtaca 1500
   aggcctcggt ggtgaagaag gcggctgcac gcaggaggcg gcaccccgac gaggccttcg 1560
   accatgaggt ctccgccttc ttccccgcca acctggactt cctgtgcctg caggaggtgt 1620
   ttgacaagcg agcagccacc aaattgaaag agcagctgca cggctacttc gagtacatcc 1680
   tgtacgacgt cggggtctac ggctgccagg gctgctgcag cttcaagtgt ctcaacagcg 1740
   gcctcctctt tgccagccgc taccccatca tggacgtggc ctatcactgt taccccaaca 1800
   agtgtaacga cgatgccctg gcctctaagg gagctctgtt tctcaaggtg caggtgggaa 1860
   gcacacctca ggaccaaaga atcgtcgggt acatcgcctg cacacacctg catgccccac 1920
   aagaggacag cgccatccgg tgtgggcagc tggacctgct tcaggactgg ctggctgatt 1980
   tccgaaaatc tacctcctcg tccagcgcag ccaaccccga ggagctggtg gcatttgacg 2040
   tcgtctgtgg agatttcaac tttgataact gctcctctga cgacaagctg gagcagcaac 2100
   actccctgtt cacccactac agggacccct gccgcctggg gcctggtgag gagaagccgt 2160
   gggccatcgg tactctgctg gacacgaacg gcctgtacga tgaggatgtg tgcacccccg 2220
   acaacctgca gaaggtcctg gagagtgagg agggccgcag ggagtacctg gcgtttccca 2280
   ccagcaagag ctcgggccag aaggggcgga aggagctgct gaagggcaac ggccggcgca 2340
   tcgactacat gctgcatgca gaggaggggc tgtgcccaga ctggaaggcc gaggtggaag 2400
   aattcagttt tatcacccag ctgtccggcc tgacggacca cctgccagta gccatgcgac 2460
   tgatggtgtc ttcgggggag gaggaggcat agctcgagca tgcatctaga gggccgcatc 2520
   atgtaattag ttatgtcacg cttacattca cgccctcccc ccacatccgc tctaaccgaa 2580
   aaggaaggag ttagacaacc tgaagtctag gtccctattt atttttttat agttatgtta 2640
   gtattaagaa cgttatttat atttcaaatt tttctttttt ttctgtacag acgcgtgtac 2700
   gcatgtaaca ttatactgaa aaccttgctt gagaaggttt tgggacgctc gaaggcttta 2760
   atttgcggcc ctgcattaat gaatcggcca acgcgcgggg agaggcggtt tgcgtattgg 2820
   gcgctcttcc gcttcctcgc tcactgactc gctgcgctcg gtcgttcggc tgcggcgagc 2880
   ggtatcagct cactcaaagg cggtaatacg gttatccaca gaatcagggg ataacgcagg 2940
   aaagaacatg tgagcaaaag gccagcaaaa ggccaggaac cgtaaaaagg ccgcgttgct 3000
   ggcgtttttc cataggctcc gcccccctga cgagcatcac aaaaatcgac gctcaagtca 3060
   gaggtggcga aacccgacag gactataaag ataccaggcg tttccccctg gaagctccct 3120
   cgtgcgctct cctgttccga ccctgccgct taccggatac ctgtccgcct ttctcccttc 3180
   gggaagcgtg gcgctttctc atagctcacg ctgtaggtat ctcagttcgg tgtaggtcgt 3240
   tcgctccaag ctgggctgtg tgcacgaacc ccccgttcag cccgaccgct gcgccttatc 3300
   cggtaactat cgtcttgagt ccaacccggt aagacacgac ttatcgccac tggcagcagc 3360
   cactggtaac aggattagca gagcgaggta tgtaggcggt gctacagagt tcttgaagtg 3420
   gtggcctaac tacggctaca ctagaaggac agtatttggt atctgcgctc tgctgaagcc 3480
   agttaccttc ggaaaaagag ttggtagctc ttgatccggc aaacaaacca ccgctggtag 3540
   cggtggtttt tttgtttgca agcagcagat tacgcgcaga aaaaaaggat ctcaagaaga 3600
   tcctttgatc ttttctacgg ggtctgacgc tcagtggaac gaaaactcac gttaagggat 3660
   tttggtcatg agattatcaa aaaggatctt cacctagatc cttttaaatt aaaaatgaag 3720
   ttttaaatca atctaaagta tatatgagta aacttggtct gacagttacc aatgcttaat 3780
   cagtgaggca cctatctcag cgatctgtct atttcgttca tccatagttg cctgactccc 3840
   cgtcgtgtag ataactacga tacgggagcg cttaccatct ggccccagtg ctgcaatgat 3900
   accgcgagac ccacgctcac cggctccaga tttatcagca ataaaccagc cagccggaag 3960
   ggccgagcgc agaagtggtc ctgcaacttt atccgcctcc attcagtcta ttaattgttg 4020
   ccgggaagct agagtaagta gttcgccagt taatagtttg cgcaacgttg ttggcattgc 4080
   tacaggcatc gtggtgtcac tctcgtcgtt tggtatggct tcattcagct ccggttccca 4140
   acgatcaagg cgagttacat gatcccccat gttgtgcaaa aaagcggtta gctccttcgg 4200
   tcctccgatc gttgtcagaa gtaagttggc cgcagtgtta tcactcatgg ttatggcagc 4260
   actgcataat tctcttactg tcatgccatc cgtaagatgc ttttctgtga ctggtgagta 4320
   ctcaaccaag tcattctgag aatagtgtat gcggcgaccg agttgctctt gcccggcgtc 4380
   aatacgggat aatagtgtat cacatagcag aactttaaaa gtgctcatca ttggaaaacg 4440
   ttcttcgggg cgaaaactct caaggatctt accgctgttg agatccagtt cgatgtaacc 4500
   cactcgtgca cccaactgat cttcagcatc ttttactttc accagcgttt ctgggtgagc 4560
   aaaaacagga aggcaaaatg ccgcaaaaaa gggaataagg gcgacacgga aatgttgaat 4620
   actcatactc ttcctttttc aatgggtaat aactgatata attaaattga agctctaatt 4680
   tgtgagttta gtatacatgc atttacttat aatacagttt tttagttttg ctggccgcat 4740
   cttctcaaat atgcttccca gcctgctttt ctgtaacgtt caccctctac cttagcatcc 4800
   cttccctttg caaatagtcc tcttccaaca ataataatgt cagatcctgt agagaccaca 4860
   tcatccacgg ttctatactg ttgacccaat gcgtctccct tgtcatctaa acccacaccg 4920
   ggtgtcataa tcaaccaatc gtaaccttca tctcttccac ccatgtctct ttgagcaata 4980
   aagccgataa caaaatcttt gtcgctcttc gcaatgtcaa cagtaccctt agtatattct 5040
   ccagtagata gggagccctt gcatgacaat tctgctaaca tcaaaaggcc tctaggttcc 5100
   tttgttactt cttctgccgc ctgcttcaaa ccgctaacaa tacctgggcc caccacaccg 5160
   tgtgcattcg taatgtctgc ccattctgct attctgtata cacccgcaga gtactgcaat 5220
   ttgactgtat taccaatgtc agcaaatttt ctgtcttcga agagtaaaaa attgtacttg 5280
   gcggataatg cctttagcgg cttaactgtg ccctccatgg aaaaatcagt caagatatcc 5340
   acatgtgttt ttagtaaaca aattttggga cctaatgctt caactaactc cagtaattcc 5400
   ttggtggtac gaacatccaa tgaagcacac aagtttgttt gcttttcgtg catgatatta 5460
   aatagcttgg cagcaacagg actaggatga gtagcagcac gttccttata tgtagctttc 5520
   gacatgattt atcttcgttt cctgcaggtt tttgttctgt gcagttgggt taagaatact 5580
   gggcaatttc atgtttcttc aacactacat atgcgtatat ataccaatct aagtctgtgc 5640
   tccttccttc gttcttcctt ctgttcggag attaccgaat caaaaaaatt tcaaagaaac 5700
   cgaaatcaaa aaaaagaata aaaaaaaaat gatgaattga attgaaaagc tagcttatcg 5760
   atgataagct gtcaaagatg agaattaatt ccacggacta tagactatac tagatactcc 5820
   gtctactgta cgatacactt ccgctcaggt ccttgtcctt taacgaggcc ttaccactct 5880
   tttgttactc tattgatcca gctcagcaaa ggcagtgtga tctaagattc tatcttcgcg 5940
   atgtagtaaa actagctaga ccgagaaaga gactagaaat gcaaaaggca cttctacaat 6000
   ggctgccatc attattatcc gatgtgacgc tgcagcttct caatgatatt cgaatacgct 6060
   ttgaggagat acagcctaat atccgacaaa ctgttttaca gatttacgat cgtacttgtt 6120
   acccatcatt gaattttgaa catccgaacc tgggagtttt ccctgaaaca gatagtatat 6180
   ttgaacctgt ataataatat atagtctagc gctttacgga agacaatgta tgtatttcgg 6240
   ttcctggaga aactattgca tctattgcat aggtaatctt gcacgtcgca tccccggttc 6300
   attttctgcg tttccatctt gcacttcaat agcatatctt tgttaacgaa gcatctgtgc 6360
   ttcattttgt agaacaaaaa tgcaacgcga gagcgctaat ttttcaaaca aagaatctga 6420
   gctgcatttt tacagaacag aaatgcaacg cgaaagcgct attttaccaa cgaagaatct 6480
   gtgcttcatt tttgtaaaac aaaaatgcaa cgcgacgaga gcgctaattt ttcaaacaaa 6540
   gaatctgagc tgcattttta cagaacagaa atgcaacgcg agagcgctat tttaccaaca 6600
   aagaatctat acttcttttt tgttctacaa aaatgcatcc cgagagcgct atttttctaa 6660
   caaagcatct tagattactt tttttctcct ttgtgcgctc tataatgcag tctcttgata 6720
   actttttgca ctgtaggtcc gttaaggtta gaagaaggct actttggtgt ctattttctc 6780
   ttccataaaa aaagcctgac tccacttccc gcgtttactg attactagcg aagctgcggg 6840
   tgcatttttt caagataaag gcatccccga ttatattcta taccgatgtg gattgcgcat 6900
   actttgtgaa cagaaagtga tagcgttgat gattcttcat tggtcagaaa attatgaacg 6960
   gtttcttcta ttttgtctct atatactacg tataggaaat gtttacattt tcgtattgtt 7020
   ttcgattcac tctatgaata gttcttacta caattttttt gtctaaagag taatactaga 7080
   gataaacata aaaaatgtag aggtcgagtt tagatgcaag ttcaaggagc gaaaggtgga 7140
   tgggtaggtt atatagggat atagcacaga gatatatagc aaagagatac ttttgagcaa 7200
   tgtttgtgga agcggtattc gcaatgggaa gctccacccc ggttgataat cagaaaagcc 7260
   ccaaaaacag gaagattgta taagcaaata tttaaattgt aaacgttaat attttgttaa 7320
   aattcgcgtt aaatttttgt taaatcagct cattttttaa cgaatagccc gaaatcggca 7380
   aaatccctta taaatcaaaa gaatagaccg agatagggtt gagtgttgtt ccagtttcca 7440
   acaagagtcc actattaaag aacgtggact ccaacgtcaa agggcgaaaa agggtctatc 7500
   agggcgatgg cccactacgt gaaccatcac cctaatcaag ttttttgggg tcgaggtgcc 7560
   gtaaagcagt aaatcggaag ggtaaacgga tgcccccatt tagagcttga cggggaaagc 7620
   cggcgaacgt ggcgagaaag gaagggaaga aagcgaaagg agcgggggct agggcggtgg 7680
   gaagtgtagg ggtcacgctg ggcgtaacca ccacacccgc cgcgcttaat ggggcgctac 7740
   agggcgcgtg gggatgatcc actagt 7766
<210> 14
   <211> 5487
   <212> DNA
   <213> Artificial
<220>
   <223> AdCGI; bicistronic adenoviral vector encoding EGFP
<400> 14
   catcatcaat aatatacctt attttggatt gaagccaata tgataatgag ggggtggagt 60
   ttgtgacgtg gcgcggggcg tgggaacggg gcgggtgacg tagtagtgtg gcggaagtgt 120
   gatgttgcaa gtgtggcgga acacatgtaa gcgacggatg tggcaaaagt gacgtttttg 180
   gtgtgcgccg gtgtacacag gaagtgacaa ttttcgcgcg gttttaggcg gatgttgtag 240
   taaatttggg cgtaaccgag taagatttgg ccattttcgc gggaaaactg aataagagga 300
   agtgaaatct gaataatttt gtgttactca tagcgcgtaa tatttgtcta gggccgcggg 360
   gactttgacc gtttacgtgg agactcgccc aggtgttttt ctcaggtgtt ttccgcgttc 420
   cgggtcaaag ttggcgtttt attattatag tcagtaatag taatcaatta cggggtcatt 480
   agttcatagc ccatatatgg agttccgcgt tacataactt acggtaaatg gcccgcctgg 540
   ctgaccgccc aacgaccccc gcccattgac gtcaataatg acgtatgttc ccatagtaac 600
   gccaataggg actttccatt gacgtcaatg ggtggagtat ttacggtaaa ctgcccactt 660
   ggcagtacat caagtgtatc atatgccaag tacgccccct attgacgtca atgacggtaa 720
   atggcccgcc tggcattatg cccagtacat gaccttatgg gactttccta cttggcagta 780
   catctacgta ttagtcatcg ctattaccat ggtgatgcgg ttttggcagt acatcaatgg 840
   gcgtggatag cggtttgact cacggggatt tccaagtctc caccccattg acgtcaatgg 900
   gagtttgttt tggcaccaaa atcaacggga ctttccaaaa tgtcgtaaca actccgcccc 960
   attgacgcaa atgggcggta ggcgtgtacg gtgggaggtc tatataagca gagctggttt 1020
   agtgaaccgt cagatccgct agcgctaccg gtcgccacca tggtgagcaa gggcgaggag 1080
   ctgttcaccg gggtggtgcc catcctggtc gagctggacg gcgacgtaaa cggccacaag 1140
   ttcagcgtgt ccggcgaggg cgagggcgat gccacctacg gcaagctgac cctgaagttc 1200
   atctgcacca ccggcaagct gcccgtgccc tggcccaccc tcgtgaccac cctgacctac 1260
   ggcgtgcagt gcttcagccg ctaccccgac cacatgaagc agcacgactt cttcaagtcc 1320
   gccatgcccg aaggctacgt ccaggagcgc accatcttct tcaaggacga cggcaactac 1380
   aagacccgcg ccgaggtgaa gttcgagggc gacaccctgg tgaaccgcat cgagctgaag 1440
   ggcatcgact tcaaggagga cggcaacatc ctggggcaca agctggagta caactacaac 1500
   agccacaacg tctatatcat ggccgacaag cagaagaacg gcatcaaggt gaacttcaag 1560
   atccgccaca acatcgagga cggcagcgtg cagctcgccg accactacca gcagaacacc 1620
   cccatcggcg acggccccgt gctgctgccc gacaaccact acctgagcac ccagtccgcc 1680
   ctgagcaaag accccaacga gaagcgcgat cacatggtcc tgctggagtt cgtgaccgcc 1740
   gccgggatca ctctcggcat ggacgagctg tacaagtgtg gcggctagta ctccggtatt 1800
   gcggtaccct tgtacgcctg ttttatactc ccttcccgta acttagacgc acaaaaccaa 1860
   gttcaataga agggggtaca aaccagtacc accacgaaca agcacttctg tttccccggt 1920
   gatgtcgtat agactgcttg cgtggttgaa agcgacggat ccgttatccg cttatgtact 1980
   tcgagaagcc cagtaccacc tcggaatctt cgatgcgttg cgctcagcac tcaaccccag 2040
   agtgtagctt aggctgatga gtctggacat ccctcaccgg tgacggtggt ccaggctgcg 2100
   ttggcggcct acctatggct aacgccatgg gacgctagtt gtgaacaagg tgtgaagagc 2160
   ctattgagct acataagaat cctccggccc ctgaatgcgg ctaatcccaa cctcggagca 2220
   ggtggtcaca aaccagtgat tggcctgtcg taacgcgcaa gtccgtggcg gaaccgacta 2280
   ctttgggtgt ccgtgtttcc ttttatttta ttgtggctgc ttatggtgac aatcacagat 2340
   tgttatcata aagcgaattg gataggatca agcttatcga taccgtcgac ctcgagctca 2400
   agcttcgaat tctgcagtcg acggtaccgc gggcccggga tccaccggat ctagataact 2460
   gatcataatc agccatacca catttgtaga ggttttactt gctttaaaaa acctcccaca 2520
   cctccccctg aacctgaaac ataaaatgaa tgcaattgtt gttgttaact tgtttattgc 2580
   agcttataat ggttacaaat aaagcaatag catcacaaat ttcacaaata aagcattttt 2640
   ttcactgcat tctagttgtg gtttgtccaa actcatcaat gtatcttatc atgtctggat 2700
   catcgatcca taacttcgta taatgtatgc tatacgaagt tatccagatc tggttctata 2760
   gtgtcaccta aatcgtatgt gtatgataca taaggttatg tattaattgt agccgcgttc 2820
   taacgacaat atgtccatag ggcccctacg tcacccgccc cgttcccacg ccccgcgcca 2880
   cgtcacaaac tccaccccct cattatcata ttggcttcaa tccaaaataa ggtatattat 2940
   tgatgatggc cgcagcggcc cctggcgtaa tagcgaagag gcccgcaccg atcgcccttc 3000
   ccaacagttg cgcagcctga atggcgaatg ggacgcgccc tgtagcggcg cattaagcgc 3060
   ggcgggtgtg gtggttacgc gcagcgtgac cgctacactt gccagcgccc tagcgcccgc 3120
   tcctttcgct ttcttccctt cctttctcgc cacgttcgcc ggctttcccc gtcaagctct 3180
   aaatcggggg ctccctttag ggttccgatt tagtgcttta cggcacctcg accccaaaaa 3240
   acttgattag ggtgatggtt cacgtagtgg gccatcgccc tgatagacgg tttttcgccc 3300
   tttgacgttg gagtccacgt tctttaatag tggactcttg ttccaaactg gaacaacact 3360
   caaccctatc tcggtctatt cttttgattt ataagggatt ttgccgattt cggcctattg 3420
   gttaaaaaat gagctgattt aacaaaaatt taacgcgaat tttaacaaaa tattaacgct 3480
   tacaatttag gtggcacttt tcggggaaat gtgcgcggaa cccctatttg tttatttttc 3540
   taaatacatt caaatatgta tccgctcatg agacaataac cctgataaat gcttcaataa 3600
   tattgaaaaa ggaagagtat gagtattcaa catttccgtg tcgcccttat tccctttttt 3660
   gcggcatttt gccttcctgt ttttgctcac ccagaaacgc tggtgaaagt aaaagatgct 3720
   gaagatcagt tgggtgcacg agtgggttac atcgaactgg atctcaacag cggtaagatc 3780
   cttgagagtt ttcgccccga agaacgtttt ccaatgatga gcacttttaa agttctgcta 3840
   tgtggcgcgg tattatcccg tattgacgcc gggcaagagc aactcggtcg ccgcatacac 3900
   tattctcaga atgacttggt tgagtactca ccagtcacag aaaagcatct tacggatggc 3960
   atgacagtaa gagaattatg cagtgctgcc ataaccatga gtgataacac tgcggccaac 4020
   ttacttctga caacgatcgg aggaccgaag gagctaaccg cttttttgca caacatgggg 4080
   gatcatgtaa ctcgccttga tcgttgggaa ccggagctga atgaagccat accaaacgac 4140
   gagcgtgaca ccacgatgcc tgtagcaatg gcaacaacgt tgcgcaaact attaactggc 4200
   gaactactta ctctagcttc ccggcaacaa ttaatagact ggatggaggc ggataaagtt 4260
   gcaggaccac ttctgcgctc ggcccttccg gctggctggt ttattgctga taaatctgga 4320
   gccggtgagc gtgggtctcg cggtatcatt gcagcactgg ggccagatgg taagccctcc 4380
   cgtatcgtag ttatctacac gacggggagt caggcaacta tggatgaacg aaatagacag 4440
   atcgctgaga taggtgcctc actgattaag cattggtaac tgtcagacca agtttactca 4500
   tatatacttt agattgattt aaaacttcat ttttaattta aaaggatcta ggtgaagatc 4560
   ctttttgata atctcatgac caaaatccct taacgtgagt tttcgttcca ctgagcgtca 4620
   gaccccgtag aaaagatcaa aggatcttct tgagatcctt tttttctgcg cgtaatctgc 4680
   tgcttgcaaa caaaaaaacc accgctacca gcggtggttt gtttgccgga tcaagagcta 4740
   ccaactcttt ttccgaaggt aactggcttc agcagagcgc agataccaaa tactgtcctt 4800
   ctagtgtagc cgtagttagg ccaccacttc aagaactctg tagcaccgcc tacatacctc 4860
   gctctgctaa tcctgttacc agtggctgct gccagtggcg ataagtcgtg tcttaccggg 4920
   ttggactcaa gacgatagtt accggataag gcgcagcggt cgggctgaac ggggggttcg 4980
   tgcacacagc ccagcttgga gcgaacgacc tacaccgaac tgagatacct acagcgtgag 5040
   ctatgagaaa gcgccacgct tcccgaaggg agaaaggcgg acaggtatcc ggtaagcggc 5100
   agggtcggaa caggagagcg cacgagggag cttccagggg gaaacgcctg gtatctttat 5160
   agtcctgtcg ggtttcgcca cctctgactt gagcgtcgat ttttgtgatg ctcgtcaggg 5220
   gggcggagcc tatggaaaaa cgccagcaac gcggcctttt tacggttcct ggccttttgc 5280
   tggccttttg ctcacatgtt ctttcctgcg ttatcccctg attctgtgga taaccgtatt 5340
   accgcctttg agtgagctga taccgctcgc cgcagccgaa cgaccgagcg cagcgagtca 5400
   gtgagcgagg aagcggaaga gcgcccaata cgcaaaccgc ctctccccgc gcgttggccg 5460
   attcattaat gcaggggccg ctgcggc 5487
<210> 15
   <211> 7318
   <212> DNA
   <213> Artificial
<220>
   <223> pGFP-nSMase3
<400> 15
   tatgccaagt acgcccccta ttgacgtcaa tgacggtaaa tggcccgcct ggcattatgc     60
   ccagtacatg accttatggg actttcctac ttggcagtac atctacgtat tagtcatcgc    120
   tattaccatg gtgatgcggt tttggcagta catcaatggg cgtggatagc ggtttgactc    180
   acggggattt ccaagtctcc accccattga cgtcaatggg agtttgtttt ggcaccaaaa    240
   tcaacgggac tttccaaaat gtcgtaacaa ctccgcccca ttgacgcaaa tgggcggtag    300
   gcgtgtacgg tgggaggtct atataagcag agctggttta gtgaaccgtc agatccgcta    360
   gcgctaccgg tcgccaccat ggtgagcaag ggcgaggagc tgttcaccgg ggtggtgccc    420
   atcctggtcg agctggacgg cgacgtaaac ggccacaagt tcagcgtgtc cggcgagggc    480
   gagggcgatg ccacctacgg caagctgacc ctgaagttca tctgcaccac cggcaagctg    540
   cccgtgccct ggcccaccct cgtgaccacc ctgacctacg gcgtgcagtg cttcagccgc    600
   taccccgacc acatgaagca gcacgacttc ttcaagtccg ccatgcccga aggctacgtc    660
   caggagcgca ccatcttctt caaggacgac ggcaactaca agacccgcgc cgaggtgaag    720
   ttcgagggcg acaccctggt gaaccgcatc gagctgaagg gcatcgactt caaggaggac 780
   ggcaacatcc tggggcacaa gctggagtac aactacaaca gccacaacgt ctatatcatg 840
   gccgacaagc agaagaacgg catcaaggtg aacttcaaga tccgccacaa catcgaggac 900
   ggcagcgtgc agctcgccga ccactaccag cagaacaccc ccatcggcga cggccccgtg 960
   ctgctgcccg acaaccacta cctgagcacc cagtccgccc tgagcaaaga ccccaacgag 1020
   aagcgcgatc acatggtcct gctggagttc gtgaccgccg ccgggatcac tctcggcatg 1080
   gacgagctgt acaagtactc agatctcgag atgacgactt tcggcgccgt ggcggaatgg 1140
   cggcttccat ctctgaggcg agcgacgcta tggatcccac agtggtttgc taagaaggcc 1200
   attttcaact ctccactgga ggctgctatg gcgttccctc acctgcagca gcccagcttt 1260
   ctactggcta gcctgaaagc tgactctata aataagccct ttgcacagca gtgccaagac 1320
   ttggttaaag tcattgagga ctttccagca aaggagctgc acaccatctt cccatggctg 1380
   gtagaaagca tttttggcag cctagatggt gtcctcgttg gctggaacct ccgctgctta 1440
   caggggcgcg tgaatcctgt ggagtacagc atcgtgatgg aatttctcga ccctggtggc 1500
   ccaatgatga agttggttta taagcttcaa gctgaagact ataagttcga ctttcctgtc 1560
   tcctacttgc ctggtcctgt gaaggcgtcc atccaggagt gcatcctccc tgacagtcct 1620
   ctgtaccaca acaaggtcca gttcacccct actgggggcc ttggtctgaa cttggccctg 1680
   aatccgttcg agtattacat attcttcttt gccttgagcc tcatcactca gaagccactt 1740
   cctgtgtccc tccacgtccg tacttcagac tgtgcctatt tcatcctggt ggacaggtac 1800
   ctgtcatggt tcctgcccac cgaaggcagt gtgcccccac cactctcctc cagcccaggg 1860
   gggaccagcc cctcaccacc tcccaggaca ccagccatac cctttgcttc ctatggcctc 1920
   caccacacta gcctcctaaa gcgacacatc tctcatcaga cgtctgtgaa tgcagacccc 1980
   gcctcccacg agatctggag gtcagaaact ctgctccagg tttttgttga aatgtggctt 2040
   catcactatt ccttggagat gtatcaaaaa atgcagtccc ctcatgccaa gctggaggtt 2100
   ctgcactacc gactcagtgt ctccagcgcc ctctacagcc ccgcccaacc cagcctccag 2160
   gccctccacg cctaccaaga gtcgttcacg cctactgagg agcatgtgtt ggtggtgcgc 2220
   ctgctgctga agcacctgca cgcctttgcc aacagcctga agccagagca ggcctcaccc 2280
   tccgcccact cccacgccac cagccccctg gaggagttca aacgggctgc tgtcccgagg 2340
   ttcgtccagc agaaactcta cctcttcttg cagcattgct ttggccactg gcccctggac 2400
   gcatcgttca gagctgtcct ggagatgtgg ctgagctacc tgcagccgtg gcggtacgcg 2460
   cctgacaagc aggctccggg cagcgactcc cagccccggt gtgtgtcgga gaaatgggca 2520
   ccctttgtcc aggagaacct gctgatgtac accaagttgt ttgtgggctt tctgaaccgc 2580
   gcgctccgca cagacctggt cagccccaag cacgcgctca tggtgttccg agtggccaaa 2640
   gtctttgccc agcccaacct ggctgagatg attcagaaag gtgagcagct attcctggag 2700
   ccagagctgg tcatccccca ccgccagcac cgactcttca cggcccccac attcactggg 2760
   agcttcctgt caccctggcc accagcggtc actgatgcct ccttcaaggt gaagagccac 2820
   gtctacagcc tggagggcca ggactgcaag tacaccccga tgtttgggcc cgaggcccgc 2880
   accctggtcc tgcgcctcgc tcagctcatc acacaggcca aacacacagc caagtccatc 2940
   tccgaccagt gtgcggagag cccggctggc cactccttcc tctcatggct gggctttagc 3000
   tccatggaca ccaatggctc ctacacagcc aacgacctgg acgagatggg gcaagacagt 3060
   gtccggaaga cagatgaata cctggagaag gccctggagt acctgcgcca gatattccgg 3120
   ctcagcgaag cgcagctcag gcagttcaca ctcgccttgg gcaccaccca ggatgagaat 3180
   ggaaaaaagc aactccccga ctgcatcgtg ggtgaggacg gactcatcct tacgcccctg 3240
   gggcggtacc agatcatcaa tgggctgcga aggtttgaaa ttgagtacca gggggacccg 3300
   gagctgcagc ccatccggag ctatgagatc gccagcttgg tccgcacact ctttaggctg 3360
   tcgtctgcca tcaaccacag atttgcagga cagatggcgg ctctgtgttc ccgggatgac 3420
   ttcctcggca gcttctgtcg ctaccacctc acagaacctg ggctggccag caggcacctg 3480
   ctgagccctg tggggcggag gcaggtggcc ggccacaccc gcggccccag gctcagcctg 3540
   cgcttcctgg gcagttaccg gacgctggtc tcgctgctgc tggccttctt cgtggcctct 3600
   ctgttctgcg tcgggcccct cccatgcacg ctgctgctca ccctgggcta tgtcctctac 3660
   gcctctgcca tgacactgct gaccgagcgg gggaagctgc accagccctg agaattctgc 3720
   agtcgacggt accgcgggcc cgggatccac cggatctaga taactgatca taatcagcca 3780
   taccacattt gtagaggttt tacttgcttt aaaaaacctc ccacacctcc ccctgaacct 3840
   gaaacataaa atgaatgcaa ttgttgttgt taacttgttt attgcagctt ataatggtta 3900
   caaataaagc aatagcatca caaatttcac aaataaagca tttttttcac tgcattctag 3960
   ttgtggtttg tccaaactca tcaatgtatc ttaacgcgta aattgtaagc gttaatattt 4020
   tgttaaaatt cgcgttaaat ttttgttaaa tcagctcatt ttttaaccaa taggccgaaa 4080
   tcggcaaaat cccttataaa tcaaaagaat agaccgagat agggttgagt gttgttccag 4140
   tttggaacaa gagtccacta ttaaagaacg tggactccaa cgtcaaaggg cgaaaaaccg 4200
   tctatcaggg cgatggccca ctacgtgaac catcacccta atcaagtttt ttggggtcga 4260
   ggtgccgtaa agcactaaat cggaacccta aagggagccc ccgatttaga gcttgacggg 4320
   gaaagccggc gaacgtggcg agaaaggaag ggaagaaagc gaaaggagcg ggcgctaggg 4380
   cgctggcaag tgtagcggtc acgctgcgcg taaccaccac acccgccgcg cttaatgcgc 4440
   cgctacaggg cgcgtcaggt ggcacttttc ggggaaatgt gcgcggaacc cctatttgtt 4500
   tatttttcta aatacattca aatatgtatc cgctcatgag acaataaccc tgataaatgc 4560
   ttcaataata ttgaaaaagg aagagtcctg aggcggaaag aaccagctgt ggaatgtgtg 4620
   tcagttaggg tgtggaaagt ccccaggctc cccagcaggc agaagtatgc aaagcatgca 4680
   tctcaattag tcagcaacca ggtgtggaaa gtccccaggc tccccagcag gcagaagtat 4740
   gcaaagcatg catctcaatt agtcagcaac catagtcccg cccctaactc cgcccatccc 4800
   gcccctaact ccgcccagtt ccgcccattc tccgccccat ggctgactaa ttttttttat 4860
   ttatgcagag gccgaggccg cctcggcctc tgagctattc cagaagtagt gaggaggctt 4920
   ttttggaggc ctaggctttt gcaaagatcg atcaagagac aggatgagga tcgtttcgca 4980
   tgattgaaca agatggattg cacgcaggtt ctccggccgc ttgggtggag aggctattcg 5040
   gctatgactg ggcacaacag acaatcggct gctctgatgc cgccgtgttc cggctgtcag 5100
   cgcaggggcg cccggttctt tttgtcaaga ccgacctgtc cggtgccctg aatgaactgc 5160
   aagacgaggc agcgcggcta tcgtggctgg ccacgacggg cgttccttgc gcagctgtgc 5220
   tcgacgttgt cactgaagcg ggaagggact ggctgctatt gggcgaagtg ccggggcagg 5280
   atctcctgtc atctcacctt gctcctgccg agaaagtatc catcatggct gatgcaatgc 5340
   ggcggctgca tacgcttgat ccggctacct gcccattcga ccaccaagcg aaacatcgca 5400
   tcgagcgagc acgtactcgg atggaagccg gtcttgtcga tcaggatgat ctggacgaag 5460
   agcatcaggg gctcgcgcca gccgaactgt tcgccaggct caaggcgagc atgcccgacg 5520
   gcgaggatct cgtcgtgacc catggcgatg cctgcttgcc gaatatcatg gtggaaaatg 5580
   gccgcttttc tggattcatc gactgtggcc ggctgggtgt ggcggaccgc tatcaggaca 5640
   tagcgttggc tacccgtgat attgctgaag agcttggcgg cgaatgggct gaccgcttcc 5700
   tcgtgcttta cggtatcgcc gctcccgatt cgcagcgcat cgccttctat cgccttcttg 5760
   acgagttctt ctgagcggga ctctggggtt cgaaatgacc gaccaagcga cgcccaacct 5820
   gccatcacga gatttcgatt ccaccgccgc cttctatgaa aggttgggct tcggaatcgt 5880
   tttccgggac gccggctgga tgatcctcca gcgcggggat ctcatgctgg agttcttcgc 5940
   ccaccctagg gggaggctaa ctgaaacacg gaaggagaca ataccggaag gaacccgcgc 6000
   tatgacggca ataaaaagac agaataaaac gcacggtgtt gggtcgtttg ttcataaacg   6060
   cggggttcgg tcccagggct ggcactctgt cgatacccca ccgagacccc attggggcca   6120
   atacgcccgc gtttcttcct tttccccacc ccacccccca agttcgggtg aaggcccagg 6180
   gctcgcagcc aacgtcgggg cggcaggccc tgccatagcc tcaggttact catatatact 6240
   ttagattgat ttaaaacttc atttttaatt taaaaggatc taggtgaaga tcctttttga 6300
   taatctcatg accaaaatcc cttaacgtga gttttcgttc cactgagcgt cagaccccgt 6360
   agaaaagatc aaaggatctt cttgagatcc tttttttctg cgcgtaatct gctgcttgca 6420
   aacaaaaaaa ccaccgctac cagcggtggt ttgtttgccg gatcaagagc taccaactct 6480
   ttttccgaag gtaactggct tcagcagagc gcagatacca aatactgtcc ttctagtgta    6540
   gccgtagtta ggccaccact tcaagaactc tgtagcaccg cctacatacc tcgctctgct    6600
   aatcctgtta ccagtggctg ctgccagtgg cgataagtcg tgtcttaccg ggttggactc    6660
   aagacgatag ttaccggata aggcgcagcg gtcgggctga acggggggtt cgtgcacaca    6720
   gcccagcttg gagcgaacga cctacaccga actgagatac ctacagcgtg agctatgaga    6780
   aagcgccacg cttcccgaag ggagaaaggc ggacaggtat ccggtaagcg gcagggtcgg    6840
   aacaggagag cgcacgaggg agcttccagg gggaaacgcc tggtatcttt atagtcctgt    6900
   cgggtttcgc cacctctgac ttgagcgtcg atttttgtga tgctcgtcag gggggcggag    6960
   cctatggaaa aacgccagca acgcggcctt tttacggttc ctggcctttt gctggccttt    7020
   tgctcacatg ttctttcctg cgttatcccc tgattctgtg gataaccgta ttaccgccat    7080
   gcattagtta ttaatagtaa tcaattacgg ggtcattagt tcatagccca tatatggagt    7140
   tccgcgttac ataacttacg gtaaatggcc cgcctggctg accgcccaac gacccccgcc    7200
   cattgacgtc aataatgacg tatgttccca tagtaacgcc aatagggact ttccattgac    7260
   gtcaatgggt ggagtattta cggtaaactg cccacttggc agtacatcaa gtgtatca      7318
<210> 16
   <211> 11
   <212> PRT
   <213> Bos taurus
<400> 16
   Lys Gly Leu Pro Tyr Leu Glu Gln Leu Phe Arg
   1               5                   10
<210> 17
   <211> 11
   <212> PRT
   <213> Homo sapiens
<400> 17
   Lys Ala Leu Glu Tyr Leu Arg Gln Ile Phe Arg
   1               5                   10
<210> 18
   <211> 178
   <212> PRT
   <213> Homo sapiens
<400> 18
   Cys Leu Gln Gly Arg Val Asn Pro Val Glu Tyr Ser Ile Val Met Glu
   1               5                   10                  15
   Phe Leu Asp Pro Gly Gly Pro Met Met Lys Leu Val Tyr Lys Leu Gln
               20                  25                  30
   Ala Glu Asp Tyr Lys Phe Asp Phe Pro Val Ser Tyr Leu Pro Gly Pro
           35                  40                  45
   Val Lys Ala Ser Ile Gln Glu Cys Ile Leu Pro Asp Ser Pro Leu Tyr
       50                  55                  60
   His Asn Lys Val Gln Phe Thr Pro Thr Gly Gly Leu Gly Leu Asn Leu
   65                  70                  75                  80
   Ala Leu Asn Pro Phe Glu Tyr Tyr Ile Phe Phe Phe Ala Leu Ser Leu
                   85                  90                  95
   Ile Thr Gln Lys Pro Leu Pro Val Ser Leu His Val Arg Thr Ser Asp
               100                 105                 110
   Cys Ala Tyr Phe Ile Leu Val Asp Arg Tyr Leu Ser Trp Phe Leu Pro
           115                 120                 125
   Thr Glu Gly Ser Val Pro Pro Pro Leu Ser Ser Ser Pro Gly Gly Thr
       130                 135                 140
   Ser Pro Ser Pro Pro Pro Arg Thr Pro Ala Ile Pro Phe Ala Ser Tyr
   145                 150                 155                 160
   Gly Leu His His Thr Ser Leu Leu Lys Arg His Ile Ser His Gln Thr
                   165                 170                 175
   Ser Val
<210> 19
   <211> 69
   <212> PRT
   <213> Homo sapiens
<400> 19
   Tyr Gln Lys Met Gln Ser Pro His Ala Lys Leu Glu Val Leu His Tyr
   1 5 10 15
   Arg Leu Ser Val Ser Ser Ala Leu Tyr Ser Pro Ala Gln Pro Ser Leu
   20 25 30
   Gln Ala Leu His Ala Tyr Gln Glu Ser Phe Thr Pro Thr Glu Glu His
   35 40 45
   Val Leu Val Val Arg Leu Leu Leu Lys His Leu His Ala Phe Ala Asn
   50 55 60
   Ser Leu Lys Pro Glu
   65
<210> 20
   <211> 51
   <212> PRT
   <213> Homo sapiens
<400> 20
   Lys Arg Ala Ala Val Pro Arg Phe Val Gln Gln Lys Leu Tyr Leu Phe
   1 5 10 15
   Leu Gln His Cys Phe Gly His Trp Pro Leu Asp Ala Ser Phe Arg Ala
   20 25 30
   Val Leu Glu Met Trp Leu Ser Tyr Leu Gln Pro Trp Arg Tyr Ala Pro
   35 40 45
   Asp Lys Gln
   50
<210> 21
   <211> 79
   <212> PRT
   <213> Homo sapiens
<400> 21
   Val Ser Glu Lys Trp Ala Pro Phe Val Gln Glu Asn Leu Leu Met Tyr
   1 5 10 15
   Thr Lys Leu Phe Val Gly Phe Leu Asn Arg Ala Leu Arg Thr Asp Leu
   20 25 30
   Val Ser Pro Lys His Ala Leu Met Val Phe Arg Val Ala Lys Val Phe
   35 40 45
   Ala Gln Pro Asn Leu Ala Glu Met Ile Gln Lys Gly Glu Gln Leu Phe
   50 55 60
   Leu Glu Pro Glu Leu Val Ile Pro His Arg Gln His Arg Leu Phe
   65 70 75
<210> 22
   <211> 47
   <212> PRT
   <213> Homo sapiens
<400> 22
   Ser Phe Lys Val Lys Ser His Val Tyr Ser Leu Glu Gly Gln Asp Cys
   1 5 10 15
   Lys Tyr Thr Pro Met Phe Gly Pro Glu Ala Arg Thr Leu Val Leu Arg
   20 25 30
   Leu Ala Gln Leu Ile Thr Gln Ala Lys His Thr Ala Lys Ser Ile
   35 40 45
<210> 23
   <211> 41
   <212> PRT
   <213> Homo sapiens
<400> 23
   Thr Ala Asn Asp Leu Asp Glu Met Gly Gln Asp Ser Val Arg Lys Thr
   1               5                   10                  15
   Asp Glu Tyr Leu Glu Lys Ala Leu Glu Tyr Leu Arg Gln Ile Phe Arg
               20                  25                  30
   Leu Ser Glu Ala Gln Leu Arg Gln Phe
           35                  40
<210> 24
   <211> 132
   <212> PRT
   <213> Homo sapiens
<400> 24
   Leu Ile Leu Thr Pro Leu Gly Arg Tyr Gln Ile Ile Asn Gly Leu Arg
   1               5                   10                  15
   Arg Phe Glu Ile Glu Tyr Gln Gly Asp Pro Glu Leu Gln Pro Ile Arg
               20                  25                  30
   Ser Tyr Glu Ile Ala Ser Leu Val Arg Thr Leu Phe Arg Leu Ser Ser
           35                  40                  45
   Ala Ile Asn His Arg Phe Ala Gly Gln Met Ala Ala Leu Cys Ser Arg
       50                  55                  60
   Asp Asp Phe Leu Gly Ser Phe Cys Arg Tyr His Leu Thr Glu Pro Gly
   65                  70                  75                  80
   Leu Ala Ser Arg His Leu Leu Ser Pro Val Gly Arg Arg Gln Val Ala
                   85                  90                  95
   Gly His Thr Arg Gly Pro Arg Leu Ser Leu Arg Phe Leu Gly Ser Tyr
               100                 105                 110
   Arg Thr Leu Val Ser Leu Leu Leu Ala Phe Phe Val Ala Ser Leu Phe
           115                 120                 125
   Cys Val Gly Pro
       130
<210> 25
   <211> 25
   <212> DNA
   <213> Artificial
<220>
   <223> forward primer for nSMase3
<400> 25
   ggaattctga tgacgacttt cggcg 25
<210> 26
   <211> 25
   <212> DNA
   <213> artificial
<220>
   <223> reverse primer nSMase3
<400> 26
   gtcgacgtca gggctggtgc agctt 25
<210> 27
   <211> 25
   <212> DNA
   <213> artificial
<220>
   <223> forward primer nSMase2
<400> 27
   gggatccatg gttttgtaca cgacc 25
<210> 28
   <211> 24
   <212> DNA
   <213> artificial
<220>
   <223> reverse primer nSMase2
<400> 28
   cctcgagcta tgcctcctcc tccc 24
<210> 29
   <211> 8078
   <212> DNA
   <213> Artificial
<220>
   <223> AdCGIpnSMase3; AdCGI vector encoding nSMase3
<400> 29
   catcatcaat aatatacctt attttggatt gaagccaata tgataatgag ggggtggagt 60
   ttgtgacgtg gcgcggggcg tgggaacggg gcgggtgacg tagtagtgtg gcggaagtgt 120
   gatgttgcaa gtgtggcgga acacatgtaa gcgacggatg tggcaaaagt gacgtttttg 180
   gtgtgcgccg gtgtacacag gaagtgacaa ttttcgcgcg gttttaggcg gatgttgtag 240
   taaatttggg cgtaaccgag taagatttgg ccattttcgc gggaaaactg aataagagga 300
   agtgaaatct gaataatttt gtgttactca tagcgcgtaa tatttgtcta gggccgcggg 360
   gactttgacc gtttacgtgg agactcgccc aggtgttttt ctcaggtgtt ttccgcgttc 420
   cgggtcaaag ttggcgtttt attattatag tcagtaatag taatcaatta cggggtcatt 480
   agttcatagc ccatatatgg agttccgcgt tacataactt acggtaaatg gcccgcctgg 540
   ctgaccgccc aacgaccccc gcccattgac gtcaataatg acgtatgttc ccatagtaac 600
   gccaataggg actttccatt gacgtcaatg ggtggagtat ttacggtaaa ctgcccactt 660
   ggcagtacat caagtgtatc atatgccaag tacgccccct attgacgtca atgacggtaa 720
   atggcccgcc tggcattatg cccagtacat gaccttatgg gactttccta cttggcagta 780
   catctacgta ttagtcatcg ctattaccat ggtgatgcgg ttttggcagt acatcaatgg 840
   gcgtggatag cggtttgact cacggggatt tccaagtctc caccccattg acgtcaatgg 900
   gagtttgttt tggcaccaaa atcaacggga ctttccaaaa tgtcgtaaca actccgcccc 960
   attgacgcaa atgggcggta ggcgtgtacg gtgggaggtc tatataagca gagctggttt 1020
   agtgaaccgt cagatccgct agcgctaccg gtcgccacca tggtgagcaa gggcgaggag 1080
   ctgttcaccg gggtggtgcc catcctggtc gagctggacg gcgacgtaaa cggccacaag 1140
   ttcagcgtgt ccggcgaggg cgagggcgat gccacctacg gcaagctgac cctgaagttc 1200
   atctgcacca ccggcaagct gcccgtgccc tggcccaccc tcgtgaccac cctgacctac 1260
   ggcgtgcagt gcttcagccg ctaccccgac cacatgaagc agcacgactt cttcaagtcc 1320
   gccatgcccg aaggctacgt ccaggagcgc accatcttct tcaaggacga cggcaactac 1380
   aagacccgcg ccgaggtgaa gttcgagggc gacaccctgg tgaaccgcat cgagctgaag 1440
   ggcatcgact tcaaggagga cggcaacatc ctggggcaca agctggagta caactacaac 1500
   agccacaacg tctatatcat ggccgacaag cagaagaacg gcatcaaggt gaacttcaag 1560
   atccgccaca acatcgagga cggcagcgtg cagctcgccg accactacca gcagaacacc 1620
   cccatcggcg acggccccgt gctgctgccc gacaaccact acctgagcac ccagtccgcc 1680
   ctgagcaaag accccaacga gaagcgcgat cacatggtcc tgctggagtt cgtgaccgcc 1740
   gccgggatca ctctcggcat ggacgagctg tacaagtgtg gcggctagta ctccggtatt 1800
   gcggtaccct tgtacgcctg ttttatactc ccttcccgta acttagacgc acaaaaccaa 1860
   gttcaataga agggggtaca aaccagtacc accacgaaca agcacttctg tttccccggt 1920
   gatgtcgtat agactgcttg cgtggttgaa agcgacggat ccgttatccg cttatgtact 1980
   tcgagaagcc cagtaccacc tcggaatctt cgatgcgttg cgctcagcac tcaaccccag 2040
   agtgtagctt aggctgatga gtctggacat ccctcaccgg tgacggtggt ccaggctgcg 2100
   ttggcggcct acctatggct aacgccatgg gacgctagtt gtgaacaagg tgtgaagagc 2160
   ctattgagct acataagaat cctccggccc ctgaatgcgg ctaatcccaa cctcggagca 2220
   ggtggtcaca aaccagtgat tggcctgtcg taacgcgcaa gtccgtggcg gaaccgacta 2280
   ctttgggtgt ccgtgtttcc ttttatttta ttgtggctgc ttatggtgac aatcacagat 2340
   tgttatcata aagcgaattg gataggatca agcttatcga taccgtcgac ctcgagatga 2400
   cgactttcgg cgccgtggcg gaatggcggc ttccatctct gaggcgagcg acgctatgga 2460
   tcccacagtg gtttgctaag aaggccattt tcaactctcc actggaggct gctatggcgt 2520
   tccctcacct gcagcagccc agctttctac tggctagcct gaaagctgac tctataaata 2580
   agccctttgc acagcagtgc caagacttgg ttaaagtcat tgaggacttt ccagcaaagg 2640
   agctgcacac catcttccca tggctggtag aaagcatttt tggcagccta gatggtgtcc 2700
   tcgttggctg gaacctccgc tgcttacagg ggcgcgtgaa tcctgtggag tacagcatcg 2760
   tgatggaatt tctcgaccct ggtggcccaa tgatgaagtt ggtttataag cttcaagctg 2820
   aagactataa gttcgacttt cctgtctcct acttgcctgg tcctgtgaag gcgtccatcc 2880
   aggagtgcat cctccctgac agtcctctgt accacaacaa ggtccagttc acccctactg 2940
   ggggccttgg tctgaacttg gccctgaatc cgttcgagta ttacatattc ttctttgcct 3000
   tgagcctcat cactcagaag ccacttcctg tgtccctcca cgtccgtact tcagactgtg 3060
   cctatttcat cctggtggac aggtacctgt catggttcct gcccaccgaa ggcagtgtgc 3120
   ccccaccact ctcctccagc ccagggggga ccagcccctc accacctccc aggacaccag 3180
   ccataccctt tgcttcctat ggcctccacc acactagcct cctaaagcga cacatctctc 3240
   atcagacgtc tgtgaatgca gaccccgcct cccacgagat ctggaggtca gaaactctgc 3300
   tccaggtttt tgttgaaatg tggcttcatc actattcctt ggagatgtat caaaaaatgc 3360
   agtcccctca tgccaagctg gaggttctgc actaccgact cagtgtctcc agcgccctct 3420
   acagccccgc ccaacccagc ctccaggccc tccacgccta ccaagagtcg ttcacgccta 3480
   ctgaggagca tgtgttggtg gtgcgcctgc tgctgaagca cctgcacgcc tttgccaaca 3540
   gcctgaagcc agagcaggcc tcaccctccg cccactccca cgccaccagc cccctggagg 3600
   agttcaaacg ggctgctgtc ccgaggttcg tccagcagaa actctacctc ttcttgcagc 3660
   attgctttgg ccactggccc ctggacgcat cgttcagagc tgtcctggag atgtggctga 3720
   gctacctgca gccgtggcgg tacgcgcctg acaagcaggc tccgggcagc gactcccagc 3780
   cccggtgtgt gtcggagaaa tgggcaccct ttgtccagga gaacctgctg atgtacacca 3840
   agttgtttgt gggctttctg aaccgcgcgc tccgcacaga cctggtcagc cccaagcacg 3900
   cgctcatggt gttccgagtg gccaaagtct ttgcccagcc caacctggct gagatgattc 3960
   agaaaggtga gcagctattc ctggagccag agctggtcat cccccaccgc cagcaccgac 4020
   tcttcacggc ccccacattc actgggagct tcctgtcacc ctggccacca gcggtcactg 4080
   atgcctcctt caaggtgaag agccacgtct acagcctgga gggccaggac tgcaagtaca 4140
   ccccgatgtt tgggcccgag gcccgcaccc tggtcctgcg cctcgctcag ctcatcacac 4200
   aggccaaaca cacagccaag tccatctccg accagtgtgc ggagagcccg gctggccact 4260
   ccttcctctc atggctgggc tttagctcca tggacaccaa tggctcctac acagccaacg 4320
   acctggacga gatggggcaa gacagtgtcc ggaagacaga tgaatacctg gagaaggccc 4380
   tggagtacct gcgccagata ttccggctca gcgaagcgca gctcaggcag ttcacactcg 4440
   ccttgggcac cacccaggat gagaatggaa aaaagcaact ccccgactgc atcgtgggtg 4500
   aggacggact catccttacg cccctggggc ggtaccagat catcaatggg ctgcgaaggt 4560
   ttgaaattga gtaccagggg gacccggagc tgcagcccat ccggagctat gagatcgcca 4620
   gcttggtccg cacactcttt aggctgtcgt ctgccatcaa ccacagattt gcaggacaga 4680
   tggcggctct gtgttcccgg gatgacttcc tcggcagctt ctgtcgctac cacctcacag 4740
   aacctgggct ggccagcagg cacctgctga gccctgtggg gcggaggcag gtggccggcc 4800
   acacccgcgg ccccaggctc agcctgcgct tcctgggcag ttaccggacg ctggtctcgc 4860
   tgctgctggc cttcttcgtg gcctctctgt tctgcgtcgg gcccctccca tgcacgctgc 4920
   tgctcaccct gggctatgtc ctctacgcct ctgccatgac actgctgacc gagcggggga 4980
   agctgcacca gccctgagaa ttctgcagtc gacggtaccg cgggcccggg atccaccgga 5040
   tctagataac tgatcataat cagccatacc acatttgtag aggttttact tgctttaaaa 5100
   aacctcccac acctccccct gaacctgaaa cataaaatga atgcaattgt tgttgttaac 5160
   ttgtttattg cagcttataa tggttacaaa taaagcaata gcatcacaaa tttcacaaat 5220
   aaagcatttt tttcactgca ttctagttgt ggtttgtcca aactcatcaa tgtatcttat 5280
   catgtctgga tcatcgatcc ataacttcgt ataatgtatg ctatacgaag ttatccagat 5340
   ctggttctat agtgtcacct aaatcgtatg tgtatgatac ataaggttat gtattaattg 5400
   tagccgcgtt ctaacgacaa tatgtccata gggcccctac gtcacccgcc ccgttcccac 5460
   gccccgcgcc acgtcacaaa ctccaccccc tcattatcat attggcttca atccaaaata 5520
   aggtatatta ttgatgatgg ccgcagcggc cctggcgtaa atagcgaaga ggcccgcacc 5580
   gatcgccctt cccaacagtt gcgcagcctg aatggcgaat gggacgcgcc ctgtagcggc 5640
   gcattaagcg cggcgggtgt ggtggttacg cgcagcgtga ccgctacact tgccagcgcc 5700
   ctagcgcccg ctcctttcgc tttcttccct tcctttctcg ccacgttcgc cggctttccc 5760
   cgtcaagctc taaatcgggg gctcccttta gggttccgat ttagtgcttt acggcacctc 5820
   gaccccaaaa aacttgatta gggtgatggt tcacgtagtg ggccatcgcc ctgatagacg 5880
   gtttttcgcc ctttgacgtt ggagtccacg ttctttaata gtggactctt gttccaaact 5940
   ggaacaacac tcaaccctat ctcggtctat tcttttgatt tataagggat tttgccgatt 6000
   tcggcctatt ggttaaaaaa tgagctgatt taacaaaaat ttaacgcgaa ttttaacaaa 6060
   atattaacgc ttacaattta ggtggcactt ttcggggaaa tgtgcgcgga acccctattt 6120
   gtttattttt ctaaatacat tcaaatatgt atccgctcat gagacaataa ccctgataaa 6180
   tgcttcaata atattgaaaa aggaagagta tgagtattca acatttccgt gtcgccctta 6240
   ttcccttttt tgcggcattt tgccttcctg tttttgctca cccagaaacg ctggtgaaag 6300
   taaaagatgc tgaagatcag ttgggtgcac gagtgggtta catcgaactg gatctcaaca 6360
   gcggtaagat ccttgagagt tttcgccccg aagaacgttt tccaatgatg agcactttta 6420
   aagttctgct atgtggcgcg gtattatccc gtattgacgc cgggcaagag caactcggtc 6480
   gccgcataca ctattctcag aatgacttgg ttgagtactc accagtcaca gaaaagcatc 6540
   ttacggatgg catgacagta agagaattat gcagtgctgc cataaccatg agtgataaca 6600
   ctgcggccaa cttacttctg acaacgatcg gaggaccgaa ggagctaacc gcttttttgc 6660
   acaacatggg ggatcatgta actcgccttg atcgttggga accggagctg aatgaagcca 6720
   taccaaacga cgagcgtgac accacgatgc ctgtagcaat ggcaacaacg ttgcgcaaac 6780
   tattaactgg cgaactactt actctagctt cccggcaaca attaatagac tggatggagg 6840
   cggataaagt tgcaggacca cttctgcgct cggcccttcc ggctggctgg tttattgctg 6900
   ataaatctgg agccggtgag cgtgggtctc gcggtatcat tgcagcactg gggccagatg 6960
   gtaagccctc ccgtatcgta gttatctaca cgacggggag tcaggcaact atggatgaac 7020
   gaaatagaca gatcgctgag ataggtgcct cactgattaa gcattggtaa ctgtcagacc 7080
   aagtttactc atatatactt tagattgatt taaaacttca tttttaattt aaaaggatct 7140
   aggtgaagat cctttttgat aatctcatga ccaaaatccc ttaacgtgag ttttcgttcc 7200
   actgagcgtc agaccccgta gaaaagatca aaggatcttc ttgagatcct ttttttctgc 7260
   gcgtaatctg ctgcttgcaa acaaaaaaac caccgctacc agcggtggtt tgtttgccgg 7320
   atcaagagct accaactctt tttccgaagg taactggctt cagcagagcg cagataccaa 7380
   atactgtcct tctagtgtag ccgtagttag gccaccactt caagaactct gtagcaccgc 7440
   ctacatacct cgctctgcta atcctgttac cagtggctgc tgccagtggc gataagtcgt 7500
   gtcttaccgg gttggactca agacgatagt taccggataa ggcgcagcgg tcgggctgaa 7560
   cggggggttc gtgcacacag cccagcttgg agcgaacgac ctacaccgaa ctgagatacc 7620
   tacagcgtga gctatgagaa agcgccacgc ttcccgaagg gagaaaggcg gacaggtatc 7680
   cggtaagcgg cagggtcgga acaggagagc gcacgaggga gcttccaggg ggaaacgcct 7740
   ggtatcttta tagtcctgtc gggtttcgcc acctctgact tgagcgtcga tttttgtgat 7800
   gctcgtcagg ggggcggagc ctatggaaaa acgccagcaa cgcggccttt ttacggttcc 7860
   tggccttttg ctggcctttt gctcacatgt tctttcctgc gttatcccct gattctgtgg 7920
   ataaccgtat taccgccttt gagtgagctg ataccgctcg ccgcagccga acgaccgagc 7980
   gcagcgagtc agtgagcgag gaagcggaag agcgcccaat acgcaaaccg cctctccccg 8040
   cgcgttggcc gattcattaa tgcaggggcc gctgcggc 8078
<210> 30
   <211> 60
   <212> DNA
   <213> Artificial
<220>
   <223> sense strand of shRNA against nSMase3
<400> 30
   gatctcccct gctgatgtac accaagttca agagacttgg tgtacatcag caggttttta 60
<210> 31
   <211> 60
   <212> DNA
   <213> Artificial
<220>
   <223> antisense strand of shRNA against nSMase3
<400> 31
   agcttaaaaa cctgctgatg tacaccaagt ctcttgaact tggtgtacat cagcagggga 60
<210> 32
   <211> 2935
   <212> DNA
   <213> Artificial
<220>
   <223> pENTRY-shnSMase3; vector encoding shRNA to nSMase3
<400> 32
   ctttcctgcg ttatcccctg attctgtgga taaccgtatt accgcctttg agtgagctga     60
   taccgctcgc cgcagccgaa cgaccgagcg cagcgagtca gtgagcgagg aagcggaaga    120
   gcgcccaata cgcaaaccgc ctctccccgc gcgttggccg attcattaat gcagctggca    180
   cgacaggttt cccgactgga aagcgggcag tgagcgcaac gcaattaata cgcgtaccgc    240
   tagccaggaa gagtttgtag aaacgcaaaa aggccatccg tcaggatggc cttctgctta    300
   gtttgatgcc tggcagttta tggcgggcgt cctgcccgcc accctccggg ccgttgcttc    360
   acaacgttca aatccgctcc cggcggattt gtcctactca ggagagcgtt caccgacaaa    420
   caacagataa aacgaaaggc ccagtcttcc gactgagcct ttcgttttat ttgatgcctg    480
   gcagttccct actctcgcgt taacgctagc atggatgttt tcccagtcac gacgttgtaa 540
   aacgacggcc agtcttaagc tcgggcccca aataatgatt ttattttgac tgatagtgac 600
   ctgttcgttg caacaaattg atgagcaatg cttttttata atgccaactt tgtacaaaaa 660
   agcaggctag cattcctgca gttctagaac tagtggatcc cccgggctgc aggaattcga 720
   acgctgacgt catcaacccg ctccaaggaa tcgcgggccc agtgtcacta ggcgggaaca 780
   cccagcgcgc gtgcgccctg gcaggaagat ggctgtgagg gacaggggag tggcgccctg 840
   caatatttgc atgtcgctat gtgttctggg aaatcaccat aaacgtgaaa tgtctttgga 900
   tttgggaatc ttataagttc tgtatgagac cacagatctc ccctgctgat gtacaccaag 960
   ttcaagagac ttggtgtaca tcagcaggtt tttaagctta tcgataccgt cgacctcgag 1020
   ggggggcccg gtaccaatcg atttaaatag ctcgctagca cccagctttc ttgtacaaag 1080
   ttggcattat aagaaagcat tgcttatcaa tttgttgcaa cgaacaggtc actatcagtc 1140
   aaaataaaat cattatttgc catccagctg atatccccta tagtgagtcg tattacatgg 1200
   tcatagctgt ttcctggcag ctctggcccg tgtctcaaaa tctctgatgt tacattgcac 1260
   aagataaaat aatatcatca tgaacaataa aactgtctgc ttacataaac agtaatacaa 1320
   ggggtgttat gagccatatt caacgggaaa cgtcgaggcc gcgattaaat tccaacatgg 1380
   atgctgattt atatgggtat aaatgggctc gcgataatgt cgggcaatca ggtgcgacaa 1440
   tctatcgctt gtatgggaag cccgatgcgc cagagttgtt tctgaaacat ggcaaaggta 1500
   gcgttgccaa tgatgttaca gatgagatgg tcagactaaa ctggctgacg gaatttatgc 1560
   ctcttccgac catcaagcat tttatccgta ctcctgatga tgcatggtta ctcaccactg 1620
   cgatccccgg aaaaacagca ttccaggtat tagaagaata tcctgattca ggtgaaaata 1680
   ttgttgatgc gctggcagtg ttcctgcgcc ggttgcattc gattcctgtt tgtaattgtc 1740
   cttttaacag cgatcgcgta tttcgtctcg ctcaggcgca atcacgaatg aataacggtt 1800
   tggttgatgc gagtgatttt gatgacgagc gtaatggctg gcctgttgaa caagtctgga 1860
   aagaaatgca taaacttttg ccattctcac cggattcagt cgtcactcat ggtgatttct 1920
   cacttgataa ccttattttt gacgagggga aattaatagg ttgtattgat gttggacgag 1980
   tcggaatcgc agaccgatac caggatcttg ccatcctatg gaactgcctc ggtgagtttt 2040
   ctccttcatt acagaaacgg ctttttcaaa aatatggtat tgataatcct gatatgaata 2100
   aattgcagtt tcatttgatg ctcgatgagt ttttctaatc agaattggtt aattggttgt 2160
   aacactggca gagcattacg ctgacttgac gggacggcgc aagctcatga ccaaaatccc 2220
   ttaacgtgag ttacgcgtcg ttccactgag cgtcagaccc cgtagaaaag atcaaaggat 2280
   cttcttgaga tccttttttt ctgcgcgtaa tctgctgctt gcaaacaaaa aaaccaccgc 2340
   taccagcggt ggtttgtttg ccggatcaag agctaccaac tctttttccg aaggtaactg 2400
   gcttcagcag agcgcagata ccaaatactg ttcttctagt gtagccgtag ttaggccacc 2460
   acttcaagaa ctctgtagca ccgcctacat acctcgctct gctaatcctg ttaccagtgg 2520
   ctgctgccag tggcgataag tcgtgtctta ccgggttgga ctcaagacga tagttaccgg 2580
   ataaggcgca gcggtcgggc tgaacggggg gttcgtgcac acagcccagc ttggagcgaa 2640
   cgacctacac cgaactgaga tacctacagc gtgagctatg agaaagcgcc acgcttcccg 2700
   aagggagaaa ggcggacagg tatccggtaa gcggcagggt cggaacagga gagcgcacga 2760
   gggagcttcc agggggaaac gcctggtatc tttatagtcc tgtcgggttt cgccacctct 2820
   gacttgagcg tcgatttttg tgatgctcgt caggggggcg gagcctatgg aaaaacgcca 2880
   gcaacgcggc ctttttacgg ttcctggcct tttgctggcc ttttgctcac atgtt 2935
<210> 33
   <211> 6869
   <212> DNA
   <213> Artificial
<220>
   <223> pLenti-shnSMase3; lentiviral vector encoding shRNA to nSMase3
<400> 33
   aatgtagtct tatgcaatac tcttgtagtc ttgcaacatg gtaacgatga gttagcaaca     60
   tgccttacaa ggagagaaaa agcaccgtgc atgccgattg gtggaagtaa ggtggtacga    120
   tcgtgcctta ttaggaaggc aacagacggg tctgacatgg attggacgaa ccactgaatt    180
   gccgcattgc agagatattg tatttaagtg cctagctcga tacataaacg ggtctctctg    240
   gttagaccag atctgagcct gggagctctc tggctaacta gggaacccac tgcttaagcc    300
   tcaataaagc ttgccttgag tgcttcaagt agtgtgtgcc cgtctgttgt gtgactctgg    360
   taactagaga tccctcagac ccttttagtc agtgtggaaa atctctagca gtggcgcccg    420
   aacagggact tgaaagcgaa agggaaacca gaggagctct ctcgacgcag gactcggctt    480
   gctgaagcgc gcacggcaag aggcgagggg cggcgactgg tgagtacgcc aaaaattttg    540
   actagcggag gctagaagga gagagatggg tgcgagagcg tcagtattaa gcgggggaga    600
   attagatcgc gatgggaaaa aattcggtta aggccagggg gaaagaaaaa atataaatta    660
   aaacatatag tatgggcaag cagggagcta gaacgattcg cagttaatcc tggcctgtta    720
   gaaacatcag aaggctgtag acaaatactg ggacagctac aaccatccct tcagacagga    780
   tcagaagaac ttagatcatt atataataca gtagcaaccc tctattgtgt gcatcaaagg    840
   atagagataa aagacaccaa ggaagcttta gacaagatag aggaagagca aaacaaaagt    900
   aagaccaccg cacagcaagc ggccgctgat cttcagacct ggaggaggag atatgaggga    960
   caattggaga agtgaattat ataaatataa agtagtaaaa attgaaccat taggagtagc   1020
   acccaccaag gcaaagagaa gagtggtgca gagagaaaaa agagcagtgg gaataggagc   1080
   tttgttcctt gggttcttgg gagcagcagg aagcactatg ggcgcagcgt caatgacgct 1140
   gacggtacag gccagacaat tattgtctgg tatagtgcag cagcagaaca atttgctgag 1200
   ggctattgag gcgcaacagc atctgttgca actcacagtc tggggcatca agcagctcca 1260
   ggcaagaatc ctggctgtgg aaagatacct aaaggatcaa cagctcctgg ggatttgggg 1320
   ttgctctgga aaactcattt gcaccactgc tgtgccttgg aatgctagtt ggagtaataa 1380
   atctctggaa cagatttgga atcacacgac ctggatggag tgggacagag aaattaacaa 1440
   ttacacaagc ttaatacact ccttaattga agaatcgcaa aaccagcaag aaaagaatga 1500
   acaagaatta ttggaattag ataaatgggc aagtttgtgg aattggttta acataacaaa 1560
   ttggctgtgg tatataaaat tattcataat gatagtagga ggcttggtag gtttaagaat 1620
   agtttttgct gtactttcta tagtgaatag agttaggcag ggatattcac cattatcgtt 1680
   tcagacccac ctcccaaccc cgaggggacc cgacaggccc gaaggaatag aagaagaagg 1740
   tggagagaga gacagagaca gatccattcg attagtgaac ggatctcgac ggtatcggat 1800
   ctggcctccg cgccgggttt tggcgcctcc cgcgtaccga gctcggatcc actagtccag 1860
   tgtggtggaa ttctgcagat atcaacaagt ttgtacaaaa aagcaggcta gcattcctgc 1920
   agttctagaa ctagtggatc ccccgggctg caggaattcg aacgctgacg tcatcaaccc 1980
   gctccaagga atcgcgggcc cagtgtcact aggcgggaac acccagcgcg cgtgcgccct 2040
   ggcaggaaga tggctgtgag ggacagggga gtggcgccct gcaatatttg catgtcgcta 2100
   tgtgttctgg gaaatcacca taaacgtgaa atgtctttgg atttgggaat cttataagtt 2160
   ctgtatgaga ccacagatct cccctgctga tgtacaccaa gttcaagaga cttggtgtac 2220
   atcagcaggt ttttaagctt atcgataccg tcgacctcga ggggggcccc ggtaccaatc 2280
   gatttaaata gctcgctagc acccagcttt cttgtacaaa gtggttgata tccagcacag 2340
   tggcggccgc tcgagtctag agggcccgcg gttcgaaggt aagcctatcc ctaaccctct 2400
   cctcggtctc gattctacgc gtaccggtta gtaatgagtt tggaattaat tctgtggaat 2460
   gtgtgtcagt tagggtgtgg aaagtcccca ggctccccag gcaggcagaa gtatgcaaag 2520
   catgcatctc aattagtcag caaccaggtg tggaaagtcc ccaggctccc cagcaggcag 2580
   aagtatgcaa agcatgcatc tcaattagtc agcaaccata gtcccgcccc taactccgcc 2640
   catcccgccc ctaactccgc ccagttccgc ccattctccg ccccatggct gactaatttt 2700
   ttttatttat gcagaggccg aggccgcctc tgcctctgag ctattccaga agtagtgagg 2760
   aggctttttt ggaggcctag gcttttgcaa aaagctcccg ggagcttgta tatccatttt 2820
   cggatctgat cagcacgtgt tgacaattaa tcatcggcat agtatatcgg catagtataa 2880
   tacgacaagg tgaggaacta aaccatggcc aagcctttgt ctcaagaaga atccaccctc 2940
   attgaaagag caacggctac aatcaacagc atccccatct ctgaagacta cagcgtcgcc 3000
   agcgcagctc tctctagcga cggccgcatc ttcactggtg tcaatgtata tcattttact 3060
   gggggacctt gtgcagaact cgtggtgctg ggcactgctg ctgctgcggc agctggcaac 3120
   ctgacttgta tcgtcgcgat cggaaatgag aacaggggca tcttgagccc ctgcggacgg 3180
   tgccgacagg tgcttctcga tctgcatcct gggatcaaag ccatagtgaa ggacagtgat 3240
   ggacagccga cggcagttgg gattcgtgaa ttgctgccct ctggttatgt gtgggagggc 3300
   taagcacaat tcgagctcgg tacctttaag accaatgact tacaaggcag ctgtagatct 3360
   tagccacttt ttaaaagaaa aggggggact ggaagggcta attcactccc aacgaagaca 3420
   agatctgctt tttgcttgta ctgggtctct ctggttagac cagatctgag cctgggagct 3480
   ctctggctaa ctagggaacc cactgcttaa gcctcaataa agcttgcctt gagtgcttca 3540
   agtagtgtgt gcccgtctgt tgtgtgactc tggtaactag agatccctca gaccctttta 3600
   gtcagtgtgg aaaatctcta gcagtagtag ttcatgtcat cttattattc agtatttata 3660
   acttgcaaag aaatgaatat cagagagtga gaggaacttg tttattgcag cttataatgg 3720
   ttacaaataa agcaatagca tcacaaattt cacaaataaa gcattttttt cactgcattc 3780
   tagttgtggt ttgtccaaac tcatcaatgt atcttatcat gtctggctct agctatcccg 3840
   cccctaactc cgcccatccc gcccctaact ccgcccagtt ccgcccattc tccgccccat 3900
   ggctgactaa ttttttttat ttatgcagag gccgaggccg cctcggcctc tgagctattc 3960
   cagaagtagt gaggaggctt ttttggaggc ctagggacgt acccaattcg ccctatagtg 4020
   agtcgtatta cgcgcgctca ctggccgtcg ttttacaacg tcgtgactgg gaaaaccctg 4080
   gcgttaccca acttaatcgc cttgcagcac atcccccttt cgccagctgg cgtaatagcg 4140
   aagaggcccg caccgatcgc ccttcccaac agttgcgcag cctgaatggc gaatgggacg 4200
   cgccctgtag cggcgcatta agcgcggcgg gtgtggtggt tacgcgcagc gtgaccgcta 4260
   cacttgccag cgccctagcg cccgctcctt tcgctttctt cccttccttt ctcgccacgt 4320
   tcgccggctt tccccgtcaa gctctaaatc gggggctccc tttagggttc cgatttagtg 4380
   ctttacggca cctcgacccc aaaaaacttg attagggtga tggttcacgt agtgggccat 4440
   cgccctgata gacggttttt cgccctttga cgttggagtc cacgttcttt aatagtggac 4500
   tcttgttcca aactggaaca acactcaacc ctatctcggt ctattctttt gatttataag 4560
   ggattttgcc gatttcggcc tattggttaa aaaatgagct gatttaacaa aaatttaacg 4620
   cgaattttaa caaaatatta acgcttacaa tttaggtggc acttttcggg gaaatgtgcg 4680
   cggaacccct atttgtttat ttttctaaat acattcaaat atgtatccgc tcatgagaca 4740
   ataaccctga taaatgcttc aataatattg aaaaaggaag agtatgagta ttcaacattt 4800
   ccgtgtcgcc cttattccct tttttgcggc attttgcctt cctgtttttg ctcacccaga 4860
   aacgctggtg aaagtaaaag atgctgaaga tcagttgggt gcacgagtgg gttacatcga 4920
   actggatctc aacagcggta agatccttga gagttttcgc cccgaagaac gttttccaat 4980
   gatgagcact tttaaagttc tgctatgtgg cgcggtatta tcccgtattg acgccgggca 5040
   agagcaactc ggtcgccgca tacactattc tcagaatgac ttggttgagt actcaccagt 5100
   cacagaaaag catcttacgg atggcatgac agtaagagaa ttatgcagtg ctgccataac 5160
   catgagtgat aacactgcgg ccaacttact tctgacaacg atcggaggac cgaaggagct 5220
   aaccgctttt ttgcacaaca tgggggatca tgtaactcgc cttgatcgtt gggaaccgga 5280
   gctgaatgaa gccataccaa acgacgagcg tgacaccacg atgcctgtag caatggcaac 5340
   aacgttgcgc aaactattaa ctggcgaact acttactcta gcttcccggc aacaattaat 5400
   agactggatg gaggcggata aagttgcagg accacttctg cgctcggccc ttccggctgg 5460
   ctggtttatt gctgataaat ctggagccgg tgagcgtggg tctcgcggta tcattgcagc 5520
   actggggcca gatggtaagc cctcccgtat cgtagttatc tacacgacgg ggagtcaggc 5580
   aactatggat gaacgaaata gacagatcgc tgagataggt gcctcactga ttaagcattg 5640
   gtaactgtca gaccaagttt actcatatat actttagatt gatttaaaac ttcattttta 5700
   atttaaaagg atctaggtga agatcctttt tgataatctc atgaccaaaa tcccttaacg 5760
   tgagttttcg ttccactgag cgtcagaccc cgtagaaaag atcaaaggat cttcttgaga 5820
   tccttttttt ctgcgcgtaa tctgctgctt gcaaacaaaa aaaccaccgc taccagcggt 5880
   ggtttgtttg ccggatcaag agctaccaac tctttttccg aaggtaactg gcttcagcag 5940
   agcgcagata ccaaatactg ttcttctagt gtagccgtag ttaggccacc acttcaagaa 6000
   ctctgtagca ccgcctacat acctcgctct gctaatcctg ttaccagtgg ctgctgccag 6060
   tggcgataag tcgtgtctta ccgggttgga ctcaagacga tagttaccgg ataaggcgca 6120
   gcggtcgggc tgaacggggg gttcgtgcac acagcccagc ttggagcgaa cgacctacac 6180
   cgaactgaga tacctacagc gtgagctatg agaaagcgcc acgcttcccg aagggagaaa 6240
   ggcggacagg tatccggtaa gcggcagggt cggaacagga gagcgcacga gggagcttcc 6300
   agggggaaac gcctggtatc tttatagtcc tgtcgggttt cgccacctct gacttgagcg 6360
   tcgatttttg tgatgctcgt caggggggcg gagcctatgg aaaaacgcca gcaacgcggc 6420
   ctttttacgg ttcctggcct tttgctggcc ttttgctcac atgttctttc ctgcgttatc 6480
   ccctgattct gtggataacc gtattaccgc ctttgagtga gctgataccg ctcgccgcag 6540
   ccgaacgacc gagcgcagcg agtcagtgag cgaggaagcg gaagagcgcc caatacgcaa 6600
   accgcctctc cccgcgcgtt ggccgattca ttaatgcagc tggcacgaca ggtttcccga 6660
   ctggaaagcg ggcagtgagc gcaacgcaat taatgtgagt tagctcactc attaggcacc 6720
   ccaggcttta cactttatgc ttccggctcg tatgttgtgt ggaattgtga gcggataaca 6780
   atttcacaca ggaaacagct atgaccatga ttacgccaag cgcgcaatta accctcacta 6840
   aagggaacaa aagctggagc tgcaagctt                              6869

## Description

The present invention provides a neutral sphingomyelinase-3 (nSMase3), a nucleic acid encoding said nSMase3, a vector containing said nucleic acid, and cells and non-human organisms transformed or transfected with said nucleic acid sequence or vector. The invention furthermore relates to the use of said nSMase3 as pharmaceutical or diagnostic agent. Test systems for candidate active agents for their therapeutic potential in diseases connected with nSMase3 are also provided.

### Background

Dilated cardiomyopathy (DCM) is a heart-muscle disease characterized by ventricular dilatation and impaired systolic function. The incidence of DCM is 7/100,000 per year, and the prevalence is 37/100,000 (Codd, M. B. et al., Circulation 80:564-572 (1989); Rakar, S. et al., Eur. Heart J. 18:117-123 (1997)). DCM is a leading cause of heart failure and the most frequent indication for heart transplantation in young patients (Dec, W. G. and Fuster, V., N. Engl. J. Med. 331:1564-1575 (1994)). Heart failure is a progressive clinical syndrome, with a median survival time of 1.7 years in men and 3.2 years in women (Ho, K. K. L. et al., Circulation 88:107-115 (1993)). Most patients die from pump failure or sudden cardiac death. Although familial occurrence of DCM has been reported for the first time by Whitfield (Whitfield, A. G., Q. J. Med. 3:119-134 (1961)), the frequency of familial etiology has been systematically examined only recently (Mestroni, L. and Giacca, M., Curr. Opin. Cardiol. 12:303-309 (1997)). Systematic examination of relatives showed that probably >25% of DCM cases are of familial etiology (Michels, V. V. et al., N. Engl. J. Med. 326:77-82 (1992); Keeling, P. J. et al., Br. Heart J. 73:417-421 (1995); Grünig, E. et al., J. Am. Coll. Cardiol. 31:186-194 (1998)). The mode of inheritance in most families seems to be autosomal dominant (Michels, V. V. et al., N. Engl. J. Med. 326:77-82 (1992)), but autosomal recessive, X-linked, and mitochondrial transmission have also been reported (Mestroni, L. and Giacca, M., Curr. Opin. Cardiol. 12:303-309 (1997)).

To date more than 15 loci for autosomal dominant DCM have been reported: on chromosomes 1p1-q1 (CMD1A), 9q13-q25 (CMD1B), 10q21-q23 (CMD1C), 1q32 (CMD1D), 3p25-p22 (CMD1E), 6q23 (CMD1F), 2q31 (CMD1G) and 2q14-q22 (CMD1H), mutations in the DES gene on 2q35 (CMD 1I), 6q23-q24 (CMD1J), and 6q12-q16 (CMD1K), in the SGCD gene on 5q33 (CMD1L), in the CSRP3 gene on 11p15.1 (CMD1M), in the TCAP gene on 17q12 (CMD1N), and in the ABCC9 gene on 12p12.1 (CMD1O) (Kass, S. et al., Nat. Genet. 7:546-551 (1994); Durand, J. B. et al., Circulation 92:3387-3389 (1995); Krajinovic, M. et al., Am. J. Hum. Genet. 57:846-852 (1995); Bowles, K. R. et al., J. Clin. Invest. 98:1355-1360 (1996); Olson, T. M. and Keating, M. T., J. Clin. Invest. 97:528-532 (1996); Messina, D. N. et al., Am. J. Hum. Genet. 61:909-917 (1997); Siu, B. L. et al., Circulation 99:1022-1026 (1999); Jung, M. et al., Am. J. Hum. Genet. 65:1068-77 (1999), Li, D. et al., Circulation 100:461-464 (1999); Schonberger, J. et al., Nature Genet. 37:418-422 (2005); Sylvius, N. et al., A. J. Hum. Genet. 68 :241-246 (2001); Tsubata, S. et al., J. Clin. Invest. 106:655-662 (2000); Knoll, R. et al., Cell 111:943-955 (2002); Bienengraeber, M. et al., Nature Genet. 36:382-387 (2004)).

Currently, DCM is not curable. Treatment usually includes drug therapy and is aimed at minimizing symptoms and preventing the development of complications and progression of the disease. The drug therapy includes Angiotensin-converting-enzyme inhibitors, beta-blockers, diuretics and anticoagulants. Some patients deteriorate in spite of treatment and may require pacemaker implantation or cardiac transplantation. Thus, there is a need for new medicaments for therapy of DCM.

Sphingomyelinases (SMases, EC3.1.4.12) are sphingomyelin (SM) phosphodiesterases (SMPD) that catalyze hydrolysis of membrane SM to form ceramide and phosphatidylserine (PS). Ceramide has been suggested to play important roles in cell cycle arrest, apoptosis, inflammation, and the eukaryotic stress response (Gulbins, E. and Kolesnick, R., Oncogene 22:7070-7077 (2003); Hannun, Y. A. and Luberto, C., Trends Cell. Biol. 10:73-80 (2000); Kolesnick, R. N. and Krönke, M., Annu. Rev. Physiol. 60:643-665 (1998); Levade, T. and Jaffrezou, J. P., Biochim. Biophys. Acta 1438:1-17 (1999)). Although ceramide can be generated by *de novo* synthesis through ceramide synthase, it is generated from SM by the action of neutral or acid SMases for the majority of cellular responses.

Different SMases have been described in eukaryotes and prokaryotes and are distinguished by their subcellular localization, pH optima, and requirement for metal ions. Three mammalian SMases have been molecularly cloned. The best characterized of these enzymes is the acid SMase (SMPD1) that is predominantly located in lysosomes but also secreted in response to different ligand-receptor interactions at the cell surface (Schuchman, E. H. et al., J. Biol. Chem. 266:8531-8539 (1991)). SMPD1-deficiency results in lipid storage disorders that manifest in Niemann Pick disease (Ferlinz, K. et al., Biochem. Biophys. Res. Commun. 179:1187-1191 (1991)). The regulated breakdown of SM to ceramide by activation of SMPD1 has been implicated in numerous cell responses from cell survival to apoptosis (Gulbins, E. and Kolesnick, R., Oncogene 22:7070-7077 (2003); Hannun, Y. A. and Luberto, C., Trends Cell. Biol. 10:73-80 (2000); Kolesnick, R. N. and Krönke, M., Annu. Rev. Physiol. 60:643-665 (1998); Levade, T. and Jaffrezou, J. P., Biochim. Biophys. Acta 1438:1-17 (1999)).

Two Mg²⁺ dependent neutral SMases, SMPD2 and SMPD3 were recently identified and cloned based on their sequence homology to bacterial nSMases (Tomiuk, S. et al., Proc. Natl. Acad. Sci. USA 95:3638-3643 (1998); Hofmann, K. et al., Proc. Natl. Acad. Sci. USA 97:5895-5900 (2000), WO 99/07855, EP-A-1204757). As the cDNAs encoding said nSMases were available, the biochemical and functional characterization of said nSMases was possible.

The ubiquitously expressed nSMase1 (SMPD2) is Mg²⁺ dependent, localizes to the endoplasmatic reticulum (ER), and is reversibly inhibited by oxidized glutathione and reactive oxygen species. Recently, it has been shown that nSMase1 has phospholipase C activity towards specific lysophospholipids (Sawai, H. et al., J. Biol. Chem. 274:38131-38139 (1999)). Using an antisense strategy, Tonetti et al. showed that nSMase1 may be involved in ceramide-mediated apoptosis triggered by T cell receptor ligation (Tonnetti, L. et al., J. Exp. Med. 189:1581-1589 (1999)). However, gene-targeted mice deficient for nSMase1 did not show any overt functional phenotype and no accumulation or changed metabolism of SM or other lipids (Zumbansen, M. and Stoffel, W., Mol. Cell Biol. 22:3633-3638 (2002)). Thus, although some insights into the properties of nSMase1 have been provided, its functional significance is still unknown.

NSMase2 (SMPD3) is expressed predominantly in brain and colocalizes with a Golgi marker in neuronal cells (Hofmann, K. et al., Proc. Natl. Acad. Sci. USA 97:5895-5900 (2000)). NSMase2 has a neutral pH optimum, depends on Mg²⁺ ions, and is activated by unsaturated fatty acids and phosphatidylserine. Biochemical characterization of nSMase2 overexpressed in yeast cells lacking inositol phosphosphingolipid phospholipase (Isc1p) revealed that *smpd3* is a structural gene for nSMase2 which acts as a *bona fide* nSMase in cells (Sawai, H. et al., J. Biol. Chem. 275:39793-39798 (2000); Marchesini, N. et al., J. Biol. Chem. 278:13775-13783 (2003)). Stable overexpression of nSMase2 in MCF7 cells resulted in a markedly decreased growth rate at the late exponential phase suggesting that nSMase2 is involved in the regulation of cell growth (Marchesini, N. et al., J. Biol. Chem. 278:13775-13783 (2003)). In mice, deletions in the gene encoding nSMase2 result in developmental and postnatal abnormalities. Stoffel, A. et al., Proc. Natl. Acad. Sci. USA 101:9079-9084 (2004), demonstrated that gene-targeted mice deficient for nSMase2 develop a novel form of dwarfism and delayed puberty as part of a hypothalamus-induced combined pituitary hormone deficiency suggesting that nSMase2 plays a pivotal role in the control of late embryonic and postnatal development. Immature architecture and delayed ossification of long bones was also observed. Strikingly, positional cloning of the recessive mutation *fragilitas ossium* (fro) in mice identified a deletion in the gene encoding nSMase2 (*Smpd3*) that led to complete loss of enzymatic activity (Aubin, I. et al., Nat. Genet. 37:803-805 (2005)). The mouse mutation *fragilitas ossium* leads to a syndrome of severe osteogenesis and dentinogenesis imperfecta with no collagen defect. At birth, homozygous fro/fro mice are smaller than normal with deformities and multiple fractures of ribs and long bones (Aubin, I. et al., Nat. Genet. 37:803-805 (2005)). Thus, *Smpd3* null mice partially recapitulate the pathology observed in homozygous fro/fro mice suffering osteogenesis and dentinogenesis imperfecta. Notably, the precise relationship between the impairment of nSMase2, its cascade of intracellular effects and the production of defective extracellular mineralized tissue is not yet fully understood.

In conclusion, the precise function of individual neutral SMases in SM turnover and in the regulation of ceramide-mediated cellular responses is not well understood up to now. Thus, there was a need for further investigations on mammalian nSMases in order to elucidate their physiological functions and to find new levers for diagnosis and therapy of nSMase-related diseases.

### Summary of the Invention

The present invention provides a novel human sphingomyelinase, nSMase3, which was furthermore cloned and characterized. Said nSMase3 is responsive to extracellular stimuli like TNF. Biochemical and functional analysis revealed that nSMase3 shares biochemical properties with nSMase1 and nSMase2, but is functionally distinct with regard to its subcellular localization to the ER.

The invention furthermore provides several non-human nSMase3 proteins from eukaryotes.

The features distinguishing the human nSMase3 of present invention from other known SMases are as follows:
(a) the amino acid sequence of human nSMase3 (SEQ ID NO:3) shows no homology with other SMase sequences besides nSMase3 sequences from other organisms;
(b) human nSMase3 is highly expressed in heart, skeletal muscle and testis;
(c) overexpression of nSMase3 in cardiomyocytes leads to increased contractility (positive inotropic effect).

In man, the gene coding for nSMase3 is located on chromosome 2, locus q21. This locus is connected with familial dilated cardiomyopathy 1H, a genetic variant of an autosomal-dominant inheritable disease. Thus, nSMase3 may play a causal role in the pathogenesis of said disease. Furthermore, diagnosis of said disease may be possible by detecting mutations in the nSMase3 gene.

nSMase3-deficient cells, especially cardiomyocytes, and corresponding animal models such as nSMase3-deficient (knock-out) mice provide a unique *in vitro* and *in vivo* model for cardiovascular diseases, especially for dilated cardiomyopathy 1H. These models can be used for testing candidate agents for therapy of said diseases.

Moreover, nSMase3 in man and animal presents itself as target for medicaments and for somatic gene therapy.

In view of the severe and mostly fatal course of dilated cardiomyopathy 1H, the lack of therapy for said disease and its unclear etiology, the nSMase3 is a key enzyme for development of novel diagnostic and therapeutic methods.

On the other hand, the high expression levels of nSMase3 in testis identify nSMase3 and its mutants as diagnostic targets for fertility disorders in men. In addition, nSMase3 is a novel candidate target for contraception in men.

Furthermore, the present invention provides a cDNA encoding said nSMase3, *smpd4.* In particular, the present disclosure provides
(1) An isolated neutral sphingomyelinase (nSMase3) having the amino acid sequence represented by one of SEQ ID NOs:3 to 9, or a functional variant, derivative or fragment thereof which possesses the enzymatic and/or immunologic properties of said nSMase3, wherein said functional variant is a fusion product comprising a nSMase3 domain and a second peptide domain or a functional non-proteinaceous component, said derivative is derived from the SEQ ID NOs:3 to 9 by addition of 1 to 2 amino acid residues, by deletion of 1 to 100 amino acid residues and/or by conservative substitution, wherein the ratio of substituted amino acid residues in comparison to the native sequence is from 0 to 30% of the total amino acids in the sequence, said fragment contains at least 7 amino acid residues of SEQ ID NOs:3 to 9, but is not consisting of aa 132 to 866 of SEQ ID NO3, and said nSMase3 is not consisting of SEQ ID NOs:6 to 9.
(2) an isolated nucleic acid sequence encoding an nSMase3 as defined in (1) above, or a nucleic acid complementary thereto;
(3) a vector comprising the nucleic acid sequence as defined in (2) above;
(4) a cell being transfected or transformed with the vector as defined in (3) above, and/or heterologously comprising the nucleic acid as defined in (2) above, and/or being capable of heterologously expressing an nSMase3 as defined in (1) above;
(5) a non-human tissue, a tissue culture, or a non-human transgenic organism comprising cells being transfected or transformed with the vector as defined in (3) above and/or heterologously comprising the nucleic acid as defined in (2) above and/or heterologously expressing the nSMase3 as defined in (1) above;
(6) the use of the cell as defined in (4) above, the non-human tissue, the tissue culture, or the non-human transgenic organism as defined in (5) above as a test cell system, test tissue or test model organism in testing candidate active agents for their therapeutic potential in diseases connected with nSMase3, preferably in heart muscle diseases, male reduced fertility or infertility, or for their potential as contraceptive in men or male animals;
(7) a method for testing candidate active agents for their therapeutic potential in diseases connected with nSMase3, preferably in heart muscle diseases, male reduced fertility or infertility, or for their potential as contraceptive in men or male animals, comprising the steps
   (a) administering the candidate agent to a culture of the cell as defined in (4) or a non-human tissue or a tissue culture as defined in (5); and
   (b) determining the effect of the candidate agent on said cell or tissue.
(8) a method for preparing the nSMASE3 as defined in (1) above, which method comprises culturing the cells as defined in (4) above and isolating the nSMASE3 from the culture;
(9) an antibody specific for the nSMase3 having the amino acid sequence represented by one of SEQ ID NOs:3 to 9;
(10) a shRNA or siRNA against nSMase3 as defined in (1) above;
(11) a vector encoding the shRNA or siRNA of (11) above;
(12) a pharmaceutical or diagnostic composition which comprises one or more of the following: the isolated nSMase3 of (1) above, the nucleic acid of (2) above, the vector of (3) and (11) above, the transformed or transfected cell of (4) above, the antibody of (9) above, and the shRNA or siRNA of (10) above, or pharmaceutically or diagnostically acceptable salts thereof;
(13) the isolated nSMase3 of (1) above, the nucleic acid of (2) above, the vector of (3) above, or pharmaceutically acceptable salts thereof for use in prevention or treatment of heart muscle diseases, male reduced fertility or infertility;
(14) the antibody of (9) above, the shRNA and siRNA of (10) above, the vector of (4) above, or pharmaceutically acceptable salts thereof for use in (a) prevention or treatment of diseases connected with increased activity of nSMase3, preferably cramps, tremor, Parkinson's disease and hypertrophic cardiomyopathy, or (b) contraception in men or male animals;
(15) an *ex-vivo* method for diagnosing and/or monitoring a disorder characterized by changes in the expression of an nSMase3 as defined in (1) above, which method comprises the
   (a)
      detection of the amount and/or activity of said nSMase3 or of the nucleic acid sequence encoding said nSMase3, and/or of mutations present in said nucleic acid sequence in comparison to the native nSMase3 encoding sequence in a biological sample from a subject having or suspected to have said disorder;
(16) a kit for performing the diagnostic method of (15) above, comprising one or more of the following: the nSMase3 of (1) above, the nucleic acid of (2) above, the vector of (3) above, the cell of (4) above, the antibody of (9) above and diagnostically acceptable salts thereof;
(17) an isolated cell, tissue or tissue culture, or a non-human transgenic organism, having a reduced abundance of nSMase3 according to SEQ ID NOs. 3-9 or being deficient in nSMase3 according to SEQ ID NOs. 3-9 or in the nSMase3 gene according to SEQ ID NOs. 1-2 or comprising a siRNA or shRNA of (10) or a vector of (11) above;
(18) the use of the cell, tissue or tissue culture, or the non-human organism of (17) above
   (i) as a model for a disease connected with a deficiency of nSMase3; or
   (ii) for the generation of antibodies to nSMase3, preferably for the generation of monoclonal antibodies including the antibodies of (10) above, and/or of antibodies capable of modulating nSMase3 activity; and
(19) the siRNA or shRNA of (10) or a vector of (11) above or the antibody of (9) above or Pharmaceutically acceptable salts thereof for use in the treatment of diseases connected with increased heart or skeletal muscle contractility and for reducing the fertility in male patients or male animals.

### Brief Description of the Figures

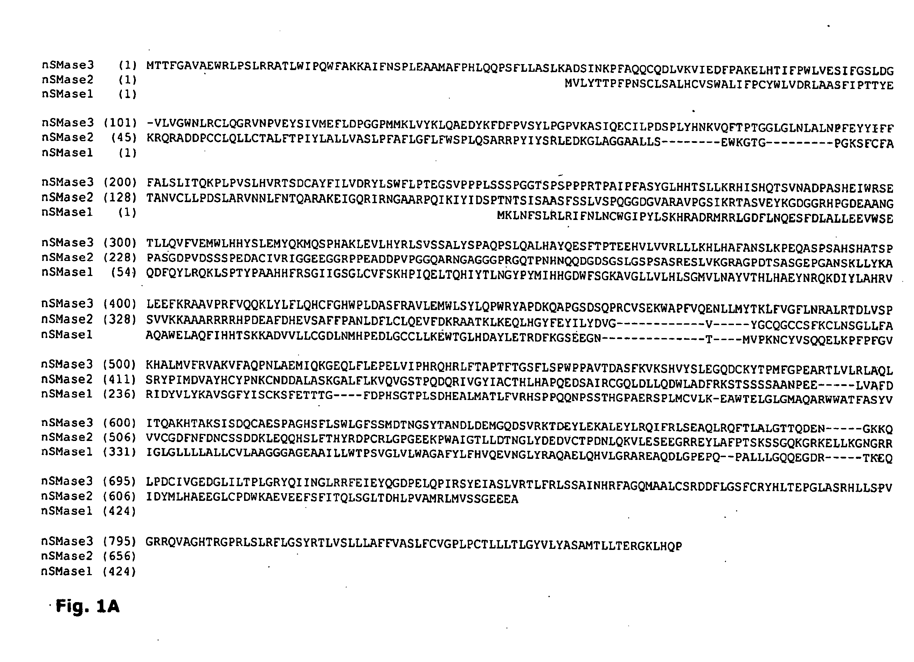

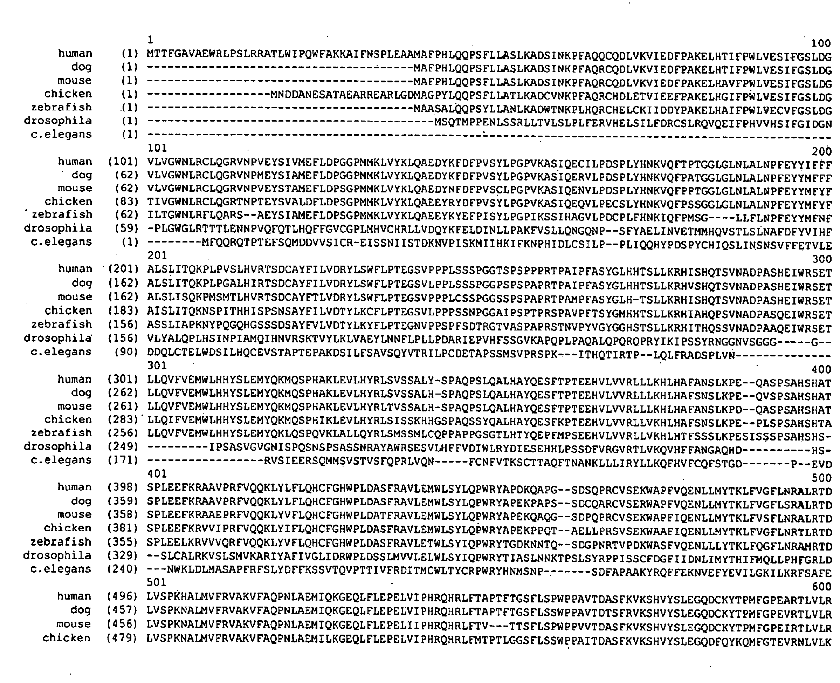

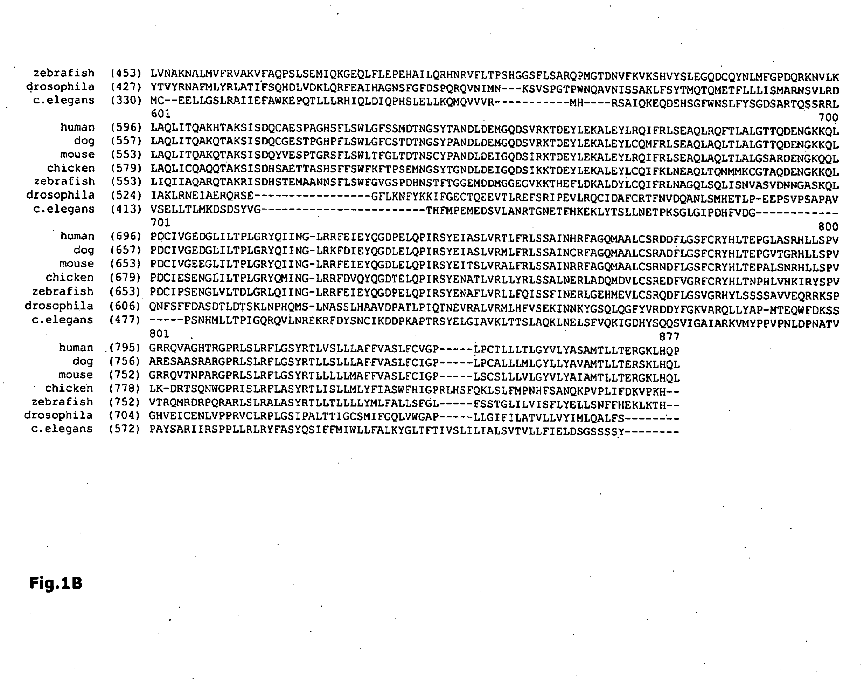

Fig. 1: Comparison of the sequence of nSMase3 with functional analogs and homologous proteins.
   A) Deduced amino acid sequence of human nSMase3 aligned with nSMase1 and nSMase2 (Genbank access numbers NP_003071 and NP_061137, respectively). Positions of amino acids are numbered on the left. Grey shaded boxes indicate identical or similar amino acids, highly conserved amino acids are shaded in black. The solid frame indicates the putative transmembrane domain, the dashed frame indicates the match with bovine peptide sequences.
   B) Proteins with significant amino acid sequence homology identified by BLAST search of the Genbank database. When more then one sequence from the same species were identified, sequences with highest degree of similarity were preferred. The sequence of human nSMase3 (SEQ ID NO:3) was aligned with deduced amino acid sequences of homologous proteins from dog (XP_543560; SEQ ID NO:4), mouse (BAB31737; SEQ ID NO:5), chicken (XP_415; SEQ ID NO:6), zebrafish (XP_706729; SEQ ID NO:7), drosophila (NP_650124; SEQ ID NO:8) and *C. elegans* (NP_492324; SEQ ID NO:9) using ClustalW algorithm.

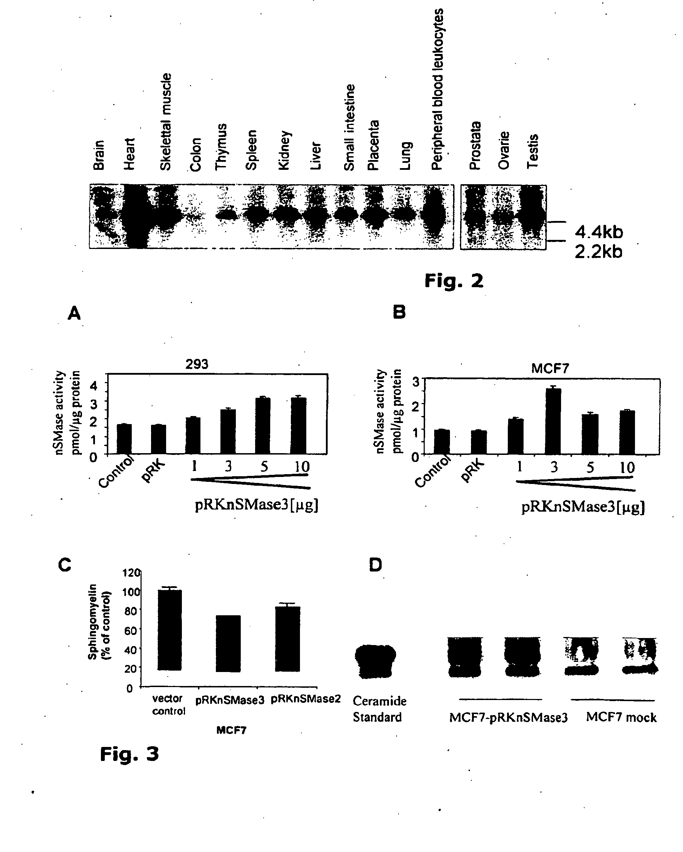

Fig. 2: Expression of nSMase3 mRNA in human tissues. Poly(A)+ mRNA from different human tissues (Clontech human 12 lane MTN (A) and human MTN II blot (B)) were analyzed by Northern blotting using as a hybridization probe a random-primed ³²P-labelled full-length nSMase3 cDNA.
Fig. 3: Overexpression of nSMase3 in 293 cells and MCF7 cells as described in example 5. A,B) 293 and MCF7 cells were transiently transfected with increasing doses of an expression plasmid, pRK-nSMase3 (SEQ ID NO:10) encoding full-length human nSMase3. 36 h after transfection, membrane fractions were assayed for nSMase activity. C) SM hydrolysis in nSMase3-transfected cells. MCF7 cells were transiently transfected with pRK-nSMase3 and pRK-nSMase2 expression vectors (SEQ ID NOs:10 and 11). Membrane fractions were analyzed for SM levels. D) Ceramide generation by nSMase3-transfected cells. Organic fractions of MCF7 cells left untreated or transfected with pRK-nSMase3 were prepared and ceramide generation was determined by DAG-kinase followed by TLC analysis.

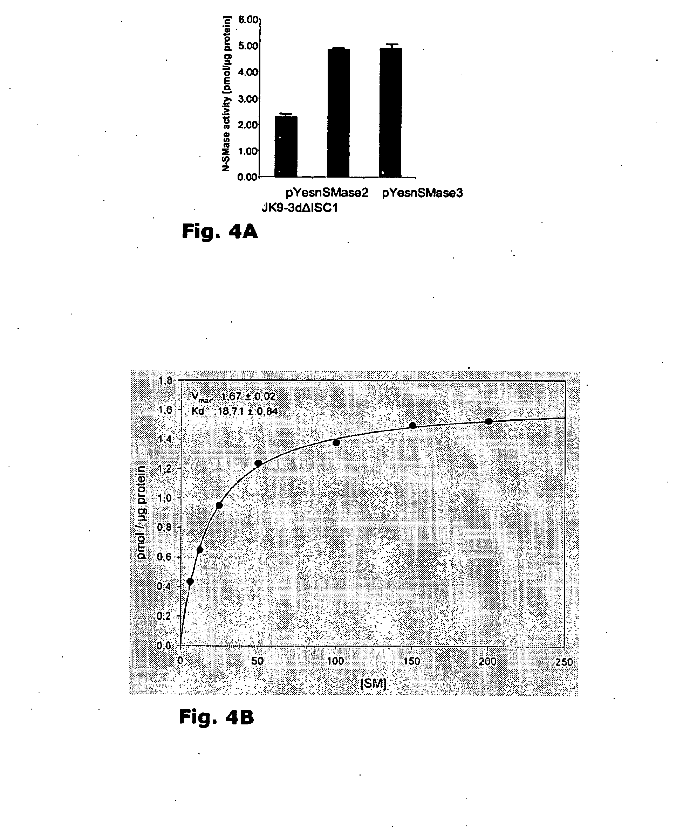

Fig. 4: Overexpression of nSMase3 in *S*. *cerevisiae*. NSMase3 as well as nSMase2 (control), respectively, were subcloned in the YES2 expression vector, and the resulting pYES-nSMase-vector (pYES-nSMase3, SEQ ID NO:12; pYES-nSMase2, SEQ ID NO:13) was transiently transfected into JK9-3dΔIsc1 yeast cells. A) SM hydrolysis was measured in microsomal fractions as described in example 6A and in the presence of 6.7 mol % Phosphatidylserine (PS) (100 µM). B) SM hydrolysis was measured for microsomal fractions of nSMase3 transfected yeast cells at increasing concentrations of SM and in the presence of 6.7 mol % PS (100 µM). Shown are results from one experiment which is representative for two separate experiments.

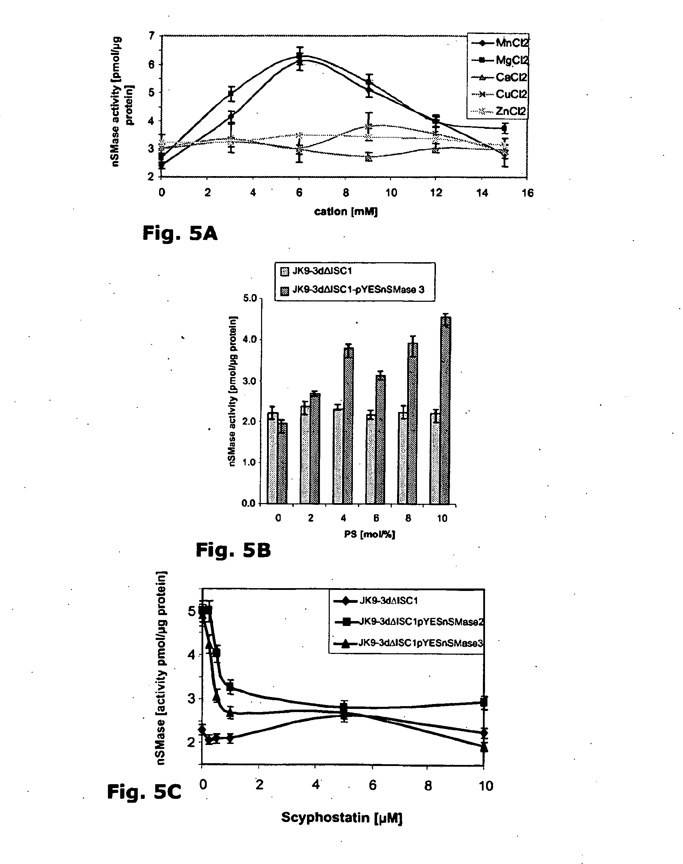

Fig. 5: Characterization of nSMase3 in yeast. nSMase activity was measured in microsomal fractions of JK9-3dΔIsc1p cells transiently transfected with pYES-nSMase3 (SEQ ID NO:12) in the presence of graded concentrations of the indicated cations (A) or PS (B). C) Microsomal fractions from nSMase3 transfected yeast cells were preincubated for 30 min at 37 °C with indicated concentrations of scyphostatin and assayed for nSMase activity. The values shown represent the average of duplicates.
Similar results were obtained in two separate experiments.

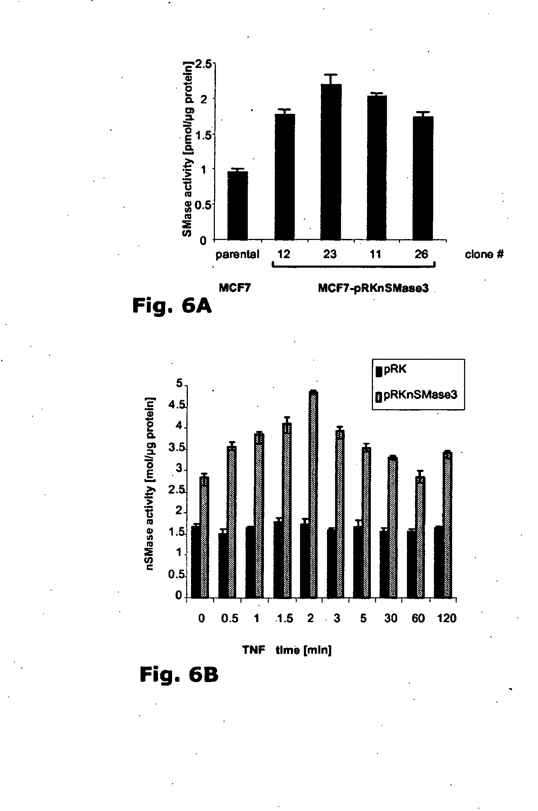

Fig. 6: TNF responsiveness of nSMase3. A) To obtain stable nSMase3 overexpressors, MCF7 cells were transfected with pRK-nSMase3 (SEQ ID NO:10) and with BMGneo coding for neomycin resistance (Karasuyama, H. and Melchers, F., Eur. J. Immunol. 18:97-104 (1988)). NSMase3 overexpressors were selected by culture in the presence of 800 µg/ml G418 and cloning by limiting dilution. B) Parental MCF7 cells and MCF7 cells stably transfected with nSMase3 (clone23), were stimulated in duplicates with TNF-α (100 ng/ml). At the indicated periods of time membrane fractions were analyzed for nSMase activity. The results are representative for three independent experiments.

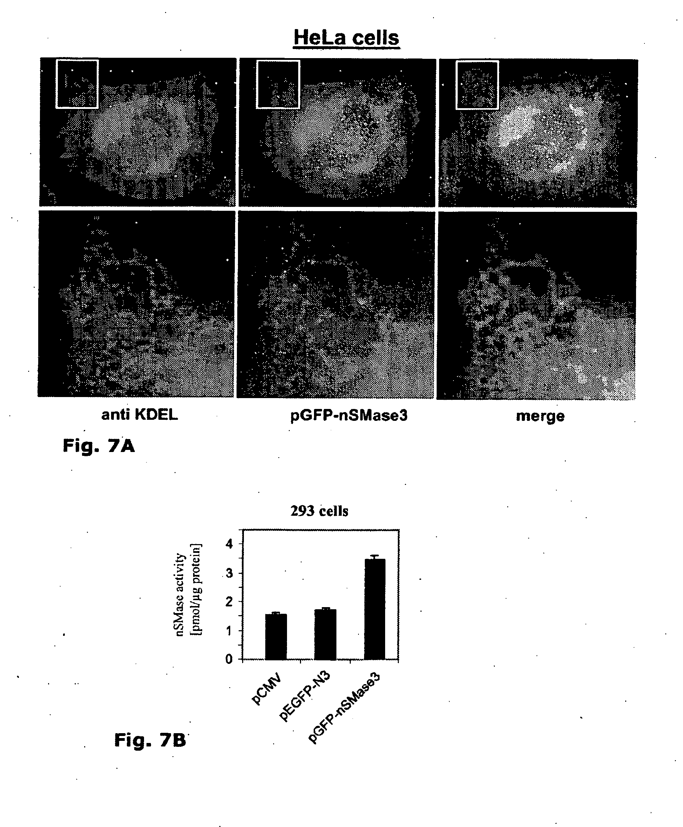

Fig. 7: Subcellular localization of nSMase3. A) HeLa cells were transiently transfected with an expression vector, pGFP-nSMase3 (SEQ ID NO:15), that encodes Green Fluorescent Protein (GFP) fused to the N-terminus of nSMase3. After 36 h, cells were stained with rat anti-KDEL antibody and Cy3-conjugated goat anti-rat Ig was used as secondary reagent. Cells were counterstained with Hoechst 33258. B) enzymatic activity of nSMase3-GFP fusion protein (GFP at the N-terminus of nSMase3) in 293 cells (see example 10).

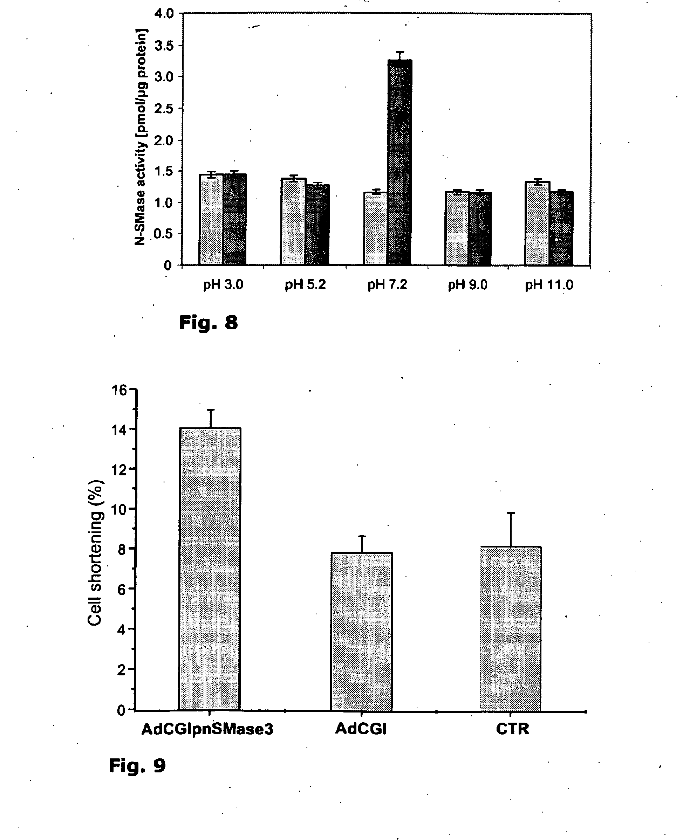

Fig 8: Enzymatic activity in nSMase3 overexpressing MCF7-nSMase3 clone 23 (black bars) or parental MCF7 (grey bars) cells at pH values ranged from 3.0 to 11.0 (example 13). Protein extracts prepared from MCF7-nSMase3 clone 23 (example 5) and parental MCF7 cells (grey bars) were submitted to a sphingomyelinase activity assay as described in example 6A.
Fig. 9: Effect of overexpression of nSMase3 on the contractility of cardiomyocytes.
Cardiomyocytes were transfected with nSMase3 cDNA using viral vectors as described in example 11. Contractility was measured as described in example 11. Systolic shortening of nSMase3-overexpressing (AdCGIpnSMase3), of mock transfected (AdCGI), and of nontransfected cardiomyocytes is shown.

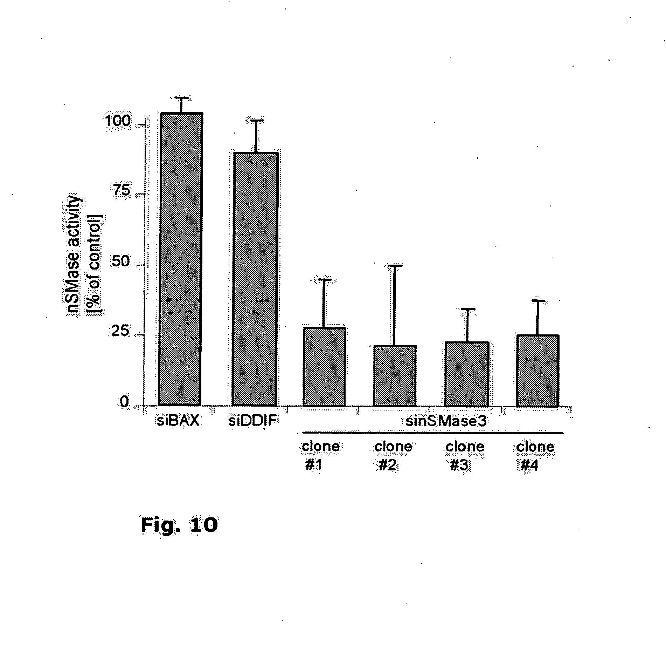

Fig. 10: Inhibition of nSMase3 activity by shRNA expression. Neutral sphingomyelinase activity was measured in HeLa cells, which were transfected with shRNA against BAX, DDIF or nSMase3 as described in example 12. Empty vector transfected cells served as a control. Four different clones of shRNA-nSMase3 transfected HeLa cells (clone Nos. 1-4) were investigated. Mean and standard deviation of the activity from 3 independent experiments are shown.

### Sequence Listing - Free Text

| SEQ ID NO: | sequence description |
|---|---|
| 1 | nSMase3 cDNA ; human |
| 2 | nSMase3 coding sequence; human |
| 3 | nSMase3 protein; human |
| 4 | nSMase3 protein; dog |
| 5 | nSMase3 protein; mouse |
| 6 | nSMase3 protein; chicken |
| 7 | nSMases3 protein; zebrafish |
| 8 | nSMase3 protein; drosophila |
| 9 | nSMase3 protein; C. elegans |
| 10 | pRK-nSMase3 |
| 11 | pRK-nSMase2 |
| 12 | pYES-nSMase3 |
| 13 | pYES-nSMase2 |
| 14 | AdCGI |
| 15. | pGFP-nSMase3 |
| 16 | bovine oligopeptide nSMase3_bovine_0 |
| 17 | human oligopeptide nSMase3_human_0 |
| 18 | human peptide nSMase3_human_1 |
| 19 | human peptide nSMase3_human_2 |
| 20 | human peptide nSMase3_human_3 |
| 21 | human peptide nSMase3_human_4 |
| 22 | human peptide nSMase3_human_5 |
| 23 | human peptide nSMase3_human_6 |
| 24 | human peptide nSMase3 human 7 |
| 25 | forward primer nSMase3 |
| 26 | reverse primer nSMase3 |
| 27 | forward primer nSMase2 |
| 28 | reverse primer nSMase2 |
| 29 | AdCGIpnSMase3 |
| 30 | sense shRNA against nSMase3 |
| 31 | antisense shRNA against nSMase3 |
| 32 | pENTRY-shnSMase3 |
| 33 | pLenti-shnSMase3 |

### Definitions

In the framework of the present invention the following abbreviations, terms and definitions are used.

The abbreviations used are: ER, endoplasmatic reticulum; GFP, green fluorescent protein; PC, phosphatidylcholine; PE, phosphatidylethanolamine; PS, phosphatidylserine; SM, sphingomyelin; SMase, sphingomyelinase; nSMase, neutral sphingomyelinase; SMPD, sphingomyelin phosphodiesterase; TNF, tumor necrosis factor.

In the context of present invention, the term "nSMase3" includes the native nSMase3, but also homologues, functional variants, derivatives and fragments thereof, provided that said homologues, functional variants, derivatives and fragments possess the enzymatic and/or immunologic properties of the native nSMase3. In a preferred embodiment of the invention, "nSMase3" refers to a native nSMase3 and its amino acid sequence, more preferably to the nSMases of the group consisting of SEQ ID NOs:3 to 9, most preferably to the nSMase3 of SEQ ID NO:3.

A "homologue" in the context of present invention designates a protein or peptide which possesses the enzymatic and/or immunologic properties of the native nSMase3, but differs from said native nSMase3 in its amino acid sequence in that one or more amino acids are substituted in comparison to the native nSMase3 sequence. Preferably, the said amino acid substitutions are conservative, i.e. the function and/or conformation of the protein is not affected by the substitutions. More preferably, one or more of the amino acids of the native protein are substituted by amino acids with similar chemical properties, e.g. Val by Ala (conservative amino acid exchange). The ratio of substituted amino acids in comparison to the native sequence is from 0 to 30% of the total amino acids in the sequence, preferably from 0 to 15%, most preferably from 0 to 5%.

A "derivative" of the nSMase3 of the invention encompasses proteins derived from the native nSMase3 amino acid sequence by substitution, addition and/or deletion of one or more amino acids. Said substitutions are conservative as defined above. Said addition products have amino acid sequences resulting from addition of 1-100, preferably 1-40, more preferably of 1-20 amino acids to the native nSMase3 sequence. The 1 to 2 added amino acids may be added as single amino acids or in continuous segments containing 2 linked amino acids. Said addition may take place at the N-and/or C-terminus and/or inside of the native nSMase3 sequence. More than one addition per sequence are allowed, but a single addition is preferred. Moreover preferred is an addition at the N- or C-terminus, and of these an addition at the N-terminus. The deletion products of the full length native nSMase3 amino acid sequences result from deletion of 1-100, preferably 1-50, more preferably 1-20 amino acids. The deleted amino acids may be deleted as single amino acids or in continuous segments containing 2 or more linked amino acids. Said deletion may take place at the N- and/or C-terminus and/or inside of the native nSMase3 sequence. More than one deletion per sequence are allowed, but a single deletion is preferred. Moreover preferred is a deletion at the N- or C-terminus, and of these a deletion at the N-terminus.

An nSMase3 "fragment" is derived from the native nSMase3 amino acid sequence by deletion of one or more amino acids at the N- and/or C-terminus. Said fragment must still possesses the enzymatic and/or immunologic properties of the native nSMase3. Thus, the minimum length of said fragment is determined by its ability to be recognized and bound by an antibody which is specific for the native protein. Preferably, said fragment has up to 100, more preferably up to 50, most preferably up to 20 amino acids and contains at least 7 amino acids, more preferably at least 15 amino acids.

A "functional variant" of a protein or peptide of the invention is a fusion protein of said protein or peptide ("nSMase3 domain") with at least one second functional protein domain or functional non-proteinaceous component ("second domain"). Said non-proteinaceous second domain may be selected from non-native chemical components (such as polyethers including PEG, polyesters, alkylation products, etc.), polysaccharides and lipids. Said second protein domain is preferably a marker protein, an immunological adjuvant, a protein conferring antibiotic resistance to a transformed cell, or a ligand for a cell surface receptor expressed by the target cell.

A "nucleic acid sequence encoding a nSMase3" may be any nucleic acid, preferably DNA or RNA, coding for the proteins or peptides of present invention, i.e. for anyone of the group consisting of the nSMase3 of SEQ ID NOs:3 to 9, their functional variants, derivatives, homologues and fragments. Said nucleic acid sequence is either identical or substantially identical to the native sequence of nSMase3 or to the nucleic acid sequence corresponding to the amino acid sequence of the functional variants, homologues, derivatives and fragments of present invention. In the context of present invention, a specific nucleic acid sequence refers to said sequence itself and nucleic acid sequences which are substantially identical thereto. A "substantially identical" nucleic acid sequence differs from the initial nucleic acid only by degenerated codon exchange. Thus, in a substantially identical nucleic acid sequence the codons inside the coding sequences are changed in a way which will not lead to a change of the amino acid sequence of the translation product (i.e. replacement of a codon by another codon out of its codon family). The nucleic acid sequences coding for native nSMase3, more preferably the sequence of SEQ ID NOs:1 and 2, and for nSMase3 derivatives and fragments are preferred in the context of present invention. More preferred are nucleic acid sequences encoding native nSMase3s, especially the nSMase3s of SEQ ID NO:3 to 9.

Preferably, said nucleic acid sequence comprises the cDNA sequence of SEQ ID NO:1, i.e. the cDNA of native human nSMase3, or is derived therefrom by degenerated codon usage. Alternatively preferred is a nucleic acid sequence comprising SEQ ID NO:2 or its exon parts, i.e. the native coding sequence for human nSMase3.

The nucleic acid sequences of the invention are preferably used as full length sequences. However, they may also be used as addition or deletion mutants of said full length sequences for the performance of present invention. The allowable additions and deletions in the nucleic acids of the invention correspond in their range and nature to the allowable amino acid additions and deletions as outlined above. In addition to the deletion or addition of complete codons the addition and deletion of single or pairwise nucleotides is possible.

A "nucleic acid sequence fragment" is a part of the full length nucleic acid sequence which is shorter that full length, but which is still able to hybridize under stringent conditions to the full length nucleic acid sequence. It contains preferably at least 15 nucleotides, more preferably at least 25 nucleotides. " "Native" is used synonymously to "naturally occurring".

The term "isolated" means that a molecule selected from the group comprising proteins, peptides and nucleic acids is/has been separated or purified from other molecules of said group, wherein said other molecules are usually associated with said isolated molecule in its organism of origin. This encompasses biochemically purified molecules, recombinantly produced and chemically synthesized molecules of said group. An "isolated cell" is a cell which is separate from its organism of origin. An "isolated tissue" is a tissue which is separate from its organism of origin, preferably a primary tissue.

The term "animal" encloses all animals with the exception of humans. Preferred animals in the context of present invention are vertebrates.

The term "vertebrate" according to the present invention relates to an animal having a backbone or spinal column including mammals, birds, reptiles, amphibians and fish. Preferred vertebrates are mammals including humans, birds and fish. Inasfar as transgenic animals are concerned, rodents, especially mice or rats, are preferred for performance of present invention.

"Tissue culture" according to the present invention refers to animal tissue, organ primordia, or the whole or part of an organ which is maintained or grown *in vitro* so as to preserve its architecture and/or function. Preferred tissue cultures in present invention are from vertebrate tissue. Furthermore, said tissue culture is preferably a primary tissue culture.

A "cell culture" according to the present invention includes isolated cells which are cultured *in vitro.* These cells can be immortalized by fusion with tumor cells (tumor derived cells) or obtained directly from their native source (primary cell culture). They may be transformed or untransformed.

The term "pharmaceutically acceptable salt(s)", as used herein, means those salts of compounds of present invention which are safe and effective for topical or systemic use in mammals and that possess the desired biological activity. The counter ion is suitable for the intended use, non-toxic, and it does not interfere with the desired biological action of the pharmaceutical composition. Pharmaceutically acceptable salts in the context of present invention include the salts reviewed in the IUPAC Handbook of Pharmaceutically Acceptable Salts (Wermuth, C.G. and Stahl, P.H., Pharmaceutical Salts: Properties, Selection and Use - A Handbook, Verlag Helvetica Chimica Acta (2002)). Said salts include also the corresponding solvates.

Accordingly, the term "diagnostically acceptable salt(s)" refers to those salts of compounds of present invention which are useful and effective for the desired diagnostic method. Their counter ion does not interfere with the reaction necessary for detection of the target compound. Said salts include also the corresponding solvates.

"Pharmaceutically or diagnostically acceptable derivatives" of the compounds of present invention are derivatives formed from the compounds by chemical modification. Said derivatives do not interfere with the pharmaceutical or diagnostic activity of said compounds. In case of proteins and peptides, they include functional variants of proteins as defined above. In case of nucleic acid molecules, they include nucleic acid molecules derivatized with marker molecules (like biotin or radioactive isotopes), connected to further nucleic acids (like, e.g., His-tags) etc..

### Detailed Description of the Invention

The present invention relates to nSMase3 and its use.

Generally, the nSMase3 of embodiment (1) of present invention shows only little sequence homology to nSMase1 and nSMase2 (Fig. 1A). In one preferred aspect of embodiment (1) said nSMase3, its homologue, functional variant, derivative or fragment thereof contains an amino acid sequence as represented by SEQ ID NO:16 or 17 (nSMase3_bovine_0, nSMase3_human_0), preferably contains an amino acid sequence as represented by SEQ ID NO:23 (nSMase3_human_6; contains amino acid sequence of SEQ ID NO:17); more preferably contains one or more of the amino acid sequences as represented by SEQ ID NOs:18 to 24 (nSMase3_human_1 to nSMase3_human_7). In another preferred aspect of embodiment (1), said nSMase3, its homologue, functional variant, derivative or fragment thereof lacks an N-terminal leader signal sequence.

In another preferred aspect, the nSMase3 according to embodiment (1) is a vertebrate nSMase3, preferably a mammalian, avian or fish nSMase3, more preferably an nSMase3 as represented by one of SEQ ID Nos:3 to 7. Even more preferably, the nSMase3 is mammalian and/or selected from the group comprising SEQ ID Nos:3 to 5. Most preferably, the nSMase3 according to embodiment (1) is human nSMase3, a homologue, functional variant, derivative or fragment thereof. Especially preferred is the human nSMase3 represented by SEQ ID NO:3.

In a further preferred aspect of embodiment (1) said nSMase3, its homologue, functional variant, derivative or fragment thereof preferably
- contains a transmembrane domain close to the C terminus, and/or
- is Mg²⁺ dependent, and/or
- is activated by TNF, and/or
- has a pH optimum at neutral pH, and/or
- colocalizes with ER markers.

The pH optimum preferably lies at a value from pH 6.8 to 7.6, more preferably at pH 7.2.

The isolated nucleic acid of embodiment (2) of the invention is a nucleic acid sequence encoding an nSMase3 as defined above in the "definitions" section. Thus, it has the preferred aspects as listed in this section. It is preferably a DNA or RNA. More preferably, it encodes a native nSMase3. Most preferably, it comprises the sequence of SEQ ID NO:1 or 2, its RNA analogon or a sequence complementary thereto.

The vector of embodiment (3) may be any state of the art vector which is suitable for transformation or transfection of procaryotic or eucaryotic cells. It is preferably an expression vector, more preferably a vector which allows regulated, i.e. inducible expression. Most preferred are the vectors used in the examples section.

The transfected or transformed cell of embodiment (4) is preferably an isolated cell. In a preferred aspect of embodiment (4), said transfected or transformed cell is selected from the group consisting of (a) mammalian cells, preferably human or murine cells, more preferably is a cardiomyocyte, myocyte, neural cell, macrophage, granulocyte, spermatogonia, Sertoli cell, Leydig cell or a tumor-transformed cell line thereof, (b) microbial cells, preferably bacterial or yeast cells, (c) insect cells and (d) plant cells. Most preferably, said cell is a primary cell cultured from tumor transformed (i.e. immortalized) cells, more preferably is derived from HeLa, HEK293, MCF7, cardiomyocytes, myocytes, neuronal cells, macrophages, granulocytes, spermatogonia, Sertoli cell or Leydig cell.

In a further preferred aspect of embodiment (4), said cell is overexpressing nSMase3.

The non-human organism according to embodiment (6) of the invention is preferably a vertebrate, an invertebrate or a plant. Said organism is more preferably a vertebrate, especially a non-human mammal, more preferably is a rodent, most preferably is a mouse or rat.

The tissue or tissue culture of embodiment (5) of the invention is preferably derived from the non-human organism as defined above. Tissue or tissue culture of human origin is also contemplated by the present invention. Furthermore, the tissue or tissue culture is preferably primary tissue or tissue culture, or it is a tissue culture of tumor transformed cells. Said tumor transformed cells are preferably derived from HeLa, HEK293, MCF7, cardiomyocytes, myocytes, neuronal cells, macrophages, granulocytes, spermatogonia, Sertoli cells or Leydig cells. In a further preferred aspect of embodiment (5), the cells contained in the tissue or tissue culture are overexpressing nSMase3.

The preferred diseases connected with nSMase3 in the use of embodiment (7) and the method of embodiment (8) are heart muscle diseases, and male reduced fertility or infertility. This is due to the fact that nSMase3 in humans is mainly expressed in heart and testis. Furthermore, the results of the nSMase3 overexpression in cardiomyocytes indicate a positive inotropic effect of nSMase3. The use (7) and method (8) are moreover suitable for the search for contraceptives in men or male animals, as testis is one of the few expression sites of nSMase3 in humans and animals.

In the method of embodiment (8), the candidate active agent is administered to the cell, tissue or non-human organism in a suitable preparation or formulation. The dosages which may be administered in the test to a non-human organism are dependent on the specific animal, its size, age and health condition. Thus, they have to be determined individually from case to case. Furthermore, the determination of a dose-effect ratio by testing different dosages is advisable.

The method of embodiment (8) is apt for identification of either suppressors or enhancers of nSMase3, and even of substituents for nSMase3. This is due to the fact that it may be performed not only with the cells/tissues/animals of embodiments (4) to (6), but also with cells/tissue/animal lacking a part or all of their native nSMase3, especially the cells/tissue/animal of embodiment (18). In the latter case, said cells/tissue/animal serve as a model for a disease connected with an nSMase3 deficiency.

The effect determination of embodiment (8)(b) is preferably done or supplemented by detection of the amount and/or activity of nSMase3 or of the nucleic acid sequence encoding nSMase3 as described for embodiment (16)(b) below.

The method (9) is preferably performed with yeast or mammalian cells, more preferably with *S*. *cerevisiae* cells or immortalized mammalian cells, most preferably with immortalized human cells including HEK293, MCF7, HeLa and immortalized cardiomyocytes, myocytes, neuronal cells, macrophages, granulocytes, spermatogonia, Sertoli cells or Leydig cells. The isolation of the nSMase3 produced by said cells may be performed by any textbook method for protein isolation and purification, but is preferably done by chromatography (including ion-exchange chromatography, hydrophobic interaction chromatography, affinity chromatography).

The antibody of embodiment (10) is preferably an antibody raised against the nSMase3 of embodiment (1). It is preferably raised in non-human animals lacking nSMase3 and/or using nSMase3 epitopes which are highly divergent from species to species. Said epitopes are more preferably selected from fragments of the nSMase3 of embodiment (1), most preferably selected from fragments of the native nSMase3 sequence. In turn, said fragments of the native nSMase3 are preferably fragments of SEQ ID NO:3 to 9. In one preferred aspect of said turn, the antibody of embodiment (10) is raised against one or more of the fragments represented by SEQ ID Nos:17 to 24, which are highly conserved regions in nSMase3s from different species. Said antibody is therefore specific for a set of several nSMase3s. In another preferred aspect of said turn, the antibody is raised against a species-specific nSMase3 fragment, especially a fragment which is not represented by SEQ ID Nos:17 to 24, and is therefore strictly species specific.

The pharmaceutical or diagnostic composition of embodiment (13) may further comprise at least a pharmaceutically or diagnostically acceptable carrier.

There are quite a few diseases, disorders and health conditions which may be connected to changes, i.e. increases or decreases, in the expression and/or activity of nSMase3. These are heart muscle diseases, prominently DCM, and male reduced fertility or infertility which may be connected with lack of nSMase3 (decrease); and other heart muscle diseases, prominently hypertrophic cardiomyopathy, cramps (including tetanus), and tremor (including Parkinson's disease) which may be connected with overexpression and/or increased activity of nSMase3 (increase). Furthermore, the use of nSMase3 inhibitors as contraceptive in men and male animals is contemplated in present invention.

In the method for diagnosing and/or monitoring a disorder characterized by changes in the expression of an nSMase3 according to embodiment (16), the detection step (b) is preferably performed using immunological or molecular biology methods. If immunological methods are used, one antibody of choice is the antibody of embodiment (10). Furthermore, the activity of nSMase3 can be detected by any method feasible to detect nSMase activity, preferably by a method implying the measurement of enzymatic SM hydrolysis.

The cell, tissue or non-human organism of embodiment (18) is lacking a part or the total of its native nSMase3 content. This is preferably due to treatment of the cell, tissue or organism with an agent suppressing or reducing nSMase3 expression or interfering with the nSMase3 coding gene, including transformation or transfection with heterologous DNA, RNAi, gene targeting including conditional and inducible gene knock out, chemical and radiation mutagenesis. In case of non-human animals or tissue therefrom, the nSMase3 deficiency is preferably generated by constitutive or inducible RNAi or by transgene or conditional gene knockout.

Said cell, tissue or non-human organism may be used as a model for diseases connected with a nSMase3 deficiency (embodiment 19 (i)), preferably for dilated cardiomyopathy and infertility in men or male animals. It may further be used for production of nSMase3 antibodies, preferably of monoclonal antibodies. Said antibodies may be used for modulation of nSMase3 activity *in vitro* or *in vivo*.

The medicament of embodiment (20) is preferably used for the treatment of hypertrophic cardiomyopathy, tetanus, muscle cramps and tremor including Parkinson's disease. The treatment of Parkinson's disease is especially preferred. The agent reducing the abundance of nSMase3 in an organism in the use (20) is selected from RNAi agents against nSMase3 including the shRNA and siRNA of embodiment (11) and the vector of embodiment (12), antibodies including the antibody of claim (10) and chemicals inhibiting nSMase3 expression and/or activity.

The effective dose in the methods of embodiments (21) and (22) is dependent on several parameters, *inter alia* the sex of the patient, his size, age and health condition. Thus, it has to be determined individually from case to case by the responsible physician.

The method of embodiment (23) uses RNAi, a tool for inhibition of gene expression (Fire, A. et al., Nature 391, 806-811 (1998)). It is based on the introduction of double stranded RNA (dsRNA) molecules into cells, whereby one strand is complementary to the coding region of a target gene. Through pairing of the specific mRNA with the introduced RNA molecule, the mRNA is degraded by a cellular mechanism. In mammalian cells, short (<30 bp) interfering RNAs (siRNA) are used in RNAi, as they circumvent the interferon response to long dsRNA (Elbashir, S.M. et al., Nature 411, 494-498 (2001)). Injection of siRNA into the tail vein of mice leads to gene inhibition which is restricted to specific organs and persists only a few days (McCaffrey, A.P. et al., Nature 418, 38-39 (2002); Lewis, D.H. et al., Nature Genet. 32, 107-108 (2002)). A further improvement of the siRNA technology is based on the intracellular transcription of the two complementary siRNA strands using separate expression units both under the control of the U6 promoter (Lee, N.S. et al., J. Nat. Biotechnol. 20(5):500-5 (2002); Du, Q. et al., Biochem. Biophys. Res. Commun. 325(1):243-9 (2004); Miyagishi, M. and Taira, K., Methods Mol. Biol.;252:483-91 (2004)) or expression of short hairpin RNA (shRNA) molecules by a single transcription unit under the control of the U6 or H1 promoter (Brummelkamp, T.R. et al., Science 296, 550-553 (2002); Paddison, P.J. et al, Genes Dev. 16, 948-958 (2002); Yu, J.Y. et al., Proc. Natl. Acad. Sci. USA 99, 6047-6052 (2002); Sui, G. et al., Proc. Natl. Acad. Sci. USA 99, 5515-5520 (2002); Paul, C.P. et al., Nature Biotechnol. 20, 505-508 (2002); Xia, H. et al., Nat. Biotechnol. 10, 1006-10 (2002); Jacque, J.M. et al., Nature 418(6896):435-8 (2002)).

Thus, shRNA and siRNA against nSMase3 and vectors encoding said RNAs are aspects of present invention (embodiments (11) and (12)).

In present invention, the RNAi target gene in embodiment (23) is the native gene coding for nSMase3. Preferred siRNAs are RNAs of a minimum length of 15 nt and a maximum length of 30 nt. Preferred shRNAs contain a sense and an antisense RNA strand which are connected by a loop; sense and antisense strand each have a minimum length of 15 nt and a maximum length of 30 nt, the loop has a minimum length of 0 nt, preferably of 5 nt, and a maximum length of 15 nt. The most preferred shRNA in the context of present invention is the shRNA having SEQ ID NO:30 or 31. Said siRNAs and shRNAs are complementary to coding regions of the nSMase3 gene, more preferably RNAs which are complementary to regions of the mRNA corresponding to any one of SEQ ID Nos:3 to 9. Said siRNAs and shRNAs may contain a few non-homologous bases, preferably at their ends, or - in case of shRNA - as a loop connecting the sense and antisense strand. However, more preferably the siRNAs do not contain any non-homologous bases. Most siRNA expression vectors are based on polymerase III dependent (Pol III) promoters (U6 or H1) that allow the production of transcripts carrying only a few non-homologous bases at their 3' ends. It has been shown that the presence of non-homologous RNA at the ends of the shRNA stretches lower the efficiency of RNAi mediated gene silencing (Xia, H. et al., Nat. Biotechnol. 10, 1006-10 (2002)).

A preferred aspect of the method (23) implies the use of shRNA as RNAi agent, more preferably the shRNA as exemplified in example 12, including the vectors and methods used therein.

In the cells, tissue, tissue culture or animals which are the result of the method of embodiment (23), the nSMase3 activity is downregulated or even completely suppressed (compare example 12). Therefore, said cells etc. are ideal model systems for the method of embodiment (8) if they are used as described in embodiment (19(I)).

In the following, the properties of the nSMase3 of present invention are exemplified on the human nSMase3 of SEQ ID NO:3. These properties, however, also apply to other nSMase3s of present invention, especially to vertebrate and even more to mammalian nSMase3s.

The human nSMase3 of present invention was biochemically characterized by overexpression in a yeast strain, JK9-3dΔIsc1p, lacking intrinsic SMase activity. Like nSMase2, nSMase3 is Mg²⁺ dependent, shows enhanced activity in the presence of phosphatidylserine, and is inhibited by scyphostatin. NSMase3 is ubiquitously expressed as a 4.6 kb mRNA species. Tumor necrosis factor (TNF) stimulates rapid activation of nSMase3 in MCF7 cells with peak activity at 1.5 min. nSMase3 lacks an N-terminal signal peptide yet contains a 23 amino acid transmembrane domain close to the C-terminus, which is indicative for the family of C-tail-anchored integral membrane proteins. Cellular localization studies of nSMase3, expressed as green fluorescent protein fusion protein, demonstrated colocalization with markers of the ER. These results suggest that sphingomyelin metabolism regulated by nSMase3 is at the receiving end of TNF signaling.

Recently, a Mg²⁺ ion dependent, 97 kDa nSMase from bovine brain was purified to homogeneity and characterized (Bernardo, K. et al., J. Biol. Chem. 275:7641-7647 (2000)). Amino acid sequencing of tryptic peptides showed no apparent homology to nSMase1 or nSMase2, nor to any known bacterial SMase. By databank searches a human protein with sequence homologies to one of the bovine nSMase peptides was identified. This human homolog was designated as nSMase3 (SMPD4). It functions as a nSMase in cells. Its gene is a structural gene for neutral sphingomyelinase and is located on chromosome 21 at locus 2q21. Furthermore, nSMase3 is activated by TNF and colocalizes with ER markers.

The nSMase3 according to embodiment (1) of the invention is evolutionary highly conserved allowing identification of homologous proteins down to the level of Bilateria

(Fig. 1B). This is in contrast to the known nSMase2 that appears to be conserved down only to the level of mammals (Hofmann, K. et al., Proc. Natl. Acad. Sci. USA 97:5895-5900 (2000); NCBI Homologene). The gene locus 2q21 is not associated with known mutations in mice so far. The phenotypic data of deletion mutants of *C. elegans* produced by large-scale RNA interference techniques are contradictory and range from lethality to absence of any obvious phenotype (Fraser, A. G. et al., Nature 408:325-330 (2000); Piano, F. et al., Curr. Biol. 12:1959-1964 (2002); Maeda, I. et al., Curr. Biol. 11:171-176 (2001)). At least two cardiovascular diseases are associated with the chromosomal locus 2q21 - dilated cardiomyopathy (CMD 1H) and susceptibility to premature coronary heart disease (Jung, M. et al., Am. J. Hum. Genet. 65:1068-1077 (1999); Pajukanta, P. et al., Am. J. Hum. Genet. 67:1481-1493 (2000)). The high expression level of the nSMase3 gene (*Smpd4*) in heart muscle suggests a role of this gene in the pathogenesis of cardiac diseases.

According to the NCBI SNP database (http://www.ncbi.nlm.nih.gov/entrez/ query.fcgi?db=Snp), the genomic region of nSMase3 shows a high degree of variability: 25 single nucleotide polymorphisms are described, 10 of them may affect mRNA sequence including four nonsynonymous mutations.

Although no significant sequence homology to nSMase1 or nSMase2 was discerned, nSMase3 displayed biochemical properties similar to nSMase2. When analyzed in yeast cells, nSMase3 like nSMase2 showed dependency for Mg²⁺ and Mn²⁺ ions. In contrast to nSMase2, Mg²⁺ or Mn²⁺ concentrations above 6 mM markedly suppressed nSMase3. As for nSMase2, Ca²⁺, copper or zinc did not support nSMase3 activity. Furthermore, when directly compared in yeast cells, nSMase3 and nSMase2 were similarly stimulated by PS, and both nSMase2 and nSMase3 were equally sensitive to inhibition by scyphostatin.

Increased nSMase activity in mammalian cells can be brought about by cDNAs encoding either a nSMase or a co-factor required for nSMase function. To establish that a structural gene encodes nSMase3, nSMase3 was expressed in inositol phosphosphingolipid phospholipase C (Isclp)-deficient JK9-3dΔIsc1p yeast cells (Example 3, Fig. 4b). The deletion of Isc1p removes residual endogenous SMase activity and previously allowed Hannun and coworkers to unequivocally define *smpd3* (nSMase2) as a gene coding for a *bona fide* neutral SMase (Marchesini, N. et al., J. Biol. Chem. 278:13775-13783 (2003)). Similarly, overexpression of nSMase3 in JK9-3d cells established robust nSMase activity in the microsomal fraction, providing pertinent evidence that nSMase3 is a structural enzyme with neutral SMase activity (Example 3, Fig. 4b).

The subcellular topology of nSMase3 expression merits some mention of its possible role in sphingolipid metabolism. The lack of an N-terminal leader signal sequence and its transmembrane domain close to the C-terminus define nSMase3 as a C-tail-anchored integral membrane protein. Notably, nSMase1 also lacks an N-terminal signal sequence, yet its two transmembrane regions are followed by 50 amino acid residues (Tomiuk, S. et al., Proc. Natl. Acad. Sci. USA 95:3638-3643 (1998)). Considering that only 40 amino acids of the nascent chain are sequestered within the eukaryotic large ribosomal subunit (Blobel, G. and Sabatini, D. D., J. Cell Biol. 45:130-145 (1970)), the transmembrane domains of nSMase1 may interact with the signal recognition particle (SRP) as long as they are part of the nascent polypeptide chain. Thus, nSMase1 is probably delivered to the ER by the SRP-dependent pathway. In contrast, like other C-tail-anchored proteins, nSMase3 possesses a hydrophobic membrane insertion sequence at the C-terminus such that it only can emerge from the ribosome after translation is terminated. Although the mechanisms determining the selection of the target membrane have not yet been identified, the principal sites of tail-anchored proteins are the ER and the mitochondrial outer membrane (Borgese, N. et al., J. Cell Biol. 161:1013-1019 (2003); Kutay, U. et al., Embo J. 14:217-223 (1995)). Indeed, nSMase3 showed an ER distribution pattern when N-terminally fused with GFP (example 10, Fig. 7a). It is worth emphasizing that nSMase2 contains two hydrophobic domains in its N-terminal portion (Hofmann, K. et al., Proc. Natl. Acad. Sci. USA 97:5895-5900 (2000)). Thus, nSMase3 distinguishes itself from nSMase1 and nSMase2 by its C-tail anchoring and membrane targeting.

Since SM is synthesized at the Golgi apparatus, the question arises whether the ER is the final destination of nSMase3. The ER localization of nSMase3 has been demonstrated with a GFP fusion protein. Although the retention in the ER is mostly transmembrane and luminal domain-dependent as has been demonstrated with cytochrome b5 (Honsho, M. et al., J. Biol. Chem. 273:20860-20866 (1998)), the N-terminal fusion to a 40 kDa protein such as GFP might alter the physiologic localization of nSMase3. More information about the precise localization of nSMase3 can be expected from stainings of endogenous nSMase3 by specific antibodies, e.g. the antibodies of embodiment (10) of the invention. Thus, nSMase3 may also be localized downstream the secretory pathway, that is, in the Golgi or in the plasma membrane.

A particular feature of the ER membrane is its capacity to support rapid, bidirectional transport of phospholipids across the bilayer (reviewed in Pomorski, T. et al., Semin Cell Dev. Biol. 12:139-148 (2001)). The location and topology of sphingolipid synthesis is rather complex with different reactions occurring in either the ER or Golgi apparatus and with distinct topologies. It is important to note that the active sites of some of these enzymes face the cytoplasm, others face the lumen, which implies transbilayer movement of sphingolipids during the biosynthetic process. The initial steps of sphingolipid synthesis take place at the cytoplasmic leaflet of the ER but later steps such as the conversion of ceramide to sphingomyelin take place for the most part at the luminal leaflet of the Golgi apparatus (for review Futerman, A. H. and Riezman, H., Trends Cell Biol. 15:312-318 (2005)). Thus, ceramide needs to be translocated to the lumen for SM synthesis. While transbilayer movements of glycosylceramides and other sphingolipids require the action of flippases, ceramide may spontaneously flip-flop in lipid bilayers. Thus, one possible physiologic action of nSMase3 would be to hydrolyse SM at the cytoplasmic leaflet of the ER membrane to allow for transbilayer movement of ceramide and subsequent conversion to SM in the lumen of the Golgi apparatus. In this scenario, nSMase3 would be instrumental for the generation and maintenance of the asymmetrical distribution of SM in eukaryotic membranes.

The present invention is described in more detail by reference to the following examples. It should be understood that these examples are for illustrative purpose only and are not to be construed as limiting the invention.

### Examples

In the following examples, standard techniques of recombinant DNA technology were used that were described in various publications, e.g. Sambrook et al. (1989), Molecular Cloning: A Laboratory Manual, Cold Spring Harbor Laboratory, or Ausubel et al. (1987), Current Protocols in Molecular Biology 1987-1988, Wiley Interscience. Unless otherwise indicated, all enzymes, reagents and kits were used according to the manufacturers' instructions.

### Example 1: Cell culture, media, cells and plasmids

MCF7 cells were cultured at 37 °C in RPMI 1640 (Biochrom, Berlin, Germany) supplemented with 10% fetal bovine serum, 2 mM L-glutamine, 100 µg/ml streptomycin and 100 U/ml penicillin (Biochrom) in a humidified 5% CO₂ incubator. HEK293 and HeLa cells were cultured at 37 °C in DMEM (Biochrom, Berlin, Germany) supplemented with 10% fetal bovine serum, 2 mM L-glutamine, 100 µg/ml streptomycin and 100 U/ml penicillin (Biochrom) in a humidified 5% CO₂ incubator. All cells were obtained from the American Type Culture Collection.

Yeast Strain and Culture Media: The *Saccharomyces cerevisiae* strain JK9-3dΔIsc1p deficient for the Isc1p gene (Sawai, H. et al., J. Biol. Chem. 275:39793-39798 (2000)) was kindly provided by Yusuf Hannun (Charleston, SC). Yeast extracts and peptone were from Difco (BD, Heidelberg, Germany). Synthetic minimal medium (SD), SD/Gal, and Ura dropout supplement were purchased from Clontech (BD, Heidelberg, Germany). Scyphostatin was a kind gift of Dr. T. Ogita and F. Nara, Sankyo Co. LTD, Tokyo, Japan.

Plasmids containing full-length coding sequences of human nSMase3 as identified by homology search (compare Example 2) (clone DKFZp434A1711Q) and nSMase2 (clone IRALp962J187Q) were isolated from clones obtained from "RZPD Deutsches Ressourcenzentrum für Genomforschung" (https://www.rzpd.de) and reamplified by PCR for subcloning.

### Example 2: Sequence identification and analysis in silico

Homology search using the bovine peptide KGLPYLEQLFR (SEQ ID NO:16) against protein sequences derived from a draft of the human genome project was performed using standalone blastp program (Altschul, S. F. et al., Nucleic Acids Res. 25:3389-3402 (1997)). Protein sequence was analyzed for conserved patterns or motifs by search against Prosite (http://ca.expasy.org/prosite/) or Pfam database (http://www.sanger.ac.uk/Software/ Pfam/search.shtml), respectively. Prediction of transmembrane region was performed using the TMHMM Algorithm (http://www.cbs.dtu.dk/services/TMHMM/). The ClustalW algorithm was employed for protein sequence alignment.

### Example 3: Expression of nSMase3 in yeast

The pYES2 yeast expression vector containing a galactose-inducible promoter was purchased from Invitrogen (Karlsruhe, Germany). cDNA of the human nSMase3 was generated by PCR, using a plasmid isolated from the clone DKFZp434A1711Q as a template and Pfu as proofreading polymerase (Promega, Mannheim, Germany). The following PCR primers were used to introduce EcoRI- and SalI-endonuclease restriction sites to facilitate subcloning:
forward 5'- GGAATTCTGATGACGACTTTCGGCG-3' (SEQ ID NO:25),
reverse 5'- GTCGACGTCAGGGCTGGTGCAGCTT-3' (SEQ ID NO:26).

YES2 and the PCR product were digested by EcoRI and SalI for 2 h at 37 °C. pYes2 was additionally dephosporylated by addition of 1 U calf intestine alkaline phosphatase (Fermentas, St. Leon-Rot, Germany) for 30 min at 37 °C. Fragments were resolved by agarose gel electrophoresis, visualized by ethidium bromide staining and excised from the gel. DNA was recovered from the gel blocks by QiaQuick Gel Extraction Kit (Qiagen, Hilden, Germany). Fragments were ligated by T4 DNA ligase (Fermentas) according to manufacturer's instructions. 2 µl of ligation reaction were transformed into competent *E. coli* XL1-blue cells.

Human nSMase2 was cloned in a similar way using a plasmid isolated from IRALp962J187Q as template, XhoI and BamHI restriction sites and the following primers:
forward 5'- GGGATCCATGGTTTTGTACACGACC-3' (SEQ ID NO:27),
reverse 5'-CCTCGAGCTATGCCTCCTCCTCCC-3' (SEQ ID NO:28).

Said cloned human nSMase2 served as a control.

The sequences of the resulting plasmids (SEQ ID NO:13 for nSMase2 and SEQ ID NO:12 for nSMase3) were confirmed by sequencing. Plasmids were transfected into yeast JK9-3dΔIsc1p cells as described by Marchesini et al. (Sawai, H. et al., J. Biol. Chem. 275:39793-39798 (2000)), and the expression of nSMase3 was induced by incubating the cells in synthetic complete Ura medium containing 2% galactose overnight.

### Example 4: Preparation of Yeast Cell Lysates

Yeast cells were suspended in buffer containing 25 mM Tris (pH 7.4), 5 mM EDTA, 1 mM phenylmethylsulfonyl fluoride, and 4 µg/ml each chymostatin, leupeptin, antipain, and pepstatin A. Cells were disrupted with glass beads as described (Sawai, H. et al., J. Biol. Chem. 275:39793-39798 (2000)). Glass beads and cell debris were removed by centrifugation at 2,000 x g for 10 min, and the supernatant was centrifuged at 100,000 × g to obtain the microsomal and cytosolic fractions. For the studies on cation and lipid effects, and on substrate requirements of nSMase3, membranes were delipidated by incubation in lysis buffer in the presence of 1% Triton^{®}X-100 and incubated at 4 °C for 1 h. The suspension was centrifuged at 100,000 × g for 90 min, and the supernatant was used for enzymatic determinations. Protein concentration was determined using a Bio-Rad protein assay reagent.

### Example 5: Overexpression of nSMase3 in mammalian cells

The enzymatic characterization of nSMase3 was assessed by transient expression in HEK293 and MCF7 (Fig. 3). cDNA constructs (see below) were transfected into MCF7 or HEK293 cells using the Ca²⁺-phosphate precipitation method (Sambrook, J. and Russel, D. W., Molecular cloning: a laboratory manual, 3rd Ed., Cold Spring Harbor Laboratory Press, NY (2001)). Briefly, 1.5*10⁶ HEK293 or 1*10⁶ MCF7 cells were seeded 24 h prior to transfection on 10 cm culture plates (Falcon Primaria, BD). 5-10 µg of plasmid DNA were used for a single transfection. 0.5 ml of DNA solution in 250 mM CaCl₂ was added dropwise to the equal amount of 2xHeBS (0.28 M NaCl, 0.05 M HEPES, 1.5 mM Na₂HPO₄) and vigorously vortexed. The transfection mixture was carefully distributed over the cell plate. For selection of stable transfectants, 800 µg/ml G418 (Invitrogen) was added to the medium. Independent colonies were picked and cultured in separate wells, and three clones from single cells expressing the highest nSMase activity *in vitro* were used as nSMase3 overexpressors, e.g. in example 9 (Fig.6A, B).

293 or MCF7 cells were transiently transfected with increasing doses of an expression plasmid, pRK-nSMase3 (SEQ ID NO:10) encoding full-length human nSMase3. 36 h after transfection, membrane fractions were assayed for nSMase activity. Enzymatic assays showed a dose-dependent increase of nSMase activity (Fig. 3A,B). Transfection of nSMase3 into MCF7 cells resulted in a decrease in SM mass, which was comparable to that obtained with nSMase2 transfectants (transfected with pRK-nSMase2, SEQ ID NO:11) used as control (Fig. 3C). It should be noted that combined DAG kinase/TLC analysis revealed ceramide generation by nSMase3 transfected MCF7 cells, which was hardly detectable with untransfected MCF7 cells (Fig. 3D), indicating that parental MCF7 cells express low level, if any, endogenous nSMase activity.

### Example 6: Assays and Detection Methods

A) Assay for nSMase: The micellar SMase assay using exogenous radiolabeled SM was described by Wiegmann *et al.* (Wiegmann, K. et al., Cell 78:1005-1015 (1994)). To measure neutral SMase, cultured cells were harvested by standard trypsin-EDTA treatment and pelleted by centrifugation (300 x g, 15 min). The pellets were dissolved in a buffer containing 20 mM Hepes (pH 7.4), 10 mM MgCl₂, 2 mM EDTA, 5 mM DTT, 0.1 mM Na₃VO₄, 0.1 mM Na₂MoO₄, 30 mM p-nitrophenylphosphate, 10 mM B-glycerophosphate, 750 µM ATP, 1 mM PMSF, 10 µM leupeptin, 10 µM pepstatin, 0.5% Chaps. After 15 min at 4 °C , cells were homogenized by repeated squeezing of the cells through an 18-gauge needle. Nuclei and cell debris were removed by a low speed centrifugation (800 x g). The protein concentration in supernatants containing the cytosolic and membrane fractions was measured using a protein assay (Pierce, Hamburg, Germany). 50 µg of protein were incubated for 2 h at 37 °C in a buffer containing 20 mM Hepes, 1 mM MgCl₂ (pH 7.4) and 2.25 µl of [N-methyl-¹⁴C]SM. Phosphorylcholine was then extracted with 800 µl of chloroform:methanol 2:1 (v/v) and 250 µl of H₂O. Radioactive phosphorylcholine produced from [¹⁴C]SM was identified by counting 200 µl of the aqueous phase by scintillation counting.
B) Sphingomyelin Measurement: Cells were seeded at 0.3 x 10⁶ cells/10-cm-dish in 8 ml of complete growth medium. The next day, the cells were labeled with [methyl-³H]choline chloride (1 µCi/ml final concentration in 10 ml of growth medium/plate) for 48 h. Cells were then chased with 10 ml complete medium. At the indicated time points, the medium from each plate was collected, and the cells were washed once with 2 ml of ice-cold PBS. Cells were scraped off on ice in 2 ml of PBS, and each plate was washed with an additional 2 ml of PBS. Cells and washes were pooled with the medium and centrifuged for 5 min at 2,000 x g (4 °C). Lipids were extracted by the method of Bligh and Dyer (Bligh, E. G. and Dyer, W. J., Can. J. Biochem. Physiol. 37:911-917 (1959)), and aliquots of 250 µl were used for phosphorus and SM determination as previously described (Wiegmann, K. et al., Cell 78:1005-1015 (1994)).
C) Ceramide Measurement: The generation of the neutral lipid cleavage product of SMases, ceramide, was detected by the DAG-kinase assay combined with TLC analysis. Briefly, MCF7 cells were washed two times in ice-cold PBS. Cultured cells were harvested by standard trypsin-EDTA treatment and pelleted by centrifugation (300 x g, 15 min). The pellets were resuspended in 1.825 ml H₂O and the supernatants were transferred into glass tubes. Subsequently, 6 ml CHCl₃/MeOH/iN HCl (100/100/1 v/v/v) were added. Tubes were sonicated for 5 min in a water bath sonicator and then centrifuged for 10 min at 6000 x g. The lower organic phase was dried down under nitrogen. The dry samples were resuspended in 2 ml 0.1 N KOH and incubated for 1 h at 37 °C (alkaline hydrolysis). 6 ml CHCl₃/MeOH/1N HCl (100/100/1 v/v/v), 1 ml CHCl₃ and 2.25 ml water were added. After vortexing the samples were centrifuged for 10 min at 6,000 x g. The lower organic phase was dried down under nitrogen and resuspended in 100 µl CHCl₃ followed by transfer into Eppendorf tubes. From this point on, the DAG-kinase assay kit protocol was followed (Amersham Biosciences). For thin-layer chromatography, plates were pre-run in a solvent system composed of methanol/chloroform (1:1). Plates were removed from the tank and air-dried. TLC-chambers were pre-equilibrated for 1 h at room temperature with the solvent system cloroform:methanol:acidic acid (65:15:5, v/v/v). Dried samples were resuspended in 20 µl of chloroform:methanol (95:5, v/v). Samples were streaked onto a 10x10 cm silica gel thin layer plate. Plates were placed in a paper-lined TLC developing tank and the solvent was allowed to migrate to the top of the plate. Plates were removed from the tank, air-dried and exposed to Kodak XAR-films at room temperature.
D) Immunofluorescence: Cells were grown on coverslips and transfected with 1 µg nSMase3 DNA constructs using ExGen 500 *in vitro* transfection reagent (Fermentas, St. Leon-Rot, Germany) and incubated for 24 h. Cells were washed twice with cold PBS, then fixed with 3% paraformaldehyde for 20 min, permeabilized with 0.1% saponin in PBS for 10 min, and blocked with 3% BSA, 0.005% sodium azide, 0.2% teleostean gelatin and 0.1% saponin in PBS for 30 min. For immunostaining, cells were incubated with rat anti-KDEL antibody (kindly provided by Dr. G. Butcher, Cambridge) for 1 h, washed with 0.1% saponin in PBS and then incubated for 30 min with Cy3-conjugated goat anti-rat IgG (Dianova, Hamburg, Germany) as secondary reagent. Nuclei were counterstained with Hoechst 33258 and examined using a confocal fluorescence microscope.

### Example 7: Identification of a human homolog to bovine nSMase

The purification to homogeneity and biochemical characterization of a Mg²⁺-dependent nSMase from bovine brain was reported previously (Bernardo, K. et al., J. Biol. Chem. 275:7641-7647 (2000)). Amino acid sequencing of tryptic peptides of bovine nSMase showed no homologies to any known nSMases. Allowing for mismatches due to possible species-specific primary sequences, extensive sequence database searches revealed a homology to the human hypothetical protein FLJ20297 (synonyms KIAA1418 and LOC55627, accession number BAA92656). The predicted mRNA contains an ORF that encodes a protein of 866 amino acids (aa), resulting in a predicted molecular mass of 97.8 kDa, which matched the molecular mass of the nSMase previously purified from bovine brain.

An alignment of the novel nSMase3 with nSMase1 and nSMase2 is shown in Fig. 1A. Apparently, nSMase3 lacks an N-terminal signal peptide. In addition, a C-terminal hydrophobic region (aa 828-850) representing a putative transmembrane domain, is followed by only 16 amino acids. The lack of an N-terminal leader sequence combined with a hydrophobic transmembrane domain close to the C-terminus defines the group of tail-anchored integral membrane proteins, whose entire functional N-terminal portion faces the cytosol (Borgese, N. et al., J. Cell Biol. 161:1013-1019 (2003)). Other than that no statistically significant patterns or motifs were recognized.

In particular, a possible ER membrane protein motif (KLHQ) is located near the C-terminus (aa 862-865) that, however, does not match with the minimal ER retention consensus sequences (KDEL, KKXX or KXKXX). PSORT II analysis, (http://psort.nibb.ac.jp) predicts that the subcellular location of this protein is most likely the endoplasmic reticulum (55.6%), followed by mitochondria (22.2%), plasma membrane (11.1%) and vesicles of the secretory system (11.1%) (Nakai, K. and Horton, P., Trends Biochem. Sci. 24:34-36 (1999)). Strikingly, the alignment with mammalian nSMase1 and nSMase2 showed no apparent homologies. The database search revealed proteins closely related to nSMase3 in mouse, dog, chicken, zebrafish, drosophila and *C. elegans* (Fig. 1B).

The gene for nSMase3 is encoded on chromosome 2, chromosomal locus 2q21.1. Although the predicted gene is comprised of about 31 alternative exons and may result in 12 different splice variants, we found that nSMase3 mRNA was ubiquitously expressed as single 4,600 nt mRNA species, with the exception of leukocytes where two signals were visible. The greatest abundance of nSMase3 mRNA was observed in striated muscle, heart muscle an testis (Fig. 2). It should be noted, that the current version of the Genebank database defines two shorter isoforms of FLJ20297 mRNA as a reference sequence. This reflects alternate splicing events that were hypothesized to occur on theoretical grounds and should result in mRNAs with 3,600 and 3,300 nucleotides, respectively. The 4.6 kb size of nSMase3 mRNA, however, suggests abundant expression of full length cDNA corresponding to the cDNA clone with accession number AB037839.

### Example 8: Characterization of nSMase3 enzymatic activity

Increased nSMase activity in mammalian cells can be brought about by cDNAs encoding either a nSMase or a cofactor required for nSMase function. To establish that nSMase3 is encoded by a structural gene for neutral sphingomyelinase, nSMase3 was expressed in JK9-3dΔIsc1 yeast cells. This strain of S. cerevisiae lacks the isc1 locus encoding an inositol phosphosphingolipid phospholipase C (Isc1p) that exerts SMase activity (Sawai, H. et al., J. Biol. Chem. 275:39793-39798 (2000)). The deletion of Isc1p removes any residual endogenous SMase activity and thus allows detection of the nSMase enzyme itself (Marchesini, N. et al., J. Biol. Chem. 278:13775-13783 (2003)). Like nSMase2, overexpression of nSMase3 in JK9-3dΔIsc1p cells established robust nSMase activity in the microsomal fraction prepared by the method of example 4 (Fig. 4A), indicating that nSMase3 is a structural nSMase. Kinetic analysis of nSMase3 in the presence of PS revealed for SM a Kₘ of 18.71 µM and a Vₘₐₓ of 1.67 pmol/µg/h (Fig. 4B). MgCl₂ and MnCl₂ at 5 mM stimulated nSMase3, while CaCl₂, CuCl₂ or ZnCl₂ did not change nSMase3 activity (Fig. 5A). PS stimulated nSMase3, while other phospholipids had only slight effects (Fig. 5B). As shown for the purified bovine nSMase (16), nSMase3 was inhibitable by scyphostatin in a dose-dependent manner (Fig. 5C).

### Example 9: TNF-induced activation of nSMase3

nSMase can be stimulated by ligand cell surface receptor interactions including the TNF/TNF receptor system. In order to test whether nSMase3 is responsive to TNF, MCF7 cells stably transfected with nSMase3 were employed and compared to parental MCF7 cells that express little if any nSMase activity (Fig. 3B,C). Stable expression of nSMase3 in MCF7 cells revealed cell clones with markedly increased nSMase activity (Fig. 6A). TNF stimulation according to Wiegmann, K. et al., Cell 78:1005-1015 (1994) of MCF7-clone23 overexpressors resulted in a 250% increase of nSMase activity within 1.5 min (Fig. 6B). In contrast, TNF did not stimulate nSMase activity in mock transfected MCF7 cells, indicating that the increments of nSMase activity observed in TNF-treated cells can be attributed to the transfected nSMase3.

### Example 10: Subcellular localization of nSMase3

In order to address the subcellular localization of nSMase3, HeLa cells were transiently transfected with DNA constructs expressing GFP-nSMase3 (N-terminally fused GFP). The fusion of GFP to the N-terminus was chosen in consideration of the primary structure of nSMase3 that predicted membrane anchoring through the C-terminal hydrophobic domain. The open reading frame of human nSMase3 (and nSMase2) was amplified by PCR using nSMase primers containing XhoI/EcoRI restriction sites as described in Example 3. PCR products were subjected to restriction digestion with corresponding restriction enzymes and the digested DNA fragments were ligated to digested pEGFP-N3 (nSMase3 fused to the N-terminus of EGFP, pGFP-nSMase3; SEQ ID NO:15) (Clontech, Germany) expression vectors.

As shown in Fig. 7A, immunofluorescence analysis of GFP-nSMase3 expression pattern revealed intensive perinuclear and diffused reticular structures significantly overlapping with ER visualized by an antibody specific for KDEL (carboxy-terminal sequence of luminal ER proteins). This confirms the predicted localization of nSMase3 as an ER membrane protein. Fusion of GFP to the N-terminus did not impair the nSMase enzymatic activity (Fig. 7B).

### Example11: Infection of cardiomyocytes with AdCGIpnSMase3 in comparison to AdCGI and non-infected controls

Single rat ventricular myocytes were obtained from adult Sprague-Dawley rats (200-250 g) by enzymatic dissociation as previously described (Hoppe et al., PNAS 98:5335-5340 (2001)). Immediately after isolation cells were resuspended in medium 199 (Bio Whittaker, Walkersville, Maryland) supplemented with 10% fetal bovine serum and 1x penicillin-streptomycin and plated for primary culture on laminin-coated (Sigma) coverslips. Infection of cardiomyocytes was performed 2 h after plating at a multiplicity of infection (MOI) of 15 to 100 p.f.u. per cell with bicistronic adenoviral vectors encoding EGFP and p97 under the control of a CMV promoter (AdCGIpnSMase3, SEQ ID NO:29). Control cells were infected with an adenovirus encoding the reporter gene EGFP alone (AdCGI, SEQ ID NO:14). Cells were maintained in infection media for 4 h at 37°C, after which it was replaced with culture medium.

Field stimulated cell contraction was measured 24 to 32 h after infection. After 24 to 32 h the cells were transferred into Tyrode's solution (NaCl 135 mM, KCI 4.0 mM, MgCl₂ 1.0 mM, NaH₂PO₄ 0.33 mM, HEPES 10.0 mM and Glucose 10.0 mM, pH adjusted to 7.3 with NaOH) with the addition of 2 mmol/l Ca²⁺. The cell suspension was placed into a stimulation chamber mounted on the stage of an inverted confocal laser scanning microscope (Leica Microsystems, Wetzlar, Germany). Field stimulation was performed with an electric field stimulator (Grass Medical Instruments, Massachusetts, USA), at a frequency of 1 Hz, with a voltage of 80 V. Cell contraction was recorded using the microscope in line scan mode at 800 Hz and stored as digital images. During the recording procedure, the cells were superfused with fresh Tyrode's solution containing 2 mM Ca²⁺.

Systolic cell shortening was significantly larger in AdCGIpnSMase3-infected cardiomyocytes (14.04±0.91%, n=40) versus AdCGI-infected controls (8.12±1.68%, p=0.00015, n=22) and non-infected controls (7.81±0.83%, p=0.017, n=33).

Diastolic cell length was not significantly different between the three groups: 89.91±2.71 µM (AdCGIpnSMase3-infected cells, n=40) versus 85.46±4.80 µM (AdCGI-infected controls, n=22) versus 91.52±4.02 µM (non-infected controls, n=33).

### Example 12: Downregulation of nSMase3 activity by expression of shRNA

A HeLa cell line deficient in nSMase3 was generated by a stable expression of nSMase3 shRNA employing a lentiviral transfection system (Invitrogen). Complementary oligonucleotides (SEQ ID NOs:30 and 31) comprising a 19 nt specific target sequence derived from the human nSMase3 mRNA, 9 nt loop and 19 nt of antisense target sequence as well as overhangs of BglII and HindIII restriction sites were synthesized by Operon (Cologne, Germany). The oligonucleotide dimers were ligated into the pEntry-sh Vector. pEntry-sh was constructed by ligation of XbaI/KpnI fragments of pSuper (OligoEngine), containing RNA-polymerase promoter H1 (TypIII-RNAPol), into XbaI/KpnI digested pENTR (Invitrogen). The resulting construct was designated pENTRY-shnSMase3 (SEQ ID NO:32). The shRNA expression cassette was subcloned into pLenti6-sh vector using LR recombinase mix II (Invitrogen) according to the manufacturer's instructions, resulting in pLenti-shnSMase3 (SEQ ID NO:33). pLenti6-sh was constructed from pLenti6/V5-DEST (Invitrogen) by partial digesting with KspI and KpnI restriction endonucleases, removal of 3' protruding termini by T4 DNA polymerase and religation of the vector.

Recombinant lentiviruses were generated according to the manufacturer's instructions: pLenti-shnSMase3 together with three packaging plasmids was transformed into 6x10⁶

HEK-293 FT cells using Lipofectamine2000 Reagent (Invitrogen). Culture supernatants were collected 24 and 48 h after infection and stored at -70 °C. Virus titer was determined according to the Invitrogen protocol using the HT1080 cell line. For generation of the shRNA expressing cell line, virus stock was applied at multiplicity of infection of 1 to the semiconfluent HeLa cells. Transfected cells were selected by presence of 10 µg/ml Blasticidin (PAA) for 10 days and subcloned. The integration of the shRNA expression cassette was verified by PCR. Efficacy of downregulation of nSMase3 mRNA was verified by Northern Blot and enzymatic assay for neutral sphingomyelinase as described in example 6A (Fig. 10). Stable expression of the nSMase3-shRNA resulted in a marked decrease of nSMase activity up to 90%. Expression of control shRNAs, like Bax-shRNA or DFIF-shRNA, which are directed to other proteins, did not affect the sphingomyelinase activity in HeLa cells.

### Example 13: pH optimum of nSMase3

In order to verify that nSMase3 is a neutral sphingomyelinase a determination of nSMase3 activity at different pH was performed. Protein extracts from MCF7-nSMase3 overexpressing cells (clone23) were prepared as described in example 6A, using Hepes buffer with different pH values ranged from 3.0 to 11.0. As shown in Fig. 8 only assay conditions at neutral pH (7.2) resulted in detectable sphingomyelinase activity. Higher or lower pH values resulted in the complete inhibition of the enzymatic activity of nSMase3. This data indicated that the nSMase3 enzyme is a true neutral sphingomyelinase.

## Claims

1. An isolated neutral sphingomyelinase (nSMase3) having the amino acid sequence represented by one of SEQ ID NOs:3 to 9, or a functional variant, derivative or fragment thereof which possesses the enzymatic and/or immunologic properties of said nSMase3, wherein said functional variant is a fusion product comprising a nSMase3 domain and a second peptide domain or a functional non-proteinaceous component, said derivative is derived from the SEQ ID NOs:3 to 9 by addition of 1 to 2 amino acid residues, by deletion of 1 to 100 amino acid residues and/or by conservative substitution, wherein the ratio of substituted amino acid residues in comparison to the native sequence is from 0 to 30% of the total amino acids in the sequence, said fragment contains at least 7 amino acid residues of SEQ ID NOs:3 to 9, but is not consisting of aa 132 to 866 of SEQ ID NO:3, and said nSMase3 is not consisting of SEQ ID NOs:6 to 9.

2. The isolated nSMase3 of claim 1, which
(i) contains an amino acid sequence as represented by SEQ ID NO: 16 or 17, preferably contains the amino acid sequence as represented by SEQ ID NO:23, more preferably contains one or more of the amino acid sequences as represented by SEQ ID NOs:18 to 24; and/or
(ii) lacks an N-terminal leader signal sequence; and/or
(iii) is a vertebrate nSMase3, preferably a mammalian nSMase3, more preferably a human nSMase3.

3. The isolated nSMase3 of claim 1 or 2, which is the human nSMase3 having SEQ ID NO:3, or which is a functional variant, derivative or fragment thereof as defined in claim 1.

4. The isolated nSMase3 of any one of claim 1 to 3, which
(i) contains a transmembrane domain close to the C terminus; and/or
(ii) is Mg²⁺ dependent; and/or
(iii) is activated by TNF; and/or
(iv) has a pH optimum at neutral pH; and/or
(v) colocalizes with ER markers.

5. An isolated nucleic acid sequence encoding the nSMase3 as defined in any one of claims 1 to 4, or a nucleic acid complementary thereto.

6. The isolated nucleic acid sequence of claim 5 which comprises the sequence of SEQ ID NO:1 or 2.

7. A vector comprising the nucleic acid sequence of claim 5 or 6.

8. A cell being transfected or transformed with the vector of claim 7 and/or heterologously comprising the nucleic acid of claim 5 or 6 and/or being capable of heterologously expressing an nSMase3 as defined in any one of claims 1 to 4.

9. A non-human tissue, a tissue culture or a non-human transgenic organism comprising cells being transfected or transformed with the vector of claim 7 and/or heterologously comprising a nucleic acid sequence as defined in claims 5 or 6 and/or heterologously expressing a nSMase3 as defined in any one of claims 1 to 4.

10. Use of the cell of claim 8, the non-human transgenic organism, the non-human tissue or the tissue culture of claim 9 as a test cell system, test tissue or test model organism in testing candidate active agents for their therapeutic potential in diseases connected with nSMase3, preferably in heart muscle diseases, male reduced fertility or infertility, or for their potential as contraceptive in men or male animals.

11. A method for testing candidate active agents for their therapeutic potential in diseases connected with nSMase3, preferably in heart muscle diseases, male reduced fertility or infertility, or for their potential as contraceptive in men or male animals, comprising the steps
(a) administering the candidate agent to a culture of the cell of claim 8, a non-human tissue or a tissue culture of claim 9; and
(b) determining the effect of the candidate agent on said cell or tissue.

12. A method for preparing the nSMase3 of any one of claims 1 to 4 which comprises culturing cells as defined in claim 8 and isolating the nSMase3 from the culture.

13. An antibody specific for the nSMase3 having the amino acid sequence represented by one of SEQ ID NOs:3 to 9.

14. A shRNA or siRNA against nSMase3 as defined in any one of claims 1 to 4.

15. The shRNA of claim 14, which comprises the sequence represented by SEQ ID NO:30 and/or 31.

16. A vector encoding the shRNA or siRNA of claim 14 or 15.

17. A pharmaceutical or diagnostic composition comprising one or more of the following: the isolated nSMase3 of anyone of claims 1 to 4, the nucleic acid of claim 5 or 6, the vector of claim 7 or 16, the transformed or transfected cell of claim 8, the shRNA or siRNA of claim 14 or 15, and the antibody of claim 13, and pharmaceutically or diagnostically acceptable salts thereof.

18. The isolated nSMase3 of anyone of claims 1 to 4, the nucleic acid of claim 5 or 6, the vector of claim 7, or pharmaceutically acceptable salts thereof for use in the prevention or treatment of heart muscle diseases, male reduced fertility or infertility.

19. The antibody of claim 13, the shRNA and siRNA of claim 14 or 15, the vector of claim 16, or pharmaceutically acceptable salts thereof for use in the (a) prevention or treatment of diseases connected with increased activity of nSMase3, preferably cramps, tremor, Parkinson's disease and hypertrophic cardiomyopathy, or (b) contraception in men or male animals.

20. An *ex-vivo* method for diagnosing and/or monitoring a disorder **characterized by** changes in the expression of an nSMase3 as defined in anyone of claims 1 to 4, which method comprises the detection of the amount and/or activity of said nSMase3 or of the nucleic acid sequence encoding said nSMase3, and/or of mutations present in said nucleic acid sequence in comparison to the native nSMase3 encoding sequence in a biological sample from a subject having or suspected to have said disorder.

21. Kit for performing the diagnostic method of claim 20, comprising one or more of the following: the nSMase3 of claims 1 to 4, the nucleic acid of claim 5 or 6, the vector of claim 7, the cell of claim 8, the antibody of claim 13, and diagnostically acceptable salts thereof.

22. An isolated cell, a non-human tissue, a tissue culture, or a non-human transgenic organism, having a reduced abundance of nSMase3 according to SEQ ID NOS:3-9 or being deficient in nSMase3 according to SEQ ID NOS:3-9 or in the nSMase3 gene according to SEQ ID NOS: 1-2, or comprising a siRNA or shRNA of claim 14 or 15 or a vector of claim 16.

23. The cell, a non-human tissue, a tissue culture or non-human organism, of claim 22, wherein the reduced abundance of nSMase3 or deficiency in nSMase3 or in the nSMase3 gene is achieved by
(i) RNAi (RNA interference), preferably using a shRNA or siRNA of claim 14 or 15 or a vector of claim 16, or
(ii) gene targeting, including conditional and inducible gene knock out, or
(iii) chemical or radiation mutagenesis.

24. Use of the cell, the non-human organism, the non-human tissue or the tissue culture of claim 22 or 23
(i) as a model for a disease connected with a deficiency of nSMAse3; or
(ii) for the generation of antibodies to nSMase3, preferably for the generation of monoclonal antibodies including the antibodies of claim 13, and/or of antibodies capable of modulating nSMase3 activity.

25. The shRNA or siRNA of claim 14 or 15, the vector of claim 16 or the antibody of claim 13 or pharmaceutically acceptable salts thereof, which are reducing the abundance of nSMase3 in an organism, for use in the treatment of diseases connected with increased heart or skeletal muscle contractility and for reducing the fertility in male patients or male animals.

26. The shRNA or siRNA of claim 14 or 15, the vector of claim 16 or the antibody of claim 13 or pharmaceutically acceptable salts thereof, which are reducing the abundance of nSMase3 in an organism, for use in the treatment of diseases connected with increased heart or skeletal muscle contractility and for reducing the fertility in male patients or male animals of claim 25, wherein the disease is one or more selected from hypertrophic cardiomyopathy, tetanus, muscle cramps, and tremor including Parkinson's disease.

## Patentansprüche

1. Isolierte neutrale Sphingomyelinase (nSMase3) mit der Aminosäuresequenz, die durch eine der SEQ ID Nr. 3 bis 9 dargestellt wird, oder eine funktionelle Variante, ein Derivat oder Fragment davon, die bzw. das die enzymatischen und/oder immunologischen Eigenschaften der nSMase3 besitzt, wobei die funktionelle Variante ein Fusionsprodukt, das eine nSMase3-Domäne und eine zweite Peptiddomäne oder eine funktionelle nichtproteinartige Komponente umfasst, wobei das Derivat von den SEQ ID Nr. 3 bis 9 durch Addition von 1 bis 2 Aminosäureresten, durch Deletion von 1 bis 100 Aminosäureresten und/oder durch konservative Substitution abgeleitet ist, wobei das Verhältnis von substituierten Aminosäureresten im Vergleich zur nativen Sequenz 0 bis 30% der gesamten Aminosäuren in der Sequenz beträgt, das Fragment wenigstens 7 Aminosäurereste der SEQ ID Nr. 3 bis 9 enthält, aber nicht aus aa 132 bis 866 der SEQ ID Nr. 3 besteht, und die nSMase3 nicht aus SEQ ID Nr. 6 bis 9 besteht.

2. Isolierte nSMase3 gemäß Anspruch 1, die
(i) eine Aminosäuresequenz, wie sie durch SEQ ID Nr. 16 oder 17 dargestellt wird, enthält, vorzugsweise die Aminosäuresequenz, wie sie durch SEQ ID Nr. 23 dargestellt wird, enthält, besonders bevorzugt eine oder mehrere der Aminosäuresequenzen, wie sie durch SEQ ID Nr. 18 bis 24 dargestellt werden, enthält; und/oder
(ii) keine N-terminale Leitsignalsequenz aufweist; und/oder
(iii) eine Vertebraten-nSMase3, vorzugsweise eine Säuger-nSMase3, besonders bevorzugt eine humane nSMase3 ist.

3. Isolierte nSMase3 gemäß Anspruch 1 oder 2, bei der es sich um die humane nSMase3 mit der SEQ ID Nr. 3 oder um eine funktionelle Variante, ein Derivat oder Fragment davon, wie sie in Anspruch 1 definiert sind, handelt.

4. Isolierte nSMase3 gemäß einem der Ansprüche 1 bis 3, die
(i) eine Transmembrandomäne in der Nähe des C-Terminus enthält; und/oder
(ii) Mg²⁺-abhängig ist; und/oder
(iii) durch TNF aktiviert wird; und/oder
(iv) ein pH-Optimum bei neutralem pH-Wert aufweist; und/oder
(v) sich mit ER-Markern kolokalisiert.

5. Isolierte Nucleinsäuresequenz, die die nSMase3 gemäß einem der Ansprüche 1 bis 4 codiert, oder dazu komplementäre Nucleinsäure.

6. Isolierte Nucleinsäuresequenz gemäß Anspruch 5, die die Sequenz von SEQ ID Nr. 1 oder 2 umfasst.

7. Vektor, der die Nucleinsäuresequenz gemäß Anspruch 5 oder 6 umfasst.

8. Zelle, die mit dem Vektor gemäß Anspruch 7 transfiziert oder transformiert ist und/oder die Nucleinsäure gemäß Anspruch 5 oder 6 heterolog umfasst und/oder eine nSMase3, wie sie in einem der Ansprüche 1 bis 4 definiert ist, heterolog exprimieren kann.

9. Nichthumanes Gewebe, Gewebekultur oder nichthumaner transgener Organismus, das bzw. die bzw. der Zellen umfasst, die mit dem Vektor gemäß Anspruch 7 transfiziert oder transformiert sind und/oder eine Nucleinsäure gemäß Anspruch 5 oder 6 heterolog umfassen und/oder eine nSMase3, wie sie in einem der Ansprüche 1 bis 4 definiert ist, heterolog exprimieren.

10. Verwendung der Zelle gemäß Anspruch 8, des nichthumanen transgenen Organismus, des nichthumanen Gewebes oder der Gewebekultur gemäß Anspruch 9 als Testzellsystem, Testgewebe oder Testmodellorganismus beim Testen von Wirkstoffkandidaten auf ihr therapeutisches Potential bei Krankheiten, die mit nSMase3 verbunden sind, vorzugsweise bei Herzmuskelkrankheiten, reduzierter Fruchtbarkeit oder Unfruchtbarkeit beim Mann, oder auf ihr Potential als Kontrazeptivum bei Männern oder männlichen Tieren.

11. Verfahren zum Testen von Wirkstoffkandidaten auf ihr therapeutisches Potential bei Krankheiten, die mit nSMase3 verbunden sind, vorzugsweise bei Herzmuskelkrankheiten, reduzierter Fruchtbarkeit oder Unfruchtbarkeit beim Mann, oder auf ihr Potential als Kontrazeptivum bei Männern oder männlichen Tieren, umfassend die Schritte:
(a) Verabreichen des Wirkstoffkandidaten an eine Kultur der Zelle gemäß Anspruch 8, ein nichthumanes Gewebe oder eine Gewebekultur gemäß Anspruch 9; und
(b) Bestimmen der Wirkung des Wirkstoffkandidaten auf die Zelle oder das Gewebe.

12. Verfahren zur Herstellung der nSMase3 gemäß einem der Ansprüche 1 bis 4, umfassend das Kultivieren von Zellen, wie sie in Anspruch 8 definiert sind, und das Isolieren der nSMase3 aus der Kultur.

13. Antikörper, der spezifisch für die nSMase3 mit der durch eine der SEQ ID Nr. 3 bis 9 dargestellten Aminosäuresequenz ist.

14. shRNA oder siRNA gegen nSMase, wie sie in einem der Ansprüche 1 bis 4 definiert ist.

15. shRNA gemäß Anspruch 14, die die durch SEQ ID Nr. 30 und/oder 31 dargestellte Sequenz umfasst.

16. Vektor, der die shRNA oder siRNA gemäß Anspruch 14 oder 15 codiert.

17. Pharmazeutische oder diagnostische Zusammensetzung, die eins oder mehrere der folgenden umfasst: die isolierte nSMase3 gemäß einem der Ansprüche 1 bis 4, die Nucleinsäure gemäß Anspruch 5 oder 6, den Vektor gemäß Anspruch 7 oder 16, die transformierte oder transfizierte Zelle gemäß Anspruch 8, die shRNA oder siRNA gemäß Anspruch 14 oder 15 und den Antikörper gemäß Anspruch 13; sowie pharmazeutisch oder diagnostisch annehmbare Salze davon.

18. Isolierte nSMase3 gemäß einem der Ansprüche 1 bis 4, Nucleinsäure gemäß Anspruch 5 oder 6, Vektor gemäß Anspruch 7 oder pharmazeutisch annehmbare Salze davon zur Verwendung bei der Prävention oder Behandlung von Herzmuskelkrankheiten, reduzierter Fruchtbarkeit oder Unfruchtbarkeit beim Mann.

19. Antikörper gemäß Anspruch 13, shRNA und siRNA gemäß Anspruch 14 oder 15, Vektor gemäß Anspruch 16 oder pharmazeutisch annehmbare Salze davon zur Verwendung bei der (a) Prävention oder Behandlung von Krankheiten, die mit einer erhöhten Aktivität von nSMase3 verbunden sind, vorzugsweise Krämpfen, Tremor, Parkinson-Krankheit und hypertrophe Kardiomyopathie, oder (b) Empfängnisverhütung bei Männern oder männlichen Tieren.

20. Ex-vivo-Verfahren zum Diagnostizieren und/oder Überwachen einer Störung, die durch Veränderungen der Expression einer nSMase3, wie sie in einem der Ansprüche 1 bis 4 definiert ist, **gekennzeichnet** ist, wobei das Verfahren den Nachweis der Menge und/oder Aktivität der nSMase3 oder der Nucleinsäuresequenz, die die nSMase3 codiert, und/oder von Mutationen, die in der Nucleinsäuresequenz im Vergleich zur nativen nSMasecodierenden Sequenz vorhanden sind, in einer biologischen Probe von einem Probanden, der an dieser Störung leidet oder von dem man annimmt, dass er daran leiden könnte, umfasst.

21. Kit zur Durchführung des diagnostischen Verfahrens gemäß Anspruch 20, der eins oder mehrere der folgenden umfasst: die nSMase3 gemäß den Ansprüchen 1 bis 4, die Nucleinsäure gemäß Anspruch 5 oder 6, den Vektor gemäß Anspruch 7, die Zelle gemäß Anspruch 8, den Antikörper gemäß Anspruch 13 und diagnostisch annehmbare Salze davon.

22. Isolierte Zelle, nichthumanes Gewebe, Gewebekultur oder nichthumaner transgener Organismus mit einer reduzierten Abundanz von nSMase3 gemäß SEQ ID Nr. 3 bis 9 oder einem Mangel an nSMase3 gemäß SEQ ID Nr. 3 bis 9 oder an dem nSMase3-Gen gemäß SEQ ID Nr. 1 bis 2 oder mit einer siRNA oder shRNA gemäß Anspruch 14 oder 15 oder einem Vektor gemäß Anspruch 16.

23. Zelle, nichthumanes Gewebe, Gewebekultur oder nichthumaner Organismus gemäß Anspruch 22, wobei die reduzierte Abundanz von nSMase3 oder der Mangel an nSMase3 oder des nSMase3-Gens erreicht wird durch
(i) RNAi (RNA-Interferenz), vorzugsweise unter Verwendung einer shRNA oder siRNA gemäß Anspruch 14 oder 15 oder eines Vektors gemäß Anspruch 16; oder
(ii) Gen-Targeting einschließlich eines bedingten oder induzierbaren Gen-Knockouts; oder
(iii) chemische oder strahlungsbedingte Mutagenese.

24. Verwendung der Zelle, des nichthumanen Organismus, des nichthumanen Gewebes oder der Gewebekultur gemäß Anspruch 22 oder 23
(i) als Modell für eine Krankheit, die mit einem Mangel an nSMase3 verbunden ist; oder
(ii) zur Erzeugung von Antikörpern gegen nSMase3, vorzugsweise zur Erzeugung von monoklonalen Antikörpern einschließlich der Antikörper gemäß Anspruch 13, und/oder von Antikörpern, die die nSMase3-Aktivität modulieren können.

25. shRNA oder siRNA gemäß Anspruch 14 oder 15, Vektor gemäß Anspruch 16 oder Antikörper gemäß Anspruch 13 oder pharmazeutisch annehmbare Salze davon, die die Abundanz von nSMase3 in einem Organismus reduzieren, zur Verwendung bei der Behandlung von Krankheiten, die mit einer erhöhten Kontraktilität von Herz- oder Skelettmuskeln verbunden sind, und zur Reduktion der Fruchtbarkeit bei männlichen Patienten oder männlichen Tieren.

26. shRNA oder siRNA gemäß Anspruch 14 oder 15, Vektor gemäß Anspruch 16 oder Antikörper gemäß Anspruch 13 oder pharmazeutisch annehmbare Salze davon, die die Abundanz von nSMase3 in einem Organismus reduzieren, zur Verwendung bei der Behandlung von Krankheiten, die mit einer erhöhten Kontraktilität von Herz- oder Skelettmuskeln verbunden sind, und zur Reduktion der Fruchtbarkeit bei männlichen Patienten oder männlichen Tieren gemäß Anspruch 25, wobei es sich bei der Krankheit um eine oder mehrere handelt, die aus hypertropher Kardiomyopathie, Tetanus, Muskelkrämpfen und Tremor einschließlich Parkinson-Krankheit ausgewählt sind.

## Revendications

1. Sphingomyélinase neutre isolée (nSMase3) ayant la séquence d'acides aminés représentée par l'un des SEQ. ID n° 3 à 9, ou un variant fonctionnel, un dérivé ou un fragment de celle-ci qui possède les propriétés enzymatiques et/ou immunologiques de ladite nSMase3, où ledit variant fonctionnel est un produit de fusion comprenant un domaine de nSMase3 et un second domaine peptidique ou un composant fonctionnel non protéique, ledit dérivé est dérivé des SEQ ID n° 3 à 9 par addition d'1 ou de 2 résidus d'acides aminés, par délétion d'1 à 100 résidus d'acides aminés et/ou par substitution conservative, où le rapport entre résidus d'acides aminés substitués et séquence native est compris entre 0 et 30 % du total des acides aminés de la séquence, ledit fragment contient au moins 7 résidus d'acides aminés des SEQ ID n° 3 à 9, mais ne se compose pas des acides aminés 132 à 866 du SEQ ID n° 3, et ladite nSMase3 ne se composant pas des SEQ ID n° 6 à 9.

2. nSMase3 isolée selon la revendication 1, qui
(i) contient une séquence d'acides aminés telle que représentée par le SEQ ID n° 16 ou 17, qui contient de préférence la séquence d'acides aminés telle que représentée par le SEQ ID n° 23, et qui contient de façon plus préférentielle une ou plusieurs des séquences d'acides aminés telles que représentées par les SEQ ID n° 18 à 24 ; et/ou
(ii) n'a pas de séquence-signal leader N-terminale ; et/ou
(iii) est une nSMase3 de vertébré, de préférence une nSMase3 de mammifère, de façon plus préférentielle une nSMase3 humaine.

3. nSMase3 isolée selon la revendication 1 ou 2, qui est la nSMase3 humaine présentant le SEQ ID n° 3, ou qui est un variant fonctionnel, un dérivé ou un fragment de celle-ci tel que défini dans la revendication 1.

4. nSMase3 isolée selon l'une quelconque des revendications 1 à 3, qui
(i) contient un domaine transmembranaire proche de l'extrémité C-terminale ; et/ou qui
(ii) est dépendante de Mg²⁺ ; et/ou qui
(iii) est activée par le TNF ; et/ou qui
(iv) a un optimum de pH à un pH neutre ; et/ou qui
(v) se colocalise avec les marqueurs de RE.

5. Séquence d'acide nucléique isolée codant pour la nSMase3 telle que définie dans l'une quelconque des revendications 1 à 4, ou acide nucléique complémentaire de celle-ci.

6. Séquence d'acide nucléique isolée selon la revendication 5, qui comprend le SEQ ID n° 1 ou 2.

7. Vecteur comprenant la séquence d'acide nucléique selon la revendication 5 ou 6.

8. Cellule transfectée ou transformée avec le vecteur selon la revendication 7 et/ou comprenant de façon hétérologue l'acide nucléique de la revendication 5 ou 6 et/ou capable d'exprimer de façon hétérologue une nSMase3 telle que définie dans l'une quelconque des revendications 1 à 4.

9. Tissu non humain, culture de tissu ou organisme transgénique non humain comprenant des cellules transfectées ou transformées avec le vecteur selon la revendication 7 et/ou comprenant de façon hétérologue une séquence d'acide nucléique telle que définie dans les revendications 5 ou 6 et/ou exprimant de façon hétérologue une nSMase3 telle que définie dans l'une quelconque des revendications 1 à 4.

10. Utilisation de la cellule selon la revendication 8, de l'organisme transgénique non humain, du tissu non humain ou de la culture de tissu selon la revendication 9 en tant que système de cellule test, tissu test ou organisme modèle test pour tester des agents actifs candidats pour leur potentiel thérapeutique dans des maladies associées à la nSMase3, de préférence des maladies du muscle cardiaque, une réduction de la fertilité ou une infécondité chez les hommes, ou pour leur potentiel en tant que contraceptif chez les hommes ou les animaux mâles.

11. Procédé pour tester des agents actifs candidats pour leur potentiel thérapeutique dans des maladies associées à la nSMase3, de préférence des maladies du muscle cardiaque, une réduction de la fertilité ou une infécondité chez les hommes, ou pour leur potentiel en tant que contraceptif chez les hommes et les animaux mâles, comprenant les étapes consistant à :
(a) administrer l'agent candidat à une culture de la cellule selon la revendication 8, à un tissu non humain ou à une culture de tissu selon la revendication 9 ; et à
(b) déterminer l'effet de l'agent candidat sur ladite cellule ou ledit tissu.

12. Procédé de préparation de la nSMase3 selon l'une quelconque des revendications 1 à 4, qui comprend la culture des cellules telles que définies dans la revendication 8 et l'isolement de la nSMase3 à partir de la culture.

13. Anticorps spécifique de la nSMase3 ayant la séquence d'acides aminés représentée par l'un des SEQ ID n° 3 à 9.

14. ARNsh ou ARNsi contre nSMase3 telle que définie dans l'une quelconque des revendications 1 à 4.

15. ARNsh de la revendication 14, qui comprend la séquence représentée par le SEQ ID n° 30 et/ou le SEQ ID n° 31.

16. Vecteur codant pour l'ARNsh ou l'ARNsi selon la revendication 14 ou 15.

17. Composition pharmaceutique ou diagnostique comprenant un ou plusieurs des éléments suivants : la nSMase3 isolée selon l'une quelconque des revendications 1 à 4, l'acide nucléique selon la revendication 5 ou 6, le vecteur selon la revendication 7 ou 16, la cellule transformée ou transfectée selon la revendication 8, l'ARNsh ou l'ARNsi selon la revendication 14 ou 15, et l'anticorps selon la revendication 13, et les sels de ceux-ci acceptables sur le plan pharmaceutique ou diagnostique.

18. nSMase3 isolée selon l'une quelconque des revendications 1 à 4, acide nucléique selon la revendication 5 ou 6, vecteur selon la revendication 7, ou sels de ceux-ci acceptables sur le plan pharmaceutique, pour une utilisation dans la prévention ou le traitement de maladies du muscle cardiaque, d'une réduction de la fertilité chez les hommes ou d'une infécondité.

19. Anticorps selon la revendication 13, ARNsh et ARNsi selon la revendication 14 ou 15, vecteur selon la revendication 16, ou sels de ceux-ci acceptables sur le plan pharmaceutique, pour une utilisation dans (a) la prévention ou le traitement de maladies associées à une activité accrue de la nSMase3, de préférence des crampes, des tremblements, la maladie de Parkinson et la cardiomyopathie hypertrophique, ou (b) la contraception chez les hommes ou les animaux mâles.

20. Procédé *ex vivo* de diagnostic et/ou de surveillance d'un trouble **caractérisé par** des modifications de l'expression d'une nSMase3 telle que définie dans l'une quelconque des revendications 1 à 4, ledit procédé comprenant la détection de la quantité et/ou de l'activité de ladite nSMase3 ou de la séquence d'acide nucléique codant pour ladite nSMase3, et/ou des mutations présentes dans ladite séquence d'acide nucléique en comparaison avec la séquence native codant pour la nSMase3, dans un échantillon biologique d'un sujet présentant ou suspecté de présenter ledit trouble.

21. Kit de réalisation du procédé de diagnostic selon la revendication 20, comprenant un ou plusieurs des éléments suivants : la nSMase3 selon les revendications 1 à 4, l'acide nucléique selon la revendication 5 ou 6, le vecteur selon la revendication 7, la cellule selon la revendication 8, l'anticorps selon la revendication 13, et des sels de ceux-ci acceptables sur le plan diagnostique.

22. Cellule isolée, tissu non humain, culture de tissu, ou organisme transgénique non humain, présentant une abondance réduite de nSMase3 selon les SEQ ID n° 3 à 9 ou étant déficient(e) en nSMase3 selon les SEQ ID n° 3 à 9 ou en gène de nSMase3 selon les SEQ ID n° 1 à 2, ou comprenant un ARNsi ou un ARNsh selon la revendication 14 ou 15 ou un vecteur selon la revendication 16.

23. Cellule, tissu non humain, culture de tissu, ou organisme non humain selon la revendication 22, dans lequel (laquelle) l'abondance réduite de nSMase3 ou la déficience en nSMase3 ou en gène de nSMase3 est obtenue par
(i) ARNi (interférence d'ARN), de préférence en utilisant un ARNsh ou un ARNsi selon la revendication 14 ou 15 ou un vecteur selon la revendication 16, ou
(ii) ciblage de gène, notamment une inactivation de gène (gene knockout) conditionnelle ou inductible, ou
(iii) une mutagenèse chimique ou par irradiation.

24. Utilisation de la cellule, de l'organisme non humain, du tissu non humain ou de la culture de tissu selon la revendication 22 ou 23
(i) en tant que modèle d'une maladie liée à une déficience en nSMase3 ; ou
(ii) pour générer des anticorps dirigés contre nSMase3, de préférence pour générer des anticorps monoclonaux, notamment les anticorps selon la revendication 13, et/ou des anticorps capables de moduler l'activité de la nSMase3.

25. ARNsh ou ARNsi selon la revendication 14 ou 15, vecteur selon la revendication 16 ou anticorps selon la revendication 13, ou sels de ceux-ci acceptables sur le plan pharmaceutique, qui réduisent l'abondance de la nSMase3 dans un organisme, pour une utilisation dans le traitement de maladies associées à une augmentation de la contractilité du coeur ou d'un muscle squelettique et pour réduire la fertilité chez les patients hommes ou les animaux mâles.

26. ARNsh ou ARNsi selon la revendication 14 ou 15, vecteur selon la revendication 16 ou anticorps selon la revendication 13, ou sels de ceux-ci acceptables sur le plan pharmaceutique, qui réduisent l'abondance de la nSMase3 dans un organisme, pour une utilisation dans le traitement de maladies associées à une augmentation de la contractilité du coeur ou d'un muscle squelettique et pour réduire la fertilité chez les patients hommes ou les animaux mâles selon la revendication 25, la maladie étant une ou plusieurs maladies sélectionnées parmi la cardiomyopathie hypertrophique, le tétanos, des crampes musculaires et des tremblements, notamment la maladie de Parkinson.
